# EUROPEAN PATENT SPECIFICATION

(11) **EP 0 684 831 B1**
(45) Date of publication and mention of the grant of the patent: **03.09.2003**
(21) Application number: 94909636.6
(22) Date of filing: 17.02.1994
(51) Int. Cl.: C12N 15/26, C12N 15/23, C12N 5/10, A61K 39/00, C12N 15/27

(54) **ALLOGENIC VACCINE AND METHOD TO SYNTHESIZE SAME**
ALLOGENES VAKZIN UND SYNTHESEMETHODE FÜR SELBIGES
VACCIN ALLOGENE ET PROCEDE DE SYNTHESE DE CE VACCIN

(30) Priority: 17.02.1993 US 18940
(43) Date of publication of application: 06.12.1995
(73) Proprietor: Sloan-Kettering Institute For Cancer Research, New York, New York 10021 (US)
(72) Inventor: GANSBACHER, Bernd Apartment 13F, New York, NY 10021 (US)
(74) Representative: VOSSIUS & PARTNER
(86) International application number: US9401631
(87) International publication number: WO94018995

(56) References cited:
- WO-A-92/05262
- WO-A-93/10219
- WO-A-95/07105
- L.M. MIR ET AL: "Potentialisation of the antitumoral effect of electrochemotherapy with allogeneic cells producing interleukin-2" COMPTE RENDU DE L' ACAD MIE DES SCIENCES PARIS SERIE III, vol. 314, 1992, pages 539-544, XP002067382
- T.S.KIM ET AL: "Immunity to B16 melanoma in mice immunized with iL-2-secreting allogeneic mouse fibroblasts expressing melanoma-associated antigens." INTERNATIONAL JOURNAL OF CANCER, vol. 51, no. 2, 8 May 1992, pages 283-289, XP002065929
- LIVINGSTON P.O. ET AL: "Serologic response of patients with stage II melanoma to allogenic melanoma cell vaccines" CANCER, vol. 56, November 1985, page 2194 XP002065930
- S. OSTRAND-ROSENBERG ET AL: "Tumor-specific immunity can be enhanced by transfection of tumor cells with syngeneic MHC-class genes or allogeneic MHC-class-I genes." INTERNATIONAL JOURNAL OF CANCER, vol. suppl 6, 1991, pages 61-68, XP002065931
- GANSBACHER B ET AL: "RETROVIRAL VECTOR-MEDIATED GAMMA-INTERFERON GENE TRANSFER INTO TUMOR CELLS GENERATES POTENT AND LONG LASTING ANTITUMOR IMMUNITY" CANCER RESEARCH, vol. 50, no. 24, 15 December 1990, pages 7820-7825, XP000197170
- GANSBACHER B. ET AL: "Retroviral vectors carrying both tHe iL-2 and IFN-gamma gene induce potent antitumor responses in murine tumors" PROCEEDINGS OF THE AMERICAN ASSOCIATION FOR CANCER RESEARCH, vol. 33, 1992, page 351 XP002067802
- YOSHIHIKO HAYASHI ET AL: "Cytotoxic T cell lines recognize autologous and allogeneic melanomas with shared or cross-reactive HLA-A" CANCER IMMUNOLOGY IMMUNOTHERAPY, vol. 34, 1992, pages 419-423, XP002065932
- S. OSANTO ET AL: "Immunization with interleukin-2 transfected melanoma cells. a phase I-II study in patients with metastatic melanoma" HUMAN GENE THERAPY, vol. 4, no. 3, June 1993, pages 323-330, XP002065933
- SEDLACEK H H: "VACCINATION FOR TREATMENT OF TUMORS: A CRITICAL COMMENT" CRITICAL REVIEWS IN ONCOGENESIS, vol. 5, no. 6, 1994, pages 555-587, XP000198151
- British Journal of Cancer, Volume 66, issued 1992, FOA et al.: "IL2 Treatment for Cancer: from Biology to Gene Therapy", pages 992-998, see entire article.
- Blood, Volume 80, No. 11, issued 01 December 1992, GANSBACHER et al.: "Retroviral Gene Transfer Induced Constitutive Expression of Interleukin-2 or Interferon-Gamma in Irradiated Human Melanoma Cells", pages 2817-2825, see entire article.
- Cancer Research, Volume 52, issued 15 November 1992, GASTL et al.: "Retroviral Vector-Mediated Lymphokine Gene Transfer into Human Renal Cancer Cells", pages 6229-6236, see entire article.
- Journal of Experimental Medicine, Volume 172, issued October 1990, GANSBACHER et al.: "Interleukin 2 Gene Transfer into Tumor Cells Abrogates Tumorigenicity and Induces Protective Immunity", pages 1217-1224, see entire article.
- Proceedings of the National Academy of Sciences, Volume 86, issued May 1989, HANTZOPOULOS et al.: "Improved Gene Expression upon Transfer of the Adenosine Deaminase Minigene Outside the Transcriptional Unit of a Retroviral Vector", pages 3519-3523, see entire article.
- Cell, Volume 71, issued 24 December 1992, CHEN et al.: "Costimulation of Antitumor Immunity by the B7 Counterreceptor for the T Lymphocyte Molecules CD28 and CTLA-4", pages 1093-1102, see entire article.
- Journal of Experimental Medicine, Volume 173, issued April 1991, COLOMBO et al.: "Granulocyte Colony-Stimulating Factor Gene Transfer Suppresses Tumorigenicity of a Murine Adenocarcinoma in Vivo", pages 889-897, see entire article.

## Description

### BACKGROUND OF THE INVENTION

Current therapeutic approaches for metastatic melanoma are inadequate. Therapeutic approaches that aim to amplify the patient's own immune response have received considerable attention based on evidence in animal models of protective immune responses to a tumor cell challenge following appropriate immunization (Borberg et al., 1972; Smith et al., 1977; Fernandez-Crus et al., 1979; Cheever et al., 1980; Shu et al., 1985).

Whereas vaccination protocols in cancer patients have not shown a clear impact on patient survival, evidence in several trials suggests that patients who develop immune responses have a significant improvement in disease-free survival and overall survival compared to non-responders (Livingston, 1991; Livingston et al.. 1985a; Livingston et al., 1985b; Livingston et al., 1987; Livingston et al., 1979; Livingston et al., 1983; Livingston and Watanabe, 1982; Livingston et al., 1985c). A major aim of vaccination trials has been to induce serologic and cytotoxic T cell (CTL) responses. Although blood and tumor infiltrates from melanoma and renal carcinoma patients contain circulating cytotoxic precursor cells with restricted reactivity for autologous melanoma (Darrow et al., 1989; Crowley et al., 1990; Crowley et al., 1991; Kawakami et al., 1992; Crowley et al., 1992) and renal carcinomas (Belldegrun et al. 1990; Radrizzani et al., 1989; Belldegrun et al., 1989; Ikemoto et al., 1992; Belldegrun et al., 1988; Koo et al., 1991; Alexander et al., 1990), it has been difficult to show that vaccination induces or amplifies serological or CTL responses. Several hypotheses exist, such as tumor cells are poor antigen presenting cells, have low or absent MHC molecules expressed on the cell surface, secrete suppressor factors or that T-cell receptor (TCR) engagement by the tumor antigens without a costimulatory signal induces anergy. IL-2 can reverse two of those mechanisms. First, IL-2 can reverse suppression of CTL responses induced by tumor growth factor-β (TGF-β) (Inge et al., 1992). Second, engagement of the TCR without a proliferative signal can induce anergy, but presence of IL-2 may prevent this anergy from developing (Harding et al., 1992; Liu et al., 1992).

Studies have shown that the introduction of cytokine genes into murine tumor cells induced increased immunogenicity and decreased tumorigenesis (Gansbacher et al. 1990a; Fearon et al. 1990; Ley et al., 1991; Watanabe et al., 1989; Gansbacher et al., 1990b; Gansbacher et al., 1992; Tepper et al., 1989; Hock et al., 1991; Porgador et al., 1992). Secretion of IL-2 or IFN-γ by a murine fibrosarcoma led to a T-cell mediated response that resulted in rejection of the tumor (Gansbacher et al., 1990a; Gansbacher et al., 1990b). Human melanoma (Crowley et al., 1990) is an especially promising candidate for this strategy of active immunization. cytotoxic T cell clones specific for autologous melanoma cells can be generated in patients by stimulating peripheral blood mononuclear cells with tumor cells in the presence of IL-2. In addition, shared melanoma antigens recognized by CTL in the context of specific MHC haplotypes (e.g., HLA-A1 and HLA-A2) have been identified (Darrow et al., 1989; Crowley et al., 1990; Crowley et al., 1991; Kawakami et al., 1992; Crowley et al., 1992). Recently, a gene encoding a melanoma antigen recognized by autologous CTL was cloned from a cosmid library made from cDNA of melanoma cells (Van Der Bruggen et al., 1991). These studies and others provide evidence that shared melanoma epitopes exist which are recognized by T cells, implying that allogeneic melanoma cells might be suitable for use in vaccination protocols (Crowley et al., 1990; Wolfel et al., 1989; Knuth et al., 1989).

Based upon evidence that there are shared tumor antigens in melanoma (Crowley et al., 1990; Kawakami et al., 1992; Wolfel et al., 1989; Knuth et al., 1989), that cytokine secretion by tumor cells increases immunogenicity (Inge et al., 1992; Harding et al., 1992; Liu et al., 1992; Gansbacher et al., 1990a; Fearon et al., 1990; Ley et al., 1991; Watanabe et al., 1989; Gansbacher et al., 1990b; Gansbacher et al., 1992; Tepper et al., 1989; Hock et al., 1991) that the frequency of CTL precursors in melanoma patients can be as high as 1:900 (Coulie et al., 1992), and that in vivo secretion of IL-2 by tumor cells increased the frequency of circulating cytotoxic precursors and of CTL's specific for the immunizing tumor in a murine model (Ley et al., 1991), applicant decided to use gene transfer technology to induce melanoma cells to secrete IL-2 or IFN-γ. These cells could potentially be administered to appropriately HLA-matched patients and lead to amplification of existing cytotoxic T cell precursors.

One distinct feature of this invention is that allogeneic vaccine which expresses at least one immunomolecule selected from the group consisting of cytokine, adhesion molecule, costimulatory factor, tumor associated antigen and tumor specific antigen is used instead of previously disclosed syngeneic vaccine approach.

### BRIEF DESCRIPTION OF FIGURES

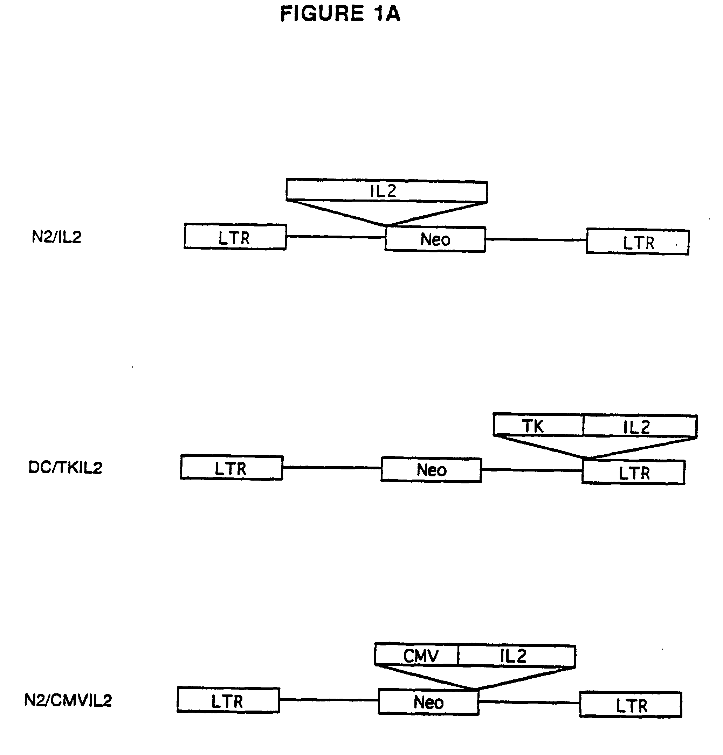

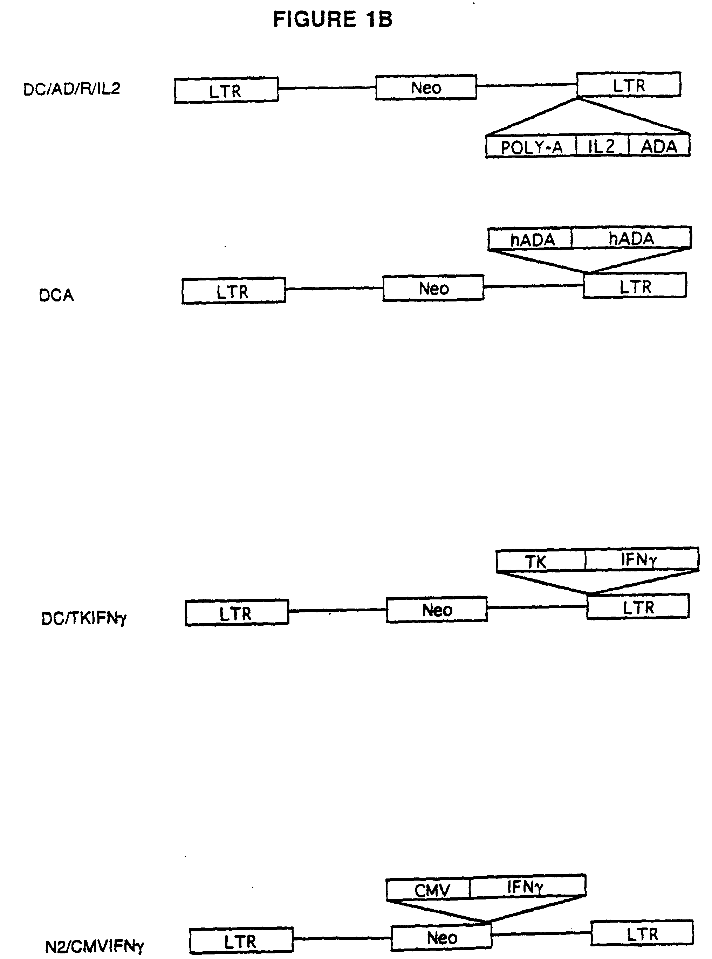

- Figures 1A and 1B: Structure of retroviral vectors containing the human IL-2 or IFN-γ cDNAs (For additional details see Materials and Methods in the first series of experiments).

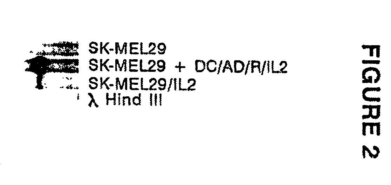

- Figure 2: Southern blot analysis of transduced SK-MEL-29. Genomic DNA was restricted with Bgl II, electrophoresed, blotted, and probed with the NeoR cDNA. As a size control the plasmid DC/AD/R/IL2 cut with SpeI was admixed with SK-MEL-29 DNA. Bgl II cuts once in the polylinker in the 3' LTR.

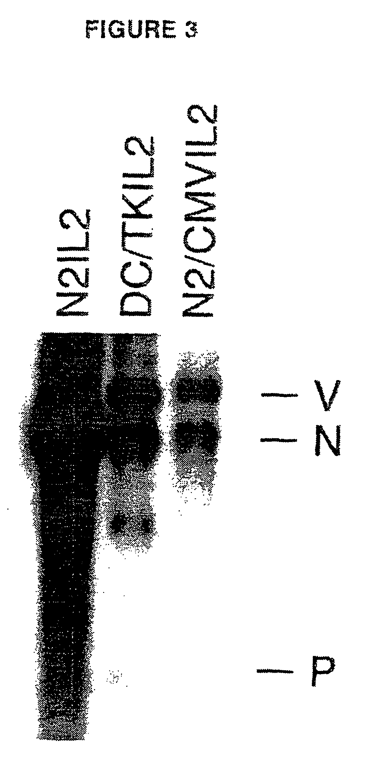

- Figure 3: Northern blot analysis of poly(A) mRNA from transduced SK-MEL-256 cells. Poly(A) RNA, fractionated on a 1% formaldehyde/agarose gel and blotted to nylon filters, was hybridized to a Mo-MuLV U3-specific probe. "V" and "N" are LTR-initiated transcripts, the unspliced virion RNA (V) and spliced Neo mRNA (N). "P" is an internal promoter initiated RNA transcript.

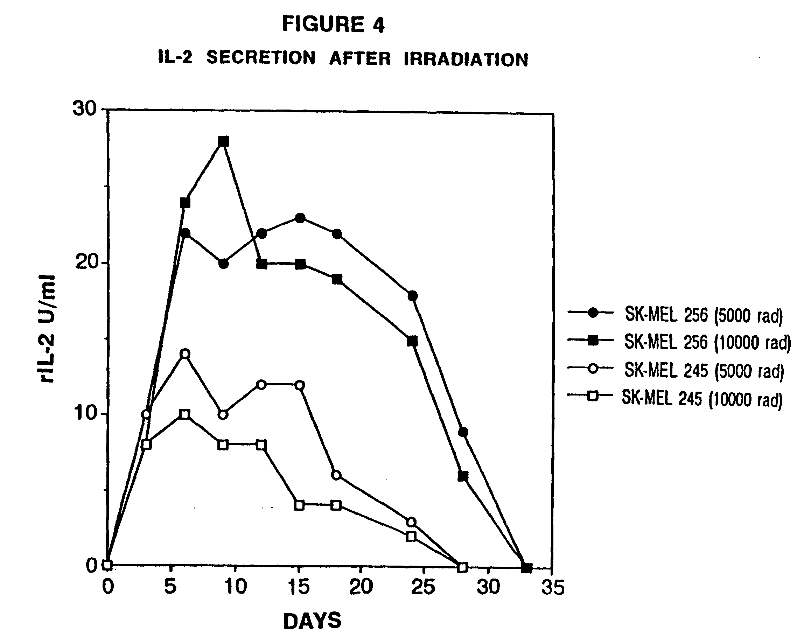

- Figure 4: IL-2 secretion by irradiated SK-MEL-256 and SK-MEL-245 cells. One million SK-MEL-256 or SK-MEL-245 cells were plated in a 60 mm dish, irradiated with 5,000 or 10,000 rad and supernatant collected every 3 days. IL-2 in the test supernatant was determined using a bioassay and an ELISA as described in Materials and Methods.

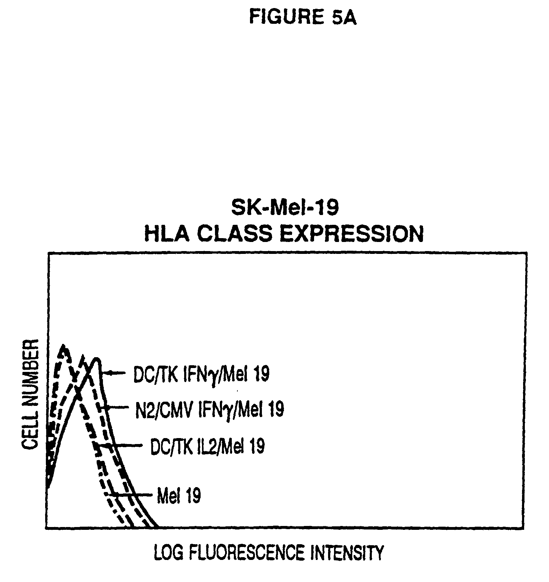

- Figures 5A - 5D: MHC class I expression of parental and cytokine secreting SK-MEL-131 cells, SK-MEL-19, SK-MEL-245 and SK-MEL-256. Live cells were labeled with monoclonal antibody W6/32 directed against a monomorphic determinant on the MHC class I molecule, followed by fluorescein isothiocyanate-conjugated affinity purified goat anti-mouse IgG (GaM-FITC) and analyzed with an EPICS V fluorescence-activated cell sorter (Coulter Electronics, Inc.).

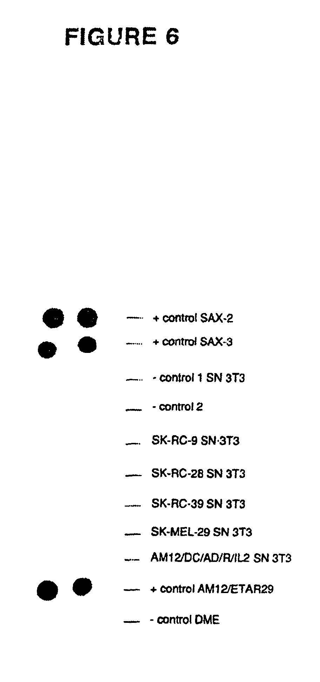

- Figure 6: NIH3T3 Amplification assay. RT was done on supernatants collected from cells that had been transduced 28 days prior. SK-MEL-29, SK-RC-28 and SK-RC-39: Supernatants taken from melanoma and renal carcinoma cell lines transduced with DC/AD/R/IL2. SAX-2: SN from NIH3T3 cells transduced with SAX-2 and known to have helper virus. The packaging cell line used to generate SAX-2 was PA 317. SN 3T3: SN taken from non-transduced NIH3T3 cells. AM12/DC/AD/R/IL2 SN 3T3: SN taken from 3T3 cells transduced with DC/AD/R/IL2. AM12/ETAR29: SN taken from producer cell line AM12/ETAR29.

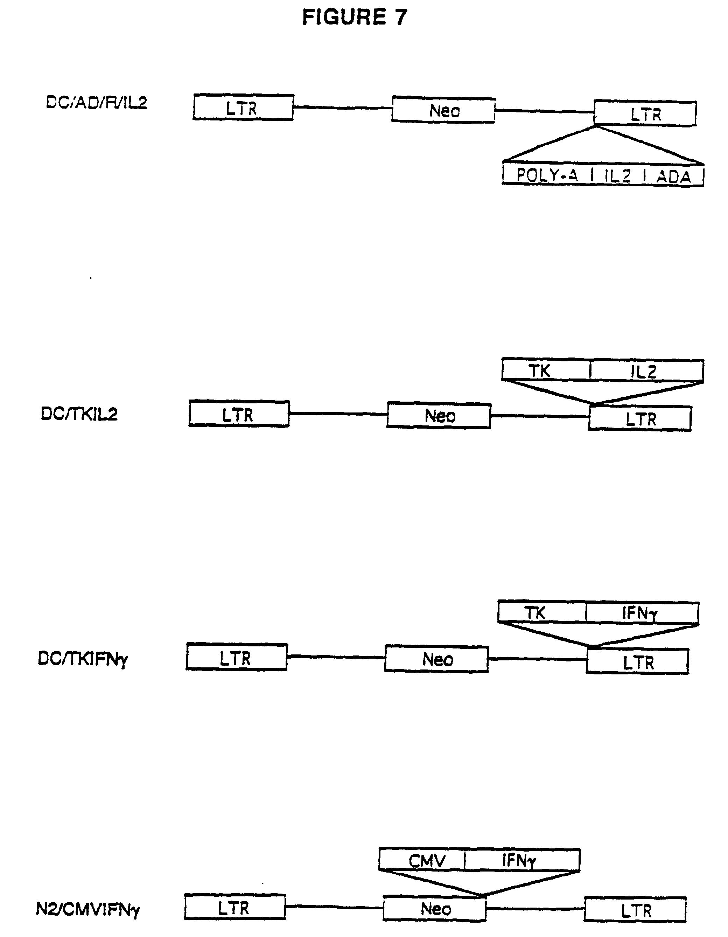

- Figure 7: Structure of retroviral vectors containing the human cDNA for IL-2 or IFNγ (for details see Material and Methods in the third series of experiments).

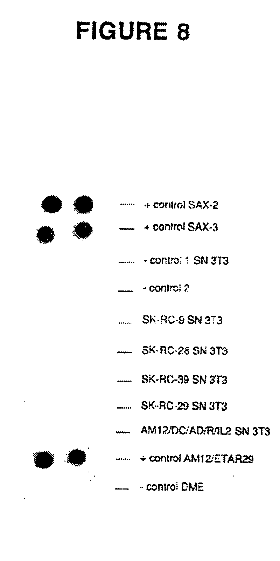

- Figure 8: Detection of replication competent murine retrovirus using the NIH3T3 amplification assay. No reverse transcriptase activity could be detected in cell culture supernatants obtained from NIH3T3 cells, which had been exposed to supernatants from the transduced RC cell lines SK-RC-9, SK-RC-28, SK-RC-29 and SK-RC-39, and the producer cell line AM12 carrying the vector construct DC/AD/R/IL2. Cultures of the producer cell line AM12/ETAR29 and helper virus-producing cell lines SAX-2 and SAX-3 were used as positive controls. Spent media from unmodified NIH/3T3 cell (controls 1 and 2) or Dulbecco's modified Eagle's medium alone served as negative controls.

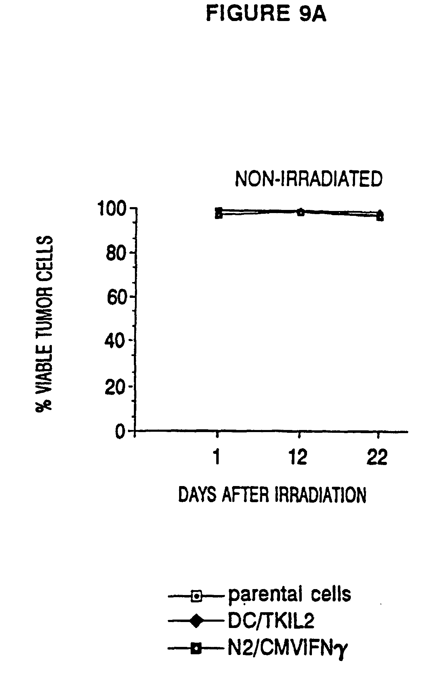

- Figures 9A - 9C: Survival of SK-RC-29 cells following *in vitro* γ-irradiation with 5,000 or 10,000 rad: 0.25 x 10⁶ tumor cells were plated in duplicate irradiated with 5,000 or 10,000 rad (day 0), and trypsinized 1, 12 and 22 days after irradiation. The percentage of viable cells was assessed by trypan-blue staining using a hemocytometer. Solid circle indicates parental cells: Solid triangle indicates DC/TKIL2: Solid square indicates N2/CMVIFHγ.

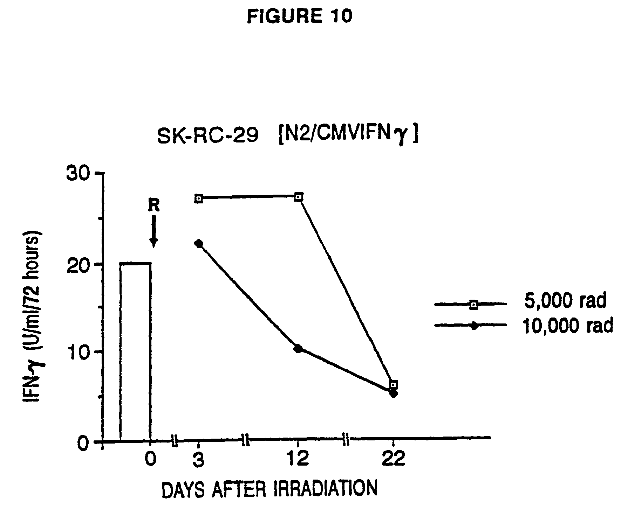

- Figure 10: Effect of γ-irradiation (R) on the release of IFNγ from SK-RC-29 cells carrying the N2/CMVIFNγ construct: 72-h culture supernatants were collected before (day 0: open bar), and 3, 12 and 22 days after irradiation. The IFN-γ concentration in supernatants was determined by bioassay and radioimmunoassay. Results represent mean values of duplicates as obtained by radioimmunoassay.

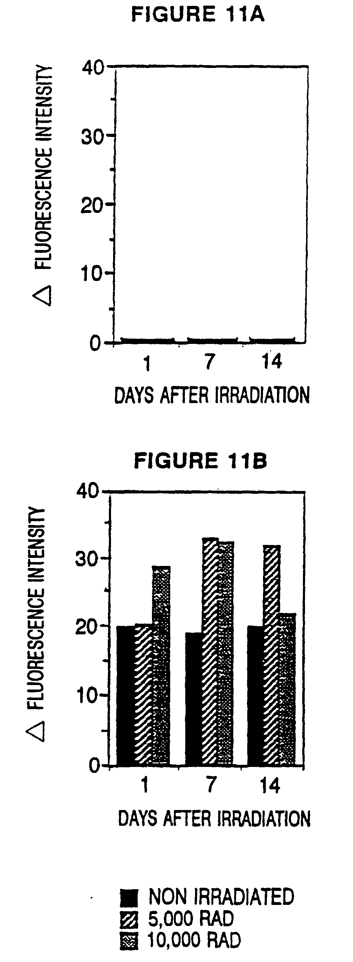

- Figures 11A, 11B: Effect of γ-irradiation on HLA-DR expression. Expression of HLA-DR antigen by (A) parental and (B) SK-RC-29 cells carrying the N2/CMVIFNγ construct was evaluated on days 1, 7 and 14 after γ-irradiation by indirect immunofluorescence. The mean fluorescence intensity as determined by FACS is expressed arbitrary units of a logarithmic scale (test sample - negative control). As negative control, primary antibody was substituted by PBS.

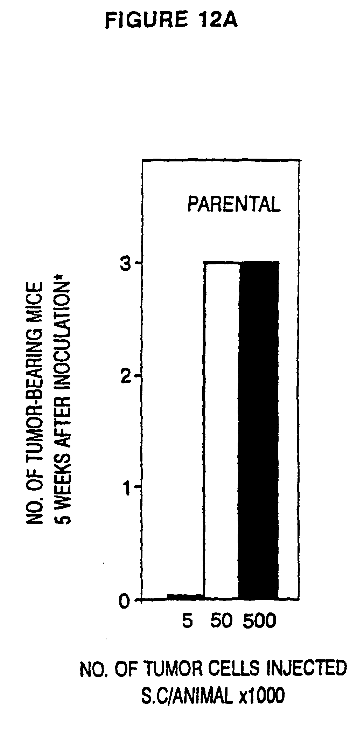

- Figures 12A - 12C: Tumor formation by parental and lymphokine-releasing SK-RC-29 cells *in vivo.* Tumor formation in BALB/c nude mice was assessed 5 weeks after s.c. tumor cell inoculation. *Three mice were used for each experiment. **At the highest dose level. SK-RC-29 cells carrying the DC/TKIL2 construct formed only small s.c. cell aggregates at the injection site (mean weight ± SD:0.02±0 g): animals given injections of parental cells or IFN-γ secreting SK-RC-29 cells carrying the N2/CMVIFNγ construct developed large s.c. tumors (mean weight ± SD:3.63±0.8g).

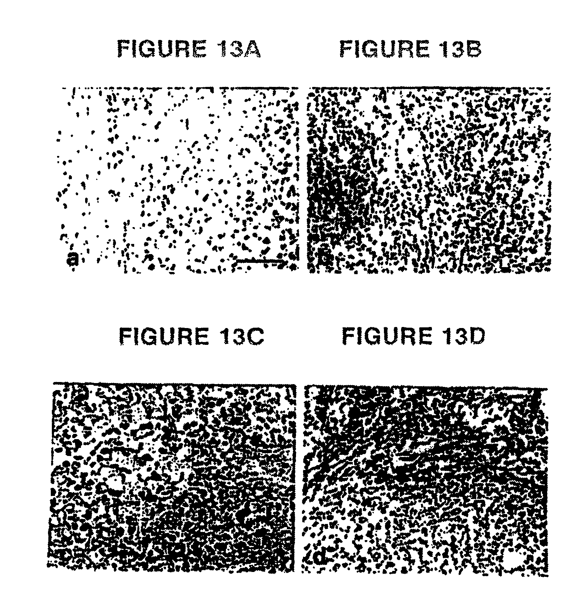

- Figures 13A - 13F: Immunohistochemical analyses of tumor xenografts grown in BALB/c nu/nu mice.a. Parental SK-RC-29 human RC cells. (b,d,a nd f) SK-RC-29 cells carrying the DC/TKIL2 construct, or (c and e) SK-RC-29 cells carrying the N2/CMVIFNγ constructs, a and b. PBS substituted for mAB and H₂O₂ step omitted (staining for endogenous peroxidase): c and d, biotinylated rat mAB anti-mouse tissue macrophages: or e and f, biotinylated mouse mAB anti-HLA-DR. Note the impressive number of (b) peroxidase-positive host mononuclear cells, (d) predominantly macrophages in stroma surrounding SK-RC-29 cells carrying the DC/TKIL2 construct, but not (a) parental or (c) IFN-γ producing SK-RC-29 cells. Positive heterogenous immunostaining for HLA-DR of (e) IFN-γ producing SK-RC-29 cells but not of (f) SK-RC-29 cells releasing IL-2 Bar, 100 µm.

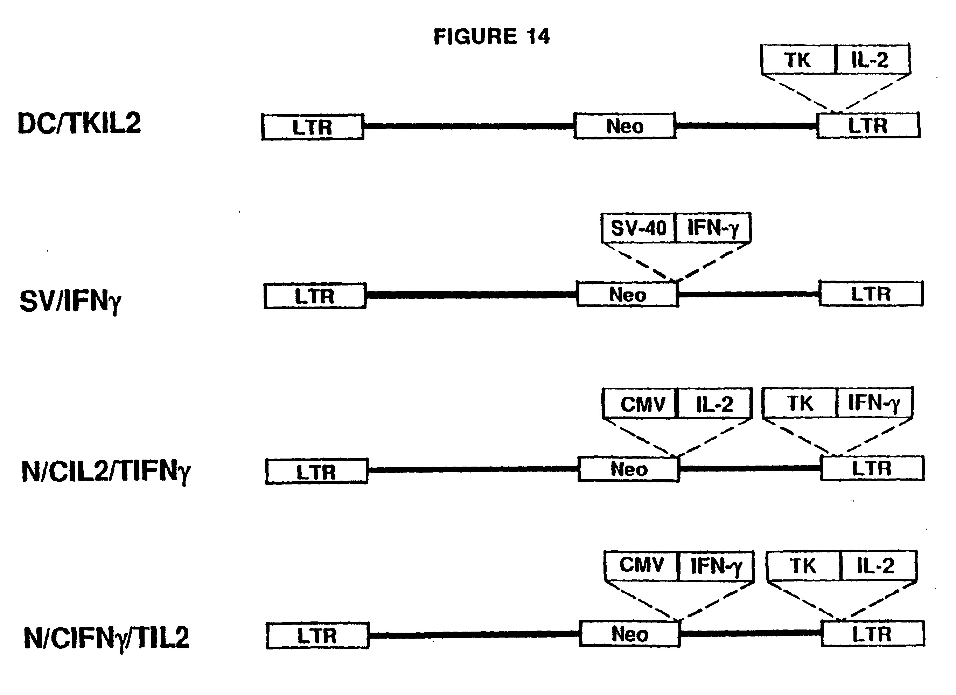

- Figure 14: Constructs showing two cytokines may be introduced into a retroviral vector.

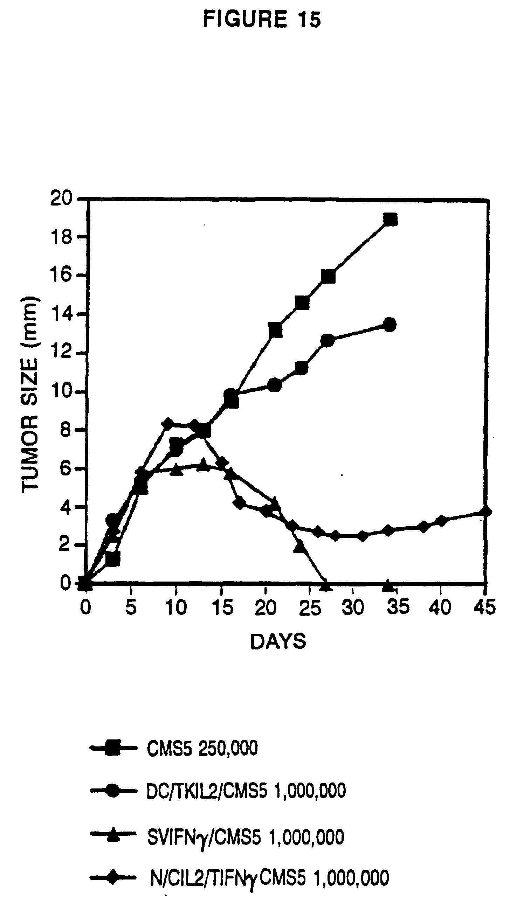

- Figure 15: Tumor growth of cytokine-secreting CMS5 cells in Balb/c mice.

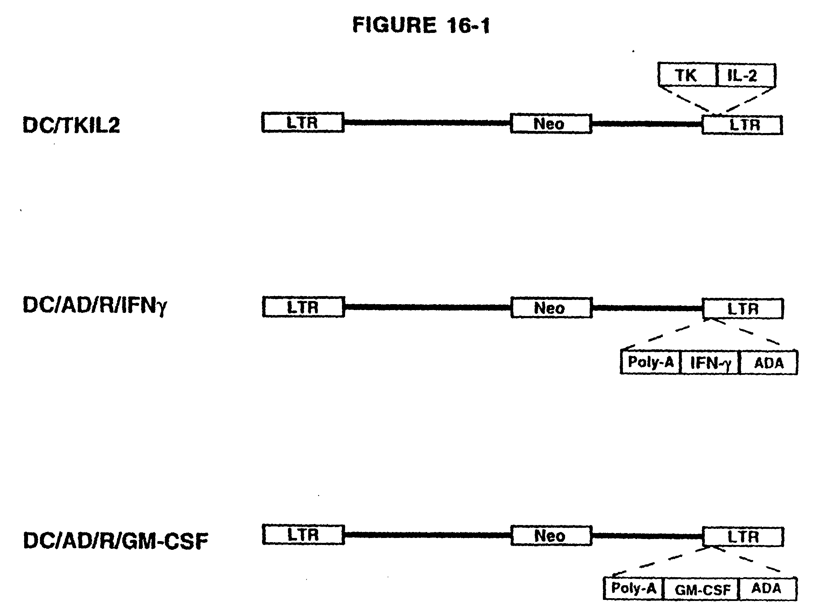

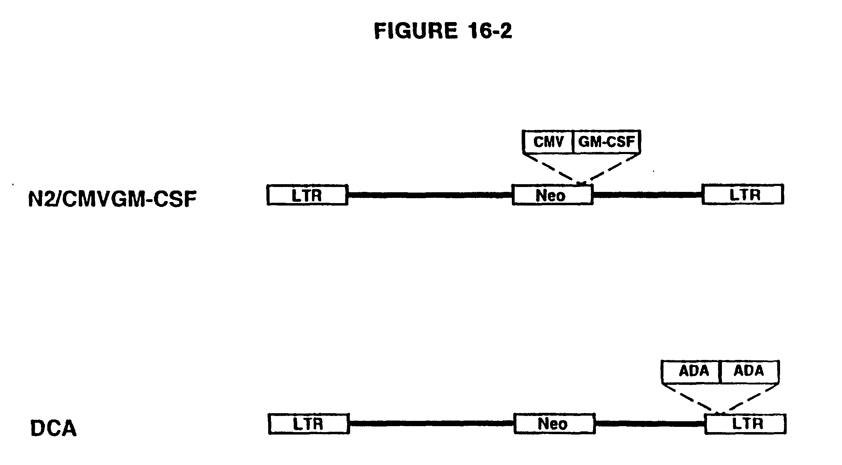

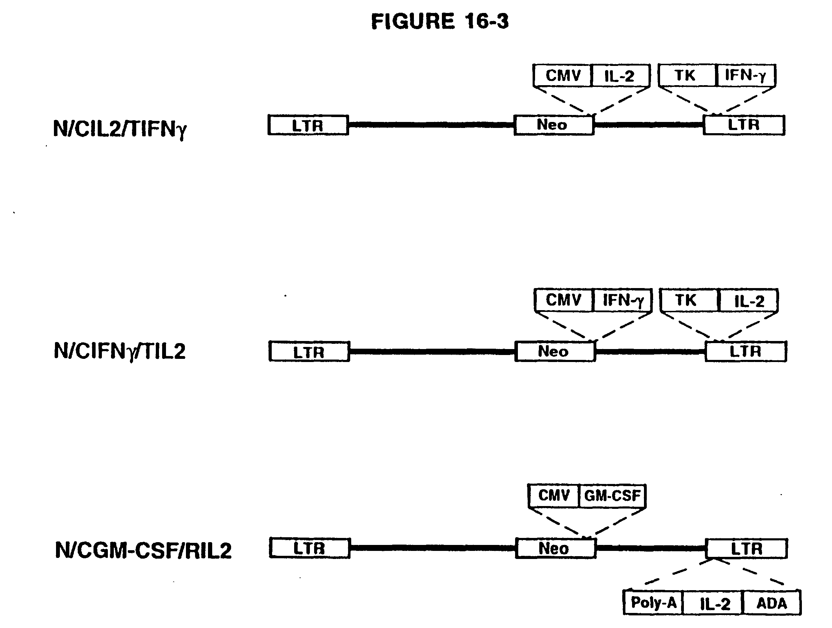

- Figures 16-1 - 16-3: Structures of retroviral vectors containing the human IL-2, mouse IFN-γ or mouse GM-CSF cDNA and of control vector containing the human ADA minigene (For additional details see Material and Method of the Six Series of Experiments).

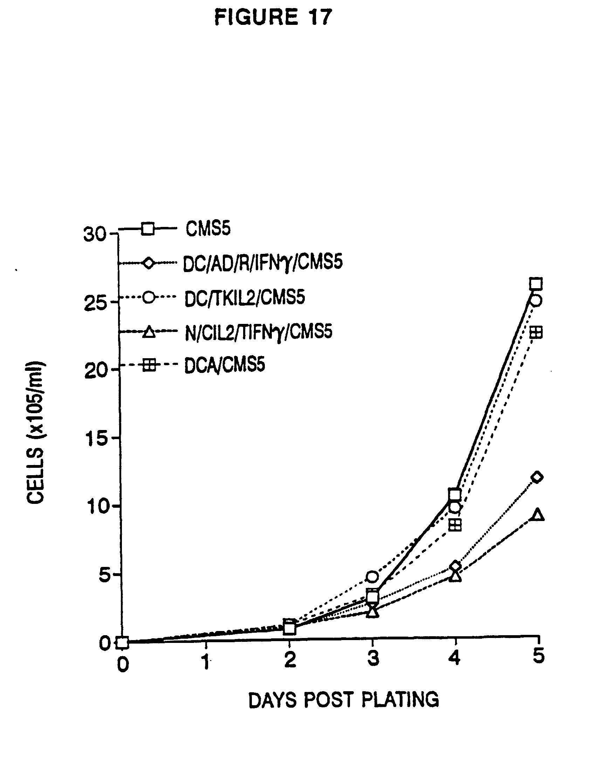

- Figure 17: Proliferation of parental and vector-transduced CMS-5 cells *in vitro*.

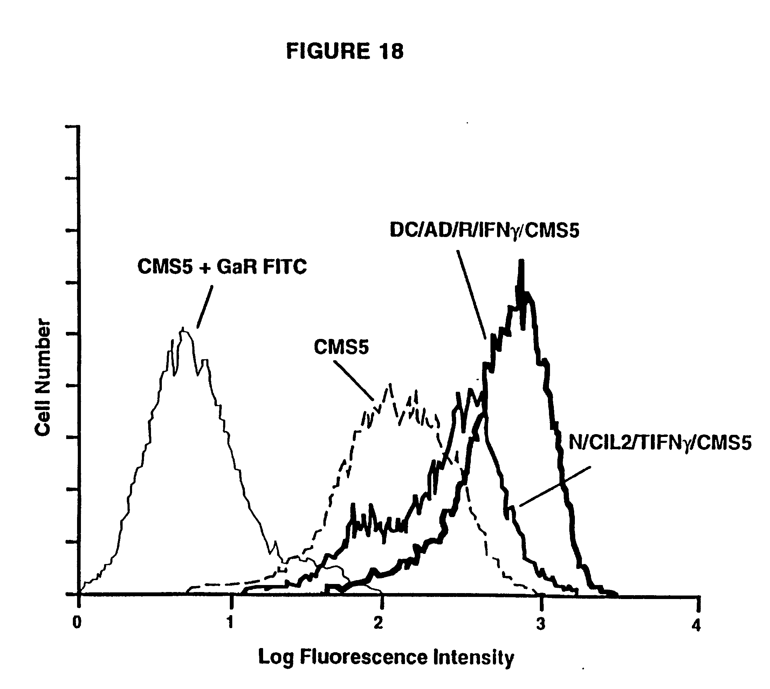

- Figure 18: Flow cytometric analysis of MHC expression on parental and cytokine-secreting CMS-5 cells. Cells were labelled with anti-MHC class I or II monoclonal antibodies, followed by incubation with FITC-conjugated goat anti-rat IgG and analysis by FACS. MHC class I expression of IFN-γ and IL-2/IFN-γ secreting CMS-5 cells was unregulated, whereas no upregulation of MHC class II expression (data not shown) was seen on transduced cells.

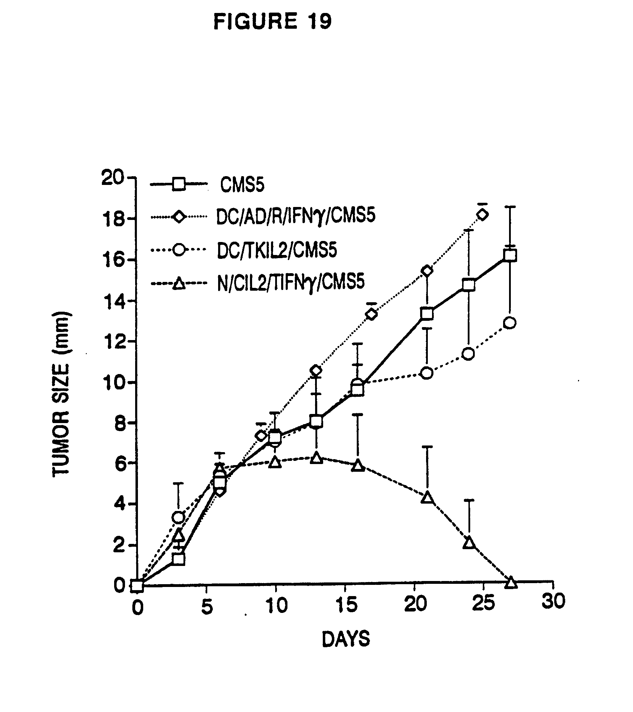

- Figure 19: Growth of parental and cytokine secreting CMS-5 clones in BALB/c mice. Groups of three mice in each category were injected with 2.5 x 10⁵ parental or 1 x 10⁶ transduced tumor cells as indicated. Results are expressed as the mean diameter (in millimeters) of tumors. Error bars represent the standard deviation of the mean. Tumor growth was monitored as described in Material and Method.

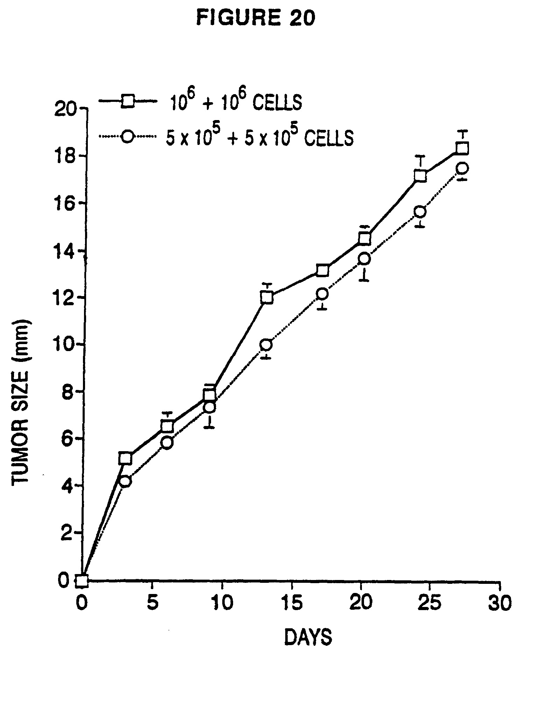

- Figure 20: Growth of a mixture of IL-2 and IFN-γ secreting CMS-5 clones in BALB/c mice. One group of BALB/c mice was injected with a mixture of 5 x 10⁵ CD/TKIL2/CMS5 cells and 5 x 10⁵ DC/AD/RIFNγ/CMS5 cells, another group was injected with 1 x 10⁶ DC/TKIL2/CMS5 cells and 1 x 10⁶ DC/AD/RIFNγ/CMS5 cells, as indicated. Results are expressed as the mean diameter (in millimeters) of tumors. Error bars represent the standard deviation of the mean.

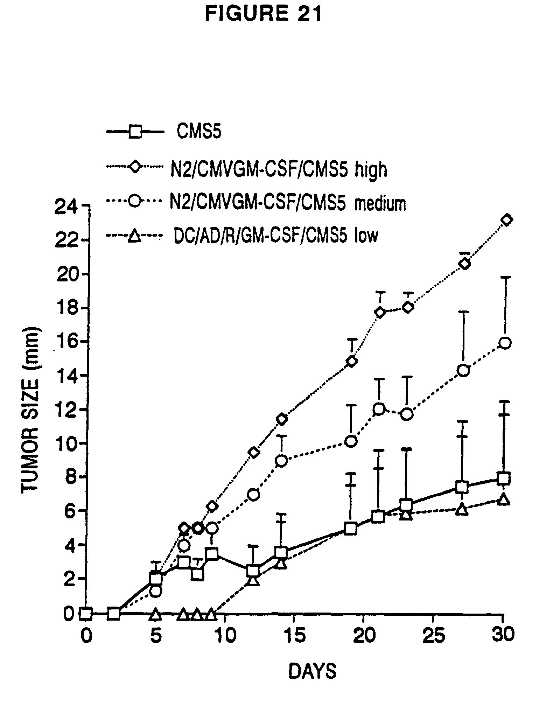

- Figure 21: Growth of CMS-5 bulk-transduced cells secreting high, medium or low amounts of GM-CSF. BALB/c mice were injected with 2.5 x 10⁵ GM-CSF transduced CMS-5 tumor cells or parental CMS-5 cells and tumor growth monitored. Results are expressed as the mean diameter (in millimeters) of tumors. Error bards represent the standard deviation of the mean.

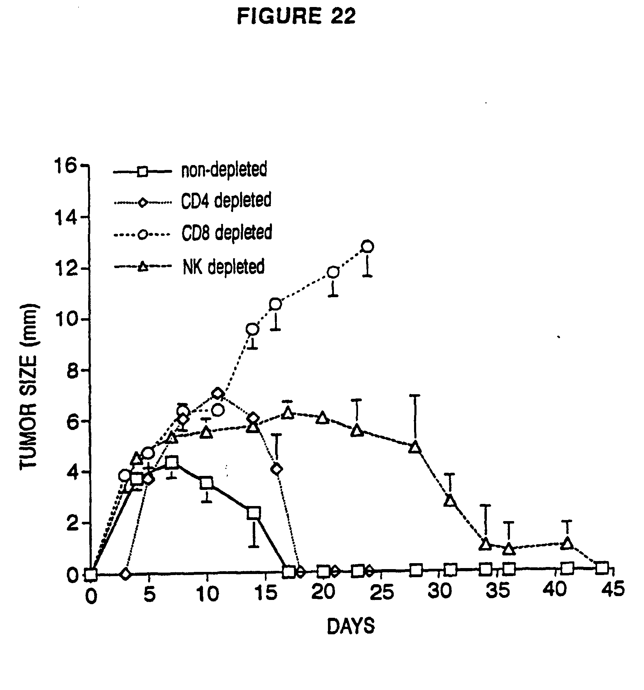

- Figure 22: Growth of IL-2/IFN-γ secreting CMS-5 cells in non-depleted and effector cell depleted BALB/c mice. Groups of three mice in each category were depleted of indicated effector cell subpopulations by i.p. injection with the corresponding antibody and injected with 1 x 10⁶ N/CIL2/TIFNγ/CMS5 cells. Similar results were obtained in another experiment. (For additional details see Material and Methods).

### DETAILED DESCRIPTION OF THE INVENTION

This invention provides a retroviral vector comprising (a) a 3'LTR; and (b) an insert having (i) a promoter, (ii) a sequence encoding an immunomolecule selected from the group consisting of cytokines, adhesion molecules, costimulatory factors, tumor associated antigens and tumor specific antigens, and (iii) a poly A signal, respectively, said promoter, sequence encoding an immunomolecule and poly A signal being aligned in a 5' to 3' orientation, wherein the insert is positioned within the 3'LTR in the orientation opposite to that of the 3'LTR.

In this invention, "immunomolecule" means molecules which can affect the immune system. One example of such molecule is cytokine. Another example of such molecule is the tumor associate or specific antigens.

Cytokines are biological molecules well known to a person of ordinary skill in the art. Examples of cytokines are interleukins which are secreted by lymphocytes. Other cytokines include, but not limited to interferons. Examples of the interferons are interferon alpha, interferon beta and interferon gamma.

Adhesion molecules are also well known to a person of ordinary skill in the art. Endothelial adhesion molecules have been identified as structures that aid in localization and attachment of leukocytes to blood vessel walls. Normally, neutrophils, monocytes, and lymphocytes are freely circulating throughout the body. But when tissue injury or injection occurs, the leucocytes need a mechanism to adhere closely to the site of inflammation.

As such, the adhesion molecules are expressed on endothelial cell surfaces in the vicinity of tissue inflammation.

A number of distinct adhesion molecule structures have been identified and characterized. The list includes endothelial leukocyte adhesion molecule (ELAM), intercellular adhesion molecule (ICAM), platelet endothelial cell adhesion molecule (PECAM), and vascular cell adhesion molecule (VCAM).

Costimulatory factors are well known in the art. The significance of the costimulation is well known (reviewed in Schwartz, 1992). An example of the costimulatory factor is B7 (Chen L. et al., 1992).

In an embodiment, this invention provides an allogeneic vaccine comprises a genetically manipulated cell which expresses at least one immunomolecule selected from the group consisting of cytokine, adhesion molecule, costimulatory factor, tumor associated antigen and tumor specific antigen, wherein the immunomolecule is a costimulatory factor. In a further embodiment, the costimulatory factor is B7/BB1 (CD80) or B70/B7-2 (Clark, E.A., et al. (Feb. 1994) Nature, 367:425-428.

Both tumor associated and tumor specific antigens are well known in the art. Tumor associated antigens are not only present in one kind of tumor. One example of the tumor associated antigen is Melanoma Associated Antigen E-1 (MAGE-1). MAGE-1 is not only found on melanoma but also breast carcinoma. However, tumor specific antigens are just found on the tumor cells.

This invention further provides the above described retroviral vector, wherein the promoter is derived from the group consisting of ADA gene, TK gene and CMV gene. This invention is not only limited to the promoters derived from ADA, TK and CMV genes. Various promoter sequences are known to a person of ordinary skill in the art and may be used in the claimed invention.

In one embodiment, the promoter is derived from the ADA gene. In another embodiment, the immunomolecule is a cytokine. In a further embodiment, the cytokine is interleukin. In another embodiment, the cytokine is interleukin-2.

This invention further provide a retroviral vector comprising (a) a 3'LTR; and (b) an insert having (i) a promoter, (ii) a sequence encoding an immunomolecule selected from the group consisting of cytokines, adhesion molecules, costimulatory factors, tumor associated antigens and tumor specific antigens, and (iii) a poly A signal, respectively, said promoter, sequence encoding an immunomolecule and poly A signal being aligned in a 5' to 3' orientation, wherein the insert is positioned within the 3'LTR in the orientation opposite to that of the 3'LTR, wherein the promoter sequence is derived from the ADA gene, the sequence encoding an immunomolecule which is interleukin-2. In a preferred embodiment, the retroviral is designated DC/AD/R/IL2 and the IL2 gene is contained in the DC/AD/R/IL2.

This invention provides a mammalian retroviral producer cell which comprises a retroviral packaging cell and a retroviral vector comprising (a) a 3'LTR; and (b) an insert having (i) a promoter, (ii) a sequence encoding an immunomolecule selected from the group consisting of cytokines, adhesion molecules, costimulatory factors, tumor associated antigens and tumor specific antigens, and (iii) a poly A signal, respectively, said promoter, sequence encoding an immunomolecule and poly A signal being aligned in a 5' to 3' orientation, wherein the insert is positioned within the 3'LTR in the orientation opposite to that of the 3'LTR.

In one embodiment, the above described mammalian retroviral producer cell comprises the retroviral vector is DC/AD/R/IL2.

In another embodiment, the retroviral packaging cell is AM12. In a further embodiment, the above described mammalian retroviral producer cell is designated AD/IL2/AM12 #12 (DC/AD/R/IL2/AM12 #12, ATCC Designation No. CRL 11210) comprising the retroviral vector DC/AD/R/IL2 and packaging cell AM12.

This cell line, AD/IL2/AM12 #12 (DC/AD/R/IL2/AM12 #12) was deposited with the American Type Culture Collection (ATCC), 12301 Parklawn Drive, Rockville, Maryland 20852, U.S.A. on December 1, 1992 under the provisions of the Budapest Treaty for the International Recognition of the Deposit of Microorganism for the Purposes of Patent Procedure. The cell line, AD/IL2/AM12 #12 (DC/AD/R/IL2/AM12 #12) was accorded ATCC designation number CRL 11210.

This invention further provides tumor cell which is infected by the retrovirus produced by the above described mammalian retroviral producer cell.

This invention also provides a tumor cell which is infected by the retrovirus produced by the mammalian retroviral producer cell designated AD/IL2/AM12 #12.

Other tumor cells could be infected by viruses generated by the above described mammalian retroviral producer cell. In one embodiment, a tumor cell is selected from the group consisting of melanoma cells, renal carcinoma cells, breast tumor cells and brain tumor cells.

In another embodiment, the infected tumor cell is derived from a melanoma. In a preferred embodiment, the tumor cell is designated SK-MEL-29/AD/IL2 (SK-MEL-29/DC/AD/R/IL2, ATCC Designation No. CRL 11208).

This cell line, SK-MEL-29/AD/IL2 (SK-MEL-29/IL2) was deposited with the American Type Culture Collection (ATCC), 12301 Parklawn Drive, Rockville, Maryland 20852, U.S.A. on December 1, 1992 under the provisions of the Budapest Treaty for the International Recognition of the Deposit of Microorganism for the Purposes of Patent Procedure. The cell line, SK-MEL-29/AD/IL2 was accorded ATCC designation number CRL 11208.

This invention further provides an infected tumor cell, wherein the tumor cell is derived from a renal carcinoma. In one embodiment, the tumor cell is designated SK-RC-28/AD/IL2 (SK-RC-28/DC/AD/R/IL2, ATCC Designation No. CRL 11209).

This cell line, SK-RC-28/AD/IL2 (SK-RC-28/DC/AD/R/IL2) was deposited with the American Type Culture Collection (ATCC), 12301 Parklawn Drive, Rockville, Maryland 20852, U.S.A. on December 1, 1992 under the provisions of the Budapest Treaty for the International Recognition of the Deposit of Microorganism for the Purposes of Patent Procedure. The cell line, SK-RC-28/AD/IL2 was accorded ATCC designation number CRL 11209.

In another embodiment, the tumor cell is designated SK-RC-39/AD/IL2 (SK-RC-39/DC/AD/R/IL2, ATCC Designation No. CRL 11210).

This cell line, SK-RC-39/AD/IL2 (SK-RC-39/DC/AD/R/IL2) was deposited with the American Type Culture Collection (ATCC), 12301 Parklawn Drive, Rockville, Maryland 20852, U.S.A. on December 1, 1992 under the provisions of the Budapest Treaty for the International Recognition of the Deposit of Microorganism for the Purposes of Patent Procedure. The cell line, SK-RC-39/AD/IL2 was accorded ATCC designation number CRL 11210.

This invention provides a genetically manipulated tumor cell useful for treating or preventing a alignant tumor in a patient which (a) expresses at least one immunomolecule selected from the group consisting of cytokines, adhesion molecules, costimulatory factors, tumor associated antigens and tumor specific antigens and (b) is allogenic to the patient.

This invention provides a method of treating a malignant tumor in a subject which comprises administering to the subject a plurality of a genetically manipulated cell which (a) expresses at least one immunomolecule selected from the group consisting of cytokines, adhesion molecules, costimulatory factors, tumor associated antigens and tumor specific antigens and (b) is allogeneic relative to the subject, so as to inhibit proliferation of the malignant tumor.

In this invention, "genetically manipulation" is defined as modification of the existing genetic makeup which are present in the cell by inserting exogenous genetic material. The methods to achieve such modification are well known in the art. For example, exogenous DNA material may be introduced into the cell by calcium phosphate precipitation technology. Other technologies such as the retroviral vector technology, electroporation, lipofection, other viral vector systems such as adeno-associated virus system, or microinjection may be used. The result of such genetically manipulation is that the genetically manipulated cell would now be able to express certain gene product which was not expressed before.

The term "allogeneic" means from the same species. Allogeneic cells are cells derived from the same species but antigenically distinct. In an embodiment, cells chosen for the uses of this invention will match some or all Human Lymphocytic Antigen (HLA) with the vaccinated patients. Different degree of matching may be used. For example, since there are class I and II of the HLA molecules and within class I, there are subclasses A, B and C, the match may be any, one, or all of the subclass, and within class II, there are DR, DP and DQ, the match may be any, one or all of these subclass. A person of ordinary skill in the art will be able to design experiments to test which matching will give optimal effects. One of such experiment is to inoculate different groups of animals/patients with different degrees of matching and then challenge with the tumor to examine the effect. Alternatively, the test may be done to tumor patients.

This invention further provides the above described method, wherein the genetically manipulated cell is a nonprofessional antigen presenting cell.

In this invention, the nonprofessional antigen presenting cells are defined as cells which are normally not used to present antigen to generate immune response against that antigen. Examples of non professionally antigen presenting cells are tumor cells and fibroblast. The non professional antigen presenting cells are well known in the art.

The nonprofessional antigen presenting cells may include, but not limited to other tumor cells such as cells derived from prostate cancers, lymphoma, colon carcinoma and bladder cancers.

This invention also provides the above described method wherein the genetically manipulated cell is a nonprofessional antigen presenting cell which is a tumor cell. In an embodiment, the tumor cell is derived from a melanoma. In another embodiment, the melanoma cell is SK-MEL-29.

This invention further provides a method of treating a malignant tumor in a subject which comprises administering to the subject a plurality of a genetically manipulated cell which (a) expresses at least one immunomolecule selected from the group consisting of cytokines, adhesion molecules, costimulatory factors, tumor associated antigens and tumor specific antigens and (b) is allogeneic relative to the subject, so as to inhibit proliferation of the malignant tumor wherein the immunomolecule is a cytokine which is interleukin-2.

In a preferred embodiment, the genetically manipulated cell is designated SK-MEL-29/AD/IL2 (SK-MEL-29/DC/AD/R/IL2, ATCC Designation No. CRL 11208).

In another embodiment, the genetically manipulated cell may be derived from SK-MEL-131.

In an embodiment, the immunomolecule is cytokine which is interferon gamma.

In a further embodiment, the genetically manipulated cell is designated DC/TKHIFNγ/MEL131 (SK-MEL-131/DC/TK/IFNγ, ATCC Designation No. CRL 11255).

This cell line, DC/TKHIFNγ/MEL131 (SK-MEL-131/DC/TKIFNγ) was deposited with the American Type Culture Collection (ATCC), 12301 Parklawn Drive, Rockville, Maryland 20852, U.S.A. on February 2, 1993 under the provisions of the Budapest Treaty for the International Recognition of the Deposit of Microorganism for the Purposes of Patent Procedure. The cell line, DC/TKHIFNγ/MEL-131 was accorded ATCC designation number 11255.

In another embodiment, the tumor cell is derived from a renal carcinoma. In a further embodiment, the renal carcinoma cell is SK-RC-28.

This invention provides the above described method wherein the immunomolecule is a cytokine which is interleukin-2.

In one embodiment of the above described method, the genetically manipulated cell is designated SK-RC-28/AD/IL2 (SK-RC-28/DC/AD/R/IL2, ATCC Designation No. CRL 11209).

In another embodiment, the renal carcinoma cell is SK-RC-39. In a further embodiment, the immunomolecule is a cytokine which is interleukin-2.

In a preferred embodiment of the above described method, the genetically manipulated cell is designated SK-RC-39/AD/IL2 (SK-RC-39/DC/AD/R/IL2, ATCC Designation No. CRL 11210).

The above described method may be practiced on tumor cell derived from a breast tumor, bladder tumor and brain tumor.

This invention further provides the above described method wherein the immunomolecule is a interleukin-2 and the interleukin-2 is the interleukin-2 contained in DC/AD/R/IL2.

This invention further provides the above described method, wherein the cytokine is an interferon. In one embodiment, the interferon is interferon gamma. In another embodiment, the cytokine is GM-CSF. In a further embodiment, the genetically manipulated cell expresses both interleukin and interferon. In a still further embodiment, the genetically manipulated cell expresses both interleukin-2 and interferon gamma. In another embodiment, the genetically manipulated cell expresses either a tumor associate antigen or a tumor specific antigen or both and at least one cytokine.

This invention further provides the above described method wherein the immunomolecule is a costimulatory factor which is B7/BB1 (CD80) or B70/B7-2 (Clark, E.A., et al. (Feb. 1994) Nature, 367:425-428). In another embodiment of the above described method wherein, the tumor associated antigen is melanoma associated antigen E-1.

In one embodiment, the tumor cell is derived from melanoma. In another embodiment, the tumor cell is derived from renal carcinoma. In a further embodiment, the tumor cell is derived from breast cancer. In another embodiment, the tumor cell is derived from brain cancer.

In a preferred embodiment, the cytokine used is GM-CSF.

This invention provides a method of treating a malignant tumor in a subject which (a) comprises administering to the subject a plurality of a genetically manipulated cell which expresses at least one immunomolecule selected from the group consisting of cytokines, adhesion proteins, costimulatory factors, tumor associated antigens and tumor specific antigens, and (b) is allogeneic relative to the subject, so as to inhibit proliferation of the malignant tumor wherein the genetically manipulated cell is a professional antigen presenting cell.

In this invention, the professionally antigen presenting cell means cells which will normally present antigen to generate an immune response. An example of such cells are the dendritic cells like Langerhans cells or macrophages (Vidard et al. 1992).

This invention also provides a method of treating a malignant tumor in a subject which comprises administering to the subject a plurality of a genetically manipulated cell which (a) expresses at least one immunomolecule selected from the group consisting of cytokines, adhesion molecules, costimulatory factors, tumor associated antigens and tumor specific antigens and (b) is allogeneic relative to the subject, so as to inhibit proliferation of the malignant tumor, wherein the genetically manipulated cell is a professionally antigen presenting cell such as normal B cell, peritoneal macrophage and other splenic antigen-presenting cells.

This invention also provides a method of treating a malignant tumor in a subject which comprises administering to the subject a plurality of a genetically manipulated cell which (a) expresses at least one immunomolecule selected from the group consisting of cytokines, adhesion molecules, costimulatory factors, tumor associated antigens and tumor specific antigens and (b) is allogeneic relative to the subject, so as to inhibit proliferation of the malignant tumor, wherein the genetically manipulated cell is a professional antigen presenting cell and the cytokine is an interleukin. One example of such interleukin is interleukin-2.

This invention also provides a method of treating a malignant tumor in a subject which comprises administering to the subject a plurality of a genetically manipulated cell which (a) expresses at least one immunomolecule selected from the group consisting of cytokines, adhesion molecules, costimulatory factors, tumor associated antigens and tumor specific antigens and (b) is allogeneic relative to the subject, so as to inhibit proliferation of the malignant tumor, wherein the genetically manipulated cell is a professional antigen presenting cell and the cytokine is an interferon. One example of such interferon is interferon gamma.

This invention also provides a method of treating a malignant tumor in a subject which comprises administering to the subject a plurality of a genetically manipulated cell which (a) expresses at least one immunomolecule selected from the group consisting of cytokines, adhesion molecules, costimulatory factors, tumor associated antigens and tumor specific antigens and (b) is allogeneic relative to the subject, so as to inhibit proliferation of the malignant tumor, wherein the genetically manipulated cell is a professional antigen presenting cell and expresses both interleukin and interferon. In one embodiment, the genetically manipulated cell expresses both interleukin-2 and interferon gamma. In another embodiment, the genetically manipulated cell expresses either tumor associate antigen or tumor specific antigen or both and at least one cytokine.

Different effective amounts of the genetically manipulated cells may be used according to this invention. A person of ordinary skill in the art can perform simple titration experiments to determine what the effective amount is required for effective immunization. An example of such titration experiment is to inject different amounts of the allogeneic vaccine to patient and then examine the immune response.

In a preferred embodiment of the above-described method, about ten to fifty million genetically manipulated cells are administered to the subject.

It is well known to a person of ordinary skill in the art that other side effects will be taken into consideration when determining the effective amount of reagents.

For the purposes of this invention "pharmaceutically acceptable carrier" means any of the standard pharmaceutical carrier. Examples of suitable carrier are well known in the art and may include, but not limited to, any of the standard pharmaceutical vehicles such as a phosphate buffered saline solutions, phosphate buffered saline containing Polysorb 80, water, emulsions such as oil/water emulsion, and various type of wetting agents.

The genetically manipulated cells of this invention may be administered intradermally, subcutaneously and intramuscularly. Other methods well known by a person of ordinary skill in the art may also be used.

This invention provides a method of preventing tumor formation in a subject comprising administering to the subject a plurality of a genetically manipulated cell which (a) expresses at least one immunomolecule selected from the group consisting of cytokines, adhesion molecules, costimulatory factors, tumor associated antigens and tumor specific antigens and (b) is allogeneic relative to the subject, so as to prevent the tumor formation.

This invention further provides a method of preventing tumor formation in a subject, wherein the genetically manipulated cell is a nonprofessional antigen present cell. In one embodiment, the nonprofessional antigen presenting cell is a tumor cell. In another embodiment, the tumor cell is derived from melanoma. In a further embodiment, the melanoma cell is SK-MEL-29.

This invention provides a method of preventing tumor formation, wherein the cytokine is interleukin-2.

This invention provides a method of preventing tumor formation, wherein the genetically manipulated cell is designated SK-MEL-29/AD/IL2 (SK-MEL-29/DC/AD/R/IL2, ATCC Designation No. CRL 11208).

This invention provides a method of preventing tumor formation, wherein the melanoma cell is SK-MEL-131.

This invention further provides a method of preventing tumor formation, wherein the interferon is interferon gamma.

In a preferred embodiment of this method of preventing tumor formation, the genetically manipulated cell is designated DC/TKHIFNγ, ATCC Designation No. CRL 11255).

This invention provides a method of preventing tumor formation, wherein the tumor cell is derived from renal carcinoma. In one embodiment, the renal carcinoma cell is SK-RC-28.

In a preferred embodiment of this method of preventing tumor formation, the genetically manipulated cell is designated SK-RC-28/AD/IL2 (SK-RC-28/DC/AD/R/IL2, ATCC Designation No. CRL 11209).

This invention provides a method of preventing tumor formation, wherein the renal carcinoma cell is SK-RC-39.

This invention provides a method of preventing tumor formation, wherein the cytokine is interleukin-2.

In an embodiment of this method of preventing tumor formation, the genetically manipulated cell is designated SK-RC-39/AD/IL2 (SK-RC-39/DC/AD/R/IL2, ATCC Designation No. CRL 11210).

In an embodiment of this method of preventing tumor formation, the tumor cell is derived from breast cancer or derived from brain cancer.

In an embodiment of this method of preventing tumor formation, wherein the interleukin-2 is the interleukin-2 contained in DC/AD/R/IL2.

In an embodiment of this claimed invention for preventing tumor formation, the cytokine is an interferon. In a further embodiment, the interferon is interferon gamma. In another embodiment, the cytokine is GM-CSF. In a further embodiment of this claimed invention for preventing tumor formation, the genetically manipulated cell expresses both interleukin and interferon. In a still further embodiment, the genetically manipulated cell expresses both interleukin-2 and interferon gamma.

In another embodiment of this claimed invention for preventing tumor formation, the genetically manipulated cell expresses either a tumor associate antigen or a tumor specific antigen or both and at least one cytokine.

In another embodiment of this claimed invention for preventing tumor formation, wherein the immunomolecule is a costimulatory factor which is B7/BB1 (CD80) or B70/B7-2 (Clark, E.A., et al. (Feb. 1994) Nature, 367:425-428).

In another embodiment, wherein the immunomolecule is a tumor associates antigen. In a further embodiment, the tumor associates antigen is melanoma associated antigen E-1.

This invention further provides a method of preventing tumor formation in a subject comprising administering to the subject a plurality of a genetically manipulated cell which (a) expresses at least one immunomolecule selected from the group consisting of cytokines, adhesion molecules, costimulatory factors, tumor associates antigens and tumor specific antigens and (b) is allogeneic relative to the subject, so as to prevent the tumor formation, wherein the genetically manipulated cell is a profession antigen presenting cell.

In one embodiment, the profession antigen presenting cell is a dendritic cell. In another embodiment, the cytokine expressed in the professional antigen presenting cell is either an interleukin or interferon. In a further embodiment, the interleukin is interleukin-2. In a still further embodiment, the interferon is interferon gamma.

In another embodiment, the genetically manipulated cell expresses both interleukin and interferon.

In one embodiment, the genetically manipulated cell expresses both interleukin-2 and interferon gamma. In another embodiment, the genetically manipulated cell expresses either tumor associate antigen or tumor specific antigen or both and at least one cytokine. In still another embodiment, about ten to fifty million genetically manipulated cells are administered to the subject.

This invention provides a method for making the genetically manipulated cells which comprises steps of a) introducing at least a gene coding for a immunomolecule selected from the group consisting of cytokine, adhesion molecule, costimulatory factor, tumor associated antigen and tumor specific antigen into cells; b) testing the cells for the expression of the introduced gene; and c) selecting the cells which express the introduced gene.

A person of ordinary skill in the art would be able to select appropriate preparation of genetically manipulated cells which are synthesized by introducing at least a gene coding for a immunomolecule selected from the group consisting of cytokine, adhesion molecule, costimulatory factor, tumor associated antigen and tumor specific antigen into cells and testing the introduced cells for the expression of the introduced gene. One criterion for such selection may be the number of cell which are expressing the introduced gene. Another criterion may be the amount of the expression of the introduced gene in the cells. Methods to determine the number of cells containing the introduced gene are well known in the art. Methods to quantitate expression of the gene in the cell are also well known.

The term "express" in this invention means not only stable expression but also transient expression. This invention is not limited to cells which can stably express at least one gene coding for a immunomolecule selected from the group consisting of cytokine, adhesion molecule, costimulatory factor, tumor associated antigen and tumor specific antigen but also cells which express the introduced gene or genes transiently.

This invention also provides a method for making a genetically manipulated cell for uses in treatment of malignant tumors or prevention of tumor formation which comprises steps of a) introducing at least a gene coding for a immunomolecule selected from the group consisting of cytokine, adhesion molecule, costimulatory factor, tumor associated antigen and tumor specific antigen into a cell, b) testing the introduced cell for the expression of the introduced gene; c) selecting cells which express the introduced gene and further comprising isolation of the cell which stably carries the introduced gene. Methods to isolate such cells are well known to a person of ordinary skill in the art.

This invention further provides the above describe method for making a genetically manipulated cell, wherein step a) further comprises: i.cloning at least one gene coding for an immunomoleucle selected from the group consisting of cytokine, adhesion molecule, a costimulatory factor, tumor associated antigen and tumor specific antigen of each gene into a retroviral vector, ii. transfecting the vector into a packaging cell generating a producer cell line which produces virus containing the cytokine or adhesion molecule gene or a costimulatory gene, and iii. transducing a cell with the virus produced in ii.

This invention further provides a method for making a genetically manipulated cell, wherein the vector is selected from a group consisting essentially of AD, DC and N2. Other retroviral vectors known in the art may be similarly used.

This invention also provides a method for making a genetically manipulated cell using the packaging cell AM12. Other packaging cells which are well known to a person of ordinary skill in the art could be used.

In a preferred embodiment, the vector used is AD and the cytokine is IL2. The producer line generated is called AD/IL2/AM12 #12 (DC/AD/R/IL2/AM12 #12).

In a another embodiment for this method for making a genetically manipulated cell, the vector used is DC and the cytokine is interferon gamma. The producer line generated is called DC/TKHIFHγ/AM12 #6 (DC/TKIFNγ/AM12 #6). This cell line, DC/TKHIFNγ/AM12 #6 (DC/TKIFNγ/AM12 #6) was deposited with the American Type Culture Collection (ATCC), 12301 Parklawn Drive, Rockville, Maryland 20852, U.S.A. on February 2, 1992 under the provisions of the Budapest Treaty for the International Recognition of the Deposit of Microorganism for the Purposes of Patent Procedure. The cell line, DC/TKHIFNγ/AM12 #6 (DC/TKIFNγ/AM12 #6) was accorded ATCC designation number CRL 11256.

In a another embodiment for this method for making a genetically manipulated cell, the vector used is N2 and the cytokine is interferon gamma. The producer line generated is called N2/CMVHIFNγ/AM12 #5 (N2/CMVIFNVγ/AM12 #5). This cell line, N2/CMVHIFNγ/AM12 #5 (N2/CMVIFNVγ/AM12 #5) was deposited with the American Type Culture Collection (ATCC), 12301 Parklawn Drive, Rockville, Maryland 20852, U.S.A. on February 2, 1993 under the provisions of the Budapest Treaty for the International Recognition of the Deposit of Microorganism for the Purposes of Patent Procedure. The cell line, N2/CMVHIFNγ/AM12 #5 (N2/CMVIFNVγ/AM12 #5) was accorded ATCC designation number CRL 11257.

In a another embodiment for this method for making a genetically manipulated cell, the vector used is N2 and the cytokine is Granulocyte Macrophage Colony Stimulating Factor (GM-CSF). The producer line generated is called N2/CMVHGM-CSF/AM12 #8. This cell line, N2/CMVHGM-CSF/AM12 #8 was deposited with the American Type Culture Collection (ATCC), 12301 Parklawn Drive, Rockville, Maryland 20852, U.S.A. on February 2, 1993 under the provisions of the Budapest Treaty for the International Recognition of the Deposit of Microorganism for the Purposes of Patent Procedure. The cell line, N2/CMVHGM-CSF/AM12 #8 was accorded ATCC designation number CRL 11258.

This invention provides a method for making a genetically manipulated cell which comprises steps of a) introducing at least one gene coding for an immunomolecule selected from the group consisting of cytokine, adhesion molecule, costimulatory factor, tumor associated antigen and tumor specific antigen into a cell; b) testing the cells for the expression of the introduced gene; and c) selecting the cells which express the introduced gene. The methods to introduce gene into a cell have been well known in the art and should not be construe to be limited to the above described method via a retroviral vector. Other methods such as transfection by calcium phosphate precipitation, lipofection, microinjection and other viral vector systems are included in this invention. In a preferred embodiment, the gene is introduced into the cell by electroporation.

This invention provides method for making a genetically manipulated cell which comprises steps of a)introducing at least one gene coding for an immunomolecule selected from the group consisting of cytokine, an adhesion molecule, costimulatory factor, tumor associated antigen and tumor specific antigen into cells; and b) testing the cells for the expression of the introduced gene; and c) selecting the cells which express the introduced gene wherein the cell is either a nonprofessional antigen-presenting cell or a professional antigen-presenting cell.

This invention further provides a method for making a genetically manipulated cell, wherein the nonprofessional antigen-presenting cells is a tumor cell. Tumor cells are well known to a person of ordinary skill in the art.

In preferred embodiments, the tumor cell is selected from a group consisting essentially of SK-MEL-29, SK-MEL-131, SK-RC-28 and SK-RC-39.

This invention provides method for making a genetically manipulated cell which comprises steps of a)introducing at least one gene coding for an immunomolecule selected from the group consisting of cytokine, an adhesion molecule, costimulatory factor, tumor associated antigen and tumor specific antigen into cells; and b) testing the cells for the expression of the introduced gene; and c) selecting the cells which express the introduced gene wherein the cytokine is selected from a group consisting essentially of IL2, interferon gamma and GM-CSF.

This invention provides a method for making a genetically manipulated cell vaccine which comprises steps of a) introducing at least a gene coding for a immunomolecule selected from the group consisting of cytokine, adhesion molecule, costimulatory factor, tumor associated antigen and tumor specific antigen into cells; b) testing the cells for the expression of the introduced gene; and c) selecting the cells which express the introduced gene, wherein the cells are either professional antigen presenting cells or nonprofessional antigen presenting cells and both an interleukin gene and an interferon gene are introduced into the cells. One such the interleukin gene is interleukin-2 gene and one such interferon gene is interferon gamma gene.

This invention provides a method for making a genetically manipulated cell which comprises steps of a) introducing at least a gene coding for a immunomolecule selected from the group consisting of cytokine, adhesion molecule, costimulatory factor, tumor associated antigen and tumor specific antigen into cells; b) testing the cells for the expression of the introduced gene; and c) selecting the cells which express the introduced gene, wherein the cells are either professional antigen presenting cells or nonprofessional antigen presenting cells and wherein either a tumor associate antigen or a tumor specific antigen or both and a cytokine gene are introduced into the cells.

This invention provides a method for making a genetically manipulated cell which comprises steps of a) introducing at least a gene coding for a immunomolecule selected from the group consisting of cytokine, adhesion molecule, costimulatory factor, tumor associated antigen and tumor specific antigen into cells; b) testing the cells for the expression of the introduced gene; and c) selecting the cells which express the introduced gene, wherein the cells are either professional antigen presenting cells or nonprofessional antigen presenting cells and wherein wherein the introduced gene codes for B7/BB1 (CD80) or B70/B7-2 (Clark, E.A., et al. (Feb. 1994) Nature, 367:425-428).

This invention provides a method for making a genetically manipulated cell which comprises steps of a) introducing at least a gene coding for a immunomolecule selected from the group consisting of cytokine, adhesion molecule, costimulatory factor, tumor associated antigen and tumor specific antigen into cells; b) testing the cells for the expression of the introduced gene; and c) selecting the cells which express the introduced gene, wherein the cells are either professional antigen presenting cells or nonprofessional antigen presenting cells and wherein the introduced gene codes for melanoma associated antigen E-1.

In this invention, the word "vaccine" is used in this context as the antigen source for activating of immune responses against established tumors, and for prophylactic immunization. Therefore, the genetically manipulated cells claimed in this invention may be called vaccine.

This invention will be better understood from the Experimental Details which follow. However, one skilled in the art will readily appreciate that the specific methods and results discussed are merely illustrative of the invention as described more fully in the claims which follow thereafter.

### EXPERIMENTAL DETAILS

### First series of ezperiments

### RETROVIRAL GENE TRANSFER INDUCED CONSTITUTIVE EXPRESSION OF IL-2 OR IFN-γ IN IRRADIATED HUMAN MELANOMA CELLS

The results of this series of experiments demonstrate that melanoma cells stably secrete these cytokines following retroviral mediated gene transfer. In addition, lymphokine secretion was maintained following irradiation of the cells, an important precaution when these cells are injected in vivo in clinical trials. Finally, these cytokines demonstrated a biological effect in vitro and in vivo in nude mice. These findings provide a logical basis for designing approaches for active immunotherapy in humans.

### MATERIALS AND METHODS

### Cell lines and Tissue culture

Five human melanoma cell lines were selected for this study. The cell lines SK-MEL-19,-29,-131,-245,-256 were established from primary tumor specimens or metastatic lesions and were obtained from a cell bank (Houghton et al., 1982; Houghton et al., 1984; Houghton et al., 1987. Cell lines were cultured in Eagle's Minimum Essential Medium (MEM), 2 mM glutamine, 1% nonessential amino acids, 100 U/ml penicillin, and 100 µg/ml streptomycin supplemented with 10% fetal bovine serum (FBS). Cultures were regularly tested for mycoplasma.

### Retroviral Vector Design and Tumor Cell Infection

The retroviral vectors N2/IL2, DC/TK/IL2, N2/CMVIL2, and DCA used in this project are identical to the ones used in the previous murine fibrosarcoma tumor model (Gansbacher et al., 1990b). DC/AD/R/IL2 was generated by cloning a minigene containing the human ADA promoter, the human IL-2 cDNA and the poly-A sequences into the 3' LTR in reverse orientation. The ADA promoter is a 832 bp fragment derived from the human ADA gene by digestion with restriction enzymes SspI and NcoI and was cloned in reverse orientation into the Klenowed Mlu I site. The poly-A signal containing a 275 bp long SmaI-AluI fragment was derived from plasmid PBC/CMV/IL2 and cloned into the Klenowed ApaI site of the 3'LTR polylinker (Cullen et al., 1988). The human IL2 cDNA used for this construct was cloned by PCR from peripheral lymphocytes of a healthy volunteer. Sequencing of the IL-2 cDNA revealed a polymorphic site in position 456 (G for A), that does not lead to a change in the amino acid sequence. Retroviral vector constructs were converted to corresponding virus using established procedures. Briefly, vector DNA (N2/IL2, DC/TKIL2, DC/TKIFNγ, N2/CMVIL2, N2/CMVINFγ, DCA and DC/AD/R/IL2) was transfected into a helper free, amphotropic packaging cell line (Markowitz et al., 1988; Markowitz et al., 1988). Colonies were isolated by G418 selection, expanded to cell lines and cell free supernatant was tested for the presence of virus. Cell lines secreting high titer of virus (10⁵ -10⁶ Neo colony forming units per ml) were used to infect melanoma cell lines. Bulk and clonal derivates of melanoma cells were isolated by G418 selection, expanded to cell lines and secretion of IL-2 or IFH-γ into the cell supernatant was measured. Absence of replication competent virus in the cytokine producing melanoma cells was shown by the inability to transfer G418 resistance to NIH 3T3 cells and by a negative reverse transcriptase assay in the HeLa and NIH3T3 amplification assay.

### Southern and Northern Blotting

High molecular weight DNA was prepared from the tumor cells by standard techniques involving sodium dodecyl sulfate (SDS), protein kinase digestion, phenol extraction, and ethanol precipitation. Each analysis was performed on 10 µg of DNA. The samples were electrophoresed and transferred to a Nylon filter (Biotrans, ICN). Filters were hybridized using the NeoR probe labelled by a random primer method (Feinberg et al., 1983). After hybridization the filters were washed and exposed to Kodak XAR x-ray films.

Poly A RNA was prepared from total cellular RNA using oligo(dT)-cellulose columns. The RNA was size fractionated on a 1% agarose gel containing formaldehyde, transferred to a nylon filter (Biotrans, ICN) and UV-crosslinked to the filter. Hybridization was performed for 24 hours as previously described (Gansbacher et al., 1989), using a cDNA probe for U3 labelled by the random primer method (Feinberg et al., 1983). The filter was washed under stringent conditions, blotted dry, wrapped in Saran Wrap and exposed at -70°C to Kodak XAR X-ray film with an intensifying screen.

### IL-2 Assay

Supernatants from 2x10⁶ semiconfluent unmodified or IL-2 secreting cells were collected after 24- 48 hours and assayed for human IL-2. Preparations of IL-2 were tested for biologic activity by assessing their ability to induce incorporation of ³H-thymidine (TdR) into the DNA of IL-2 dependent cultured T cells (CTC) in a proliferation assay as previously described (Zier, 1982). Units are given in Cetus Units. Results were confirmed by immunoassay using an ELISA kit (Genzyme). Briefly, fifty thousand CTC were mixed with serial dilutions of the test samples and cultured at 37° in 5% CO₂. After approximately 60 hours the cultures were pulsed with 1 µCi ³H-TdR (New England Nuclear, specific activity 6.7 Ci/µM) for the final 12 hours of growth.

### Human IFN-γ assay

Supernatants were analyzed for IFN-γ in a bioassay based on the antiviral activity in the cell culture supernatant, as determined by the reduction of the cytopathic effects of vesicular stomatitis virus on WISH cells (Rubinstein et al., 1987). Results were confirmed using a commercially available radioimmunoassay (RIA) for human IFN-γ (Centocor, Malvern, PA). Human IFN-γ bioactivity in the supernatant was blocked by a neutralizing murine monoclonal antibody specific for human IFN-γ (Olympus Corp., Lake Success, NY).

### Flow cytometry

Two hundred thousand cells were resuspended in phosphate buffered saline (PBS) containing 2% FBS, 0.02% sodium azide, and distributed into microtiter wells. The cells were centrifuged and the supernatant was poured off. One hundred µl per 10⁶ cells of diluted monoclonal antibody W6/32, directed against a monomorphic determinant on the MHC class I molecule, was then added and the cells incubated on ice for 30 minutes. The cells were washed three times with 100 µl of cold PBS as described above and, after gently breaking up the cell pellet, 100 µl/10⁶ of fluorescein isothiocyanate (FITC)-conjugated goat anti mouse-IgG were added to each sample. The cells were incubated on ice, in the dark, for another 30 minutes and washed as described above. They were then immediately analyzed for percent fluorescence with an Epics C cytometer equipped with logarithmic amplifiers for fluorescence measurements. Fluorescence originating from dead cells was eliminated by threshold gating of forward and 90-degree light scatter signals (Gansbacher et al., 1986).

### Irradiation

Parental and lymphokine secreting melanoma cells were irradiated at room temperature with a 6 MV Varian CL6-100 linear accelerator at a dose-rate of 100 rad/min and then plated in several 60mm dishes at identical concentrations. Irradiated cells were cultured at 37°C in 5% CO2 atmosphere. Cells were refed with 3ml/dish of fresh RPMI containing 10% FCS every 3-4 days and supernatants collected at the indicated time points. Spent medium was frozen at -20°C until assaying. After supernatant collection cells were trypsinized and counted.

### Replication Competent Murine Retrovirus Detection Assay

One ml of the experimental sample was mixed with 3 ml of DMEM, 10% FCS, 5mM glutamine and polybrene 8 µg/ml and then added to a 60 mm plate containing 10⁵ NIH3T3 cells passaged 12 hours previously. Infected NIH3T3 cells were cultured for 21 days, passaging the cells at a 1:5 dilution every 3 days. One half of this cell population was assayed for G418 resistance the other sample was used for collection of supernatant for determination of reverse transcriptase activity. Reverse transcriptase activity was determined by incubating 10 µl of sample media with 40 µl 50 mM Tris pH 8.0, 20 mM DTT, 0.6 mM MnCl₂, 60 mM NaCl, 0.05% NP, 5µg/ml oligo dT, 10 µg/ml polyA, 62 µM dTTP, 10 µM P³² dTTP (3000 Ci/mmol) for one hour at 37°C. Medium alone was used as negative control to determine background activity. Ten µl of the reaction mixture was then spotted on a DEAE DE81 filter disc, washed 2X with SSC at 25°C for 2x 15 minutes, rinsed with 95% ethanol and air dried. Filter radioactivity was measured by exposure of the X-ray film for 3-5 hours and quantitated by scintillation counting.

### RESULTS

### Generation of Tumor Cell Lines Expressing IL-2 or IFN-γ

Figure 1 shows the retroviral vector constructs that were used to introduce and express the human IL-2 or IFN-γ genes in tumor cells. All were derived from Moloney murine leukemia virus (MLV) and are based on the high titer N2 retroviral vector, which also contains the bacterial neomycin resistance (Neo) selectable gene. Table 1 lists the 5 human melanoma cell lines that were transduced with IL-2 or IFN-γ vector constructs. These cell lines include both pigmented and non-pigmented strains, and represent both early and late stages of melanocyte differentiation (Houghton et al., 1982; Houghton et al., 1984; Houghton et al., 1987). Following growth in G418 containing medium, Southern blot analysis was used to show that an intact provirus was present. A Southern blot analysis from SK-MEL-29 cells transduced with DC/AD/R/IL2 is shown in Figure 2 to demonstrate that the proviral DNA was integrated in a nonrearranged form and that the transfer of the miniunit from the 3' to the 5'LTR had taken place. A Northern blot of transduced SK-MEL-256, representative of those obtained with 3 of the vectors used, is shown in Figure 3 to demonstrate that the transduced cells were expressing vector-specific RNA transcripts of the proper size. These include one unspliced transcript "V" consisting of the virion RNA and one spliced transcript "N" consisting of the Neo mRNA, both derived from the LTR, as well as one smaller transcript "P" originating from the internal promoter. Uninfected cells expressed none of these messages.

### IL-2 and IFN-γ Secretion by Tumor Cells

Secretion of IL-2 or IFN-γ by melanoma cells transduced with the retroviral vectors was determined using a bioassay and ELISA for IL-2 and a bioassay and RIA for IFN-γ (Table 1).

**Table 1**

| IL-2 OR IFN-γ SECRETION FROM VECTOR TRANSDUCED MELANOMA CELLS¹ | | |
|---|---|---|
| SK-MEL 19 | DC/TKIL2 | 4 |
| | | |
| | DC/TKIFNγ | 3 |
| | N2/CMVIFNγ | 6 |
| SK-MEL 29 | DC/AD/R/IL2 | 40 |
| SK-MEL 131 | DC/TKIL2 | 14 |
| | | |
| | DC/TKIFNγ | 6 |
| | N2/CMVIFNγ | 6 |
| SK-MEL 245 | DC/TKIL2 | 2 |
| | N2IL2 | 10 |
| | N2/CMVIL2 | 5 |
| | | |
| | DC/TKIFNγ | 3 |
| | N2/CMVIFNγ | 1 |
| SK-MEL 256 | DC/TKIL2 | 5 |
| | N2IL-2 | 14 |
| | N2/CMVIL2 | 1 |
| | | |
| | DC/TKIFNγ | 6 |
| | N2/CMVIFNγ | 8 |

| | | |
|---|---|---|
| ¹ IL-2 or IFN-γ concentrations in the test supernatants are in U/ml/10⁶ cells/24 hrs and were determined by bioassays and confirmed by ELISA for IL-2 and RIA for IFN-γ. Results in this table represent values (mean of duplicates) obtained from the IL-2 bioassay and IFN-γ RIA. | | |

Data given in Table 1 are representative for transduced bulk infected cell populations. Clonal populations were isolated and analyzed as well. The IL-2 produced by single clonal populations varied widely ranging from 691 U/ml/10⁶ cells to 0 U/ml/10⁶ cells (Table 2).

**Table 2**

| **IL2 PRODUCTION BY TRANSDUCED CLONED MELANOMA CELLS**^{**1**} | | |
|---|---|---|
| **SK-MEL-19/DC/TK/IL2:** | Clone # 4 | 61 U/ml |
| | # 3 | 36 U/ml |
| | # 6 | 0 U/ml |
| **SK-MEL-29/DC/AD/R/IL2:** | Clone # 6 | 620 U/ml |
| | # 8 | 168 U/ml |
| | # 12 | 0 U/ml |
| **SK-MEL-131/DC/TK/IL2:** | Clone # 1 | 691 U/ml |
| | # 6 | 314 U/ml |
| | # 14 | 0 U/ml |

| | | |
|---|---|---|
| ¹ IL-2 concentrations in the test supernatants were determined by bioassays and confirmed by ELISA. Results in this table represent values of selected clones to show the wide range of IL-2 secretion. | | |

As expected from previous results, the cell lines varied in their expression and secretion of cytokines depending on the particular retroviral vector used. In all cases human IL-2 or IFN-γ activity could be neutralized by an appropriate monoclonal antibody (data not shown).

Melanoma cells transduced with the control vector DCA did not secrete detectable amounts of IL2 or IFN-γ (data not shown). Secretion of human IL-2 or IFN-γ had no discernible effect on cell morphology, pigmentation or on growth rate in culture compared to parental cells or cells transduced with DCA (data not shown). Some of these transduced cell lines have been in culture longer than 6 months and have continued to secrete stable amounts of IL-2 or IFN-γ.

### Irradiated melanoma cells continue to secrete IL-2

In the context of immunotherapy, transduced cells injected intradermally could serve both as a source of continuous local cytokine production and of tumor antigen. Although cytokine-secreting tumor cells exhibit decreased tumorigenesis and would most likely not grow in vivo, experimetns were designed to test whether irradiating them as a further precaution would affect cytokine secretion. The results demonstrated that following irradiation with up to 10,000 rad, cytokine production persisted for 3-4 weeks (Table 3 and Figure 4).

**Table 3**

| **IL2 PRODUCTION OF SK-MEL-29/DC/AD/R/IL2 AFTER IRRADIATION WITH 10.000 rads**^{**1**} | | | | | |
|---|---|---|---|---|---|
| DAYS | U/ml | # of ml | TOTAL UNITS | TOTAL CELLS | U/10⁶ CELLS |
| | | | | | |
| 1 - 5 | 155 | 3.5 | 543 | 1.4 x 10⁶ | 39 |
| 9 - 13 | 190 | 3.5 | 665 | 1.1 x 10⁶ | 60 |
| 17- 21 | 193 | 3.6 | 695 | 0.6 x 10⁶ | 116 |

| | | | | | |
|---|---|---|---|---|---|
| ¹ Transduced cells were irradiated with 10.000 rad, plated at identical concentrations in separate dishes and supernatants collected at the indicated time from a single plate. After supernatant collection cells were trypsinized and counted. | | | | | |

Post irradiation IL-2 secretion increased, possibly due to increased leakage from radiation damaged cells (Table 3). The duration of IL-2 secretion was influenced by the amount of radiation delivered to the cells and in this way defined by the radiosensitivity of each cell line. The irradiated cell lines were incapable of growth in culture as demonstrated by the decreasing cell number over time (Table 3). In addition, the irradiated IL-2 secreting tumor cells were not able to form tumors in nude mice. This has been a consistent finding in different tumor types. IFN-γ secretion after irradiation was not tested, but in a human renal carcinoma model used to study the effect of IFN-γ gene transduction, secretion of IFN-γ persisted for several weeks after irradiation (Gastl et al., 1992). These results illustrate that it is possible to lethally irradiate the cells before injection into patients and continue to have cytokine secretion for several weeks.

### Uprequlation of MHC class I by IFN-γ

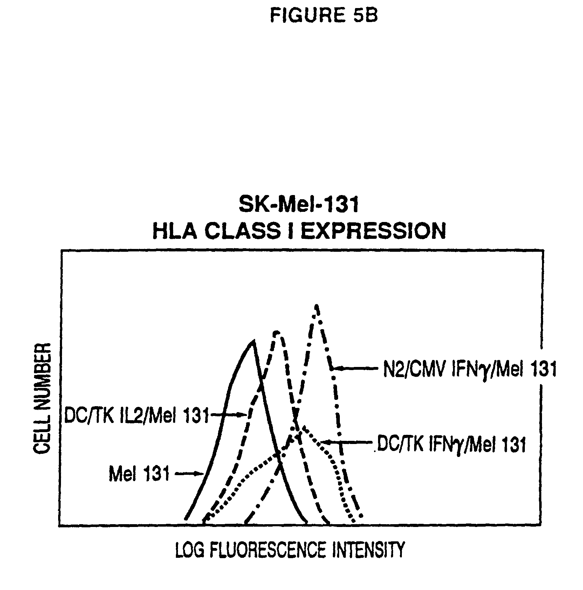

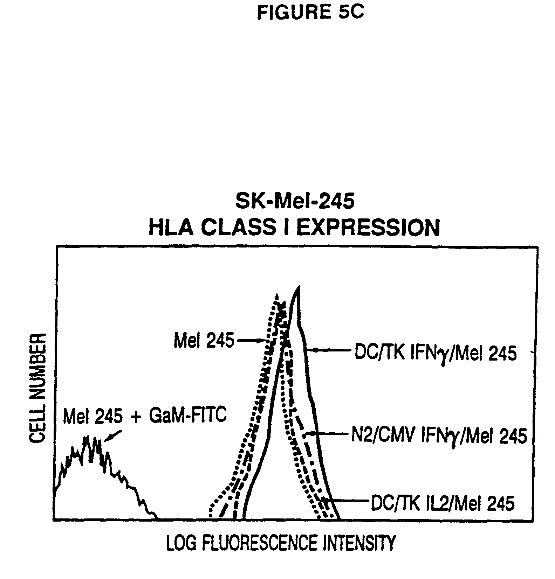

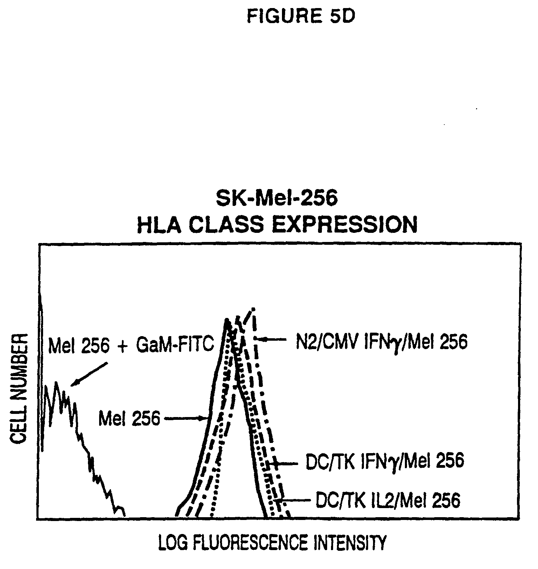

IFN-γ is able to upregulate surface expression of MHC antigens on a variety of cell types (Wallach et al., 1982; Collins et al., 1984; Rosa et al., 1983). Since antigen is recognized by T cells only when presented in the context of MHC class I or II molecules, it is believed that an important mechanism by which IFN-γ exerts an anti-tumor effect is to upregulate antigen presentation by augmenting MHC expression. To test whether there was a biological effect of IFN-γ on MHC expression by melanoma cells, MHC class I antigen expression was examined after IFN-γ transduction. A variable upregulation of MHC class I was observed on the IFN-γ secreting melanoma lines. In analogy to observations described for IL-2, a wide variation in IFN-γ expression was observed. Other investigators using one of the IFN-γ vectors described in this report observed a similar finding in an experimental murine system (Restifo et al., 1992). MHC class I was expressed on parental SK-MEL-131 tumor cells before infection and levels were upregulated following IFN-γ gene transduction (Figure 5). SK-MEL-245 and SK-MEL-256 also expressed HLA class I molecules on the cell surface. IFN-γ secretion induced modest upregulation on SK-MEL-245, but not on SK-MEL-256 cells. SK-MEL-19 did not express HLA class I molecules and IFN-γ secretion had no effect (Figure 5). Thus, upregulation of class I molecules after IFN-γ transduction was variable, and induction of MHC must be evaluated on an individual basis.

### Growth in Nude Mice

To test cells representing a range of IL-2 secretion SK-MEL-29 and SK-MEL-245 cells, which secrete 40 and 2 U IL-2/ml/10⁶ respectively, were chosen to be injected into nude mice. Groups of three BALB/c *nu*/*nu* mice were injected subcutaneously with 2.5 x 10⁵, 5 x 10⁵ and 1 x 10⁶ cells of parental or corresponding IL-2 secreting bulk selected melanoma cell populations. All mice injected with parental cells developed palpable tumors within 4 weeks, while all mice injected with IL-2 secreting tumor cells remained tumor free during an observation period of 3 months (data not shown). None of the IL-2 secreting melanoma cell lines generated tumors in nude mice, showing that the secreted IL2 was biologically active and suggesting that effector cells, possibly NK cells, are activated in vivo. IL-2 or IFN-γ secreting tumor cells irradiated with 10,000 rads and immediately injected into nude mice did not form tumors either. Non-irradiated IFN-γ secreting tumor cells formed tumors as expected since IFN-γ action is species specific (Langer et al., 1988).

### Replication Competent Retroviruses Are Not Generated

Replication competent murine retroviruses may arise during the experimental procedures by recombination with endogenous retroviral like sequences. The presence of replication competent murine retrovirus was assayed in a group of transduced tumor cell lines. Melanoma and renal carcinoma cell lines had been transduced 21-28 days previously with DC/AD/R/IL2 and the supernatants used to infect NIH3T3 cells. After growing the NIH3T3 cells for 21 days G418 resistance and reverse transcriptase activity were assayed. No G418 resistance was detected. No reverse transcriptase activity was seen using the HeLa and NIH3T3 Amplification Assay (Figure 6)

### DISCUSSION

In this report, it was demonstrated that it is possible to transduce human melanoma cells with the IL-2 or IFN-γ gene using retroviral vectors and to induce them to secrete cytokines constitutively. Expression of a variety of cytokine genes including IL-2 (Gansbacher et al., 1990b; Fearon et al., 1990; Ley et al., 1991), IL-4 (Tepper et al., 1989), IFN-γ (Gansbacher et al., 1990a; Watanabe et al., 1988), IL-6 (Porgador et al., 1992) and IL-7 (Hock et al., 1991) has been shown to exert anti-tumor effects in animal models. Several of these studies have demonstrated a role for cytotoxic T lymphocytes in tumor rejection (Gansbacher et al., 1990a; Fearon et al., 1990; Ley et al., 1991; Gansbacher et al., 1990b; Hock et al., 1991; Porgador et al., 1992). Cytokine gene therapy in melanoma was examined, with the aim of using transduced cells in an active immunotherapy protocol. Melanoma is among the best studied human cancers shown to express tumor antigens (Van Der Bruggen et al., 1991) and these antigens have epitopes recognized by CTLs (Coulie et al., 1992). Both shared and unique (e.g., expressed only by autologous tumor cells) determinants can be recognized by CTL in an MHC restricted fashion on melanoma cells, either together with HLA-A1 or with HLA-A2 (Crowley et al., 1990; Crowley et al., 1991; Kawakami et al., 1992; Crowley et al., 1992). Most importantly from a therapeutic perspective, one of the genes cloned from a human melanoma cell was expressed on a variety of tumor cells, including non-melanoma cells, but has not yet been detected on normal cells (Van Der Bruggen et al., 1991).

CTLs have been shown to exert a tumoricidal effect on melanoma cells (De Vries et al., 1984; Anichini et al., 1985; Mukherji et al., 1983). Coulie et al. reported that CTL precursors could be detected in the peripheral blood of melanoma patients, with a frequency ranging from 1/900 to 1/33,000 (Coulie et al., 1992). The frequency of cytotoxic precursors in these melanoma patients is in the range of that observed after successful immune responses against viruses such as cytomegaly virus (CMV), herpes simplex virus (HSV), and varicella zoster (Hickling et al., 1987; Borystewicz et al., 1988). These findings are important because they suggest vaccination protocols with the goal of increasing the frequency of CTLs, rather than inducing them de novo. Evidence that such expansion can occur was reported in the P815 tumor model where in vivo secretion of IL-2 by the tumor cells increased the frequency of circulating tumor specific CTL's 2-4 fold (Ley et al., 1991).

Four IL-2 constructs and two IFN-γ constructs were used in these studies (Figure 1). IL-2 and IFN-γ were chosen because they previously demonstrated efficacy in decreasing the tumorigenesis of cells that secrete them and because they act on both effector and tumor cells, possibly in a synergistic fashion (Gansbacher et al., 1990a; Fearon et al., 1992; Ley et al., 1991; Gansbacher et al., 1990b; Watanabe et al., 1988). As expected, cells infected with the recombinant vectors varied with respect to the amount of cytokine secreted (Table 1). Within bulk infected cells clones secreting from 0 U/ml to 691 U/ml/10⁶ cells were found. If transduced tumor cells express a known tumor antigen such as MAGE-1 one can select the highest IL-2 and MAGE-1 expressor clone for vaccination in the attempt to maximize clonal expansion of the MAGE-1-specific CTL population in vivo. If the tumor antigen is not known it is reasonable to use bulk selected cytokine secreting tumor cells as vaccine to maintain antigenic heterogeneity.

Following irradiation with 10,000 rad, the cells continued to produce significant amounts of IL-2 for 3 to 4 weeks (Figure 4 and Table 3). Gamma irradiation actually caused increased cytokine release for a short period, possibly due to leakage of cytokines from dying cells. These observations may be important in clinical application where it is desired to avoid injecting viable tumor cells into patients.

IFN-γ secretion by tumor cells increased MHC class I expression in some lines but not in others was observed, although the basis of this phenomenon is not apparent (Figure 5). Since tumor antigens are presented to T cells in context of HLA class I (Crowley et al., 1990; Crowley et al., 1991; Van Der Bruggen et al. 1991) it will be important to select tumor cells that are IFN-γ responsive for vaccination protocols.

The results of this series of experiments demonstrated a biological effect in vivo, since the IL-2 secreting cells were rejected in BALB/c *nu*/*nu* mice while the normal counterparts formed tumors. Since nude mice lack T cells but do have NK cells, that NK cell activation participated in tumor rejection was assumed. These results support only a notion that the secreted IL2 is biologically active and do not predict the effect on a CTL population. Human IFN-γ secreting tumor cells was used as a control to exclude the possibility that other factors induced by the retroviral infection would by responsible for tumor rejection. IFN-γ action is species specific (Langer et al., 1988) and melanoma cells secreting human IFN-γ formed tumors identically to parental melanoma cells.

These experimental data demonstrate that IL-2 or IFN-γ secreting melanoma cells are available for clinical use. Autologous cells may or may not be preferable to allogeneic cells for vaccination protocols, however in only 20-30% of melanoma patients can tumor cell lines be established. Since shared tumor antigens are MHC class I restricted (Crowley et al., 1990; Crowley et al., 1991; Van Der Bruggen et al. 1991) it should be possible to use established allogeneic cell lines transduced with cytokine genes for vaccination. At least two MHC-class I molecules, HLA-A1 and HLA-A2, have been described to present shared melanoma antigens (Crowley et al., 1990; Crowley et al. 1991; Van Der Bruggen et al., 1991). Since 40% of the Caucasian population express the HLA-A2 molecules, the use of allogeneic melanoma cells that express HLA-A2 and shared melanoma antigens is an attractive strategy. In this study, SK-MEL-29, SK-MEL-245, and SK-MEL-256 (Table 1) express the HLA-A2 molecule. HLA-A2 matched melanoma patients would be candidates for such a vaccination protocol since most HLA-A2 positive melanomas express shared melanoma antigen (Kawakami et al., 1992). HLA-A matched allogeneic irradiated tumor cells that secrete IL-2 have the potential to activate and amplify cytotoxic effector cells that recognize peptides presented by autologous tumor cells in the context of matched HLA. Data published recently show an 80 fold more potent expansion of the antigen specific clone if the antigen presenting cells express the antigen with a costimulatory signal (Liu et al., 1992).

One of the cell lines described in this report, SK-MEL-29, has already been used as an allogeneic vaccine in 17 melanoma patients (Livingston et al., 1985). SK-MEL-29 expresses well characterized melanocyte differentiation antigens. None of the vaccinated patients developed an antibody response against these differentiation antigens documenting that irradiated tumor cells alone are inadequate as stimulators of a humoral antitumor response. Induction of high affinity IgG responses requires CD4⁺ T cell help. Thus, high affinity IgG production against melanocyte differentiation antigens would also be an indication that such vaccination could activate host T cells.

Despite the supporting experimental data, a vaccination approach using IL-2 secreting tumor cells may fail. For example, irradiation may decrease immunogenicity of tumor cells, vector design may not be optimal and the amounts of IL-2 secreted could be insufficient, or tumor cells could secrete suppressor factors preventing successful expansion of CTL. Systematic approaches to each of the potential problems will be needed. The present results demonstrate that retroviral vectors can be used to introduce cytokine genes into human tumor cells. The transduced tumor cell lines can be maintained in culture for several months during which time they continue to secrete cytokines in a stable manner. Rejection of IL-2 secreting tumors by nude mice and upregulation of MHC class I genes on some of the tumor cells demonstrates that the secreted cytokines are biologically active.

These data provide scientific and practical support for active vaccination protocols using retroviral cytokine gene transfer in an attempt to increase the frequency of preexisting cytotoxic precursors in the circulation of tumor bearing patients.

Cytokines are important modulators of host antitumor responses. Two of these cytokines, Interleukin-2 (IL-2) and Interferon-gamma (IFN-γ), are produced after antigen (Ag) induced activation of helper lymphocytes. The cytokines are released into the immediate vicinity where they either interact with the appropriate receptors on effector cell populations or are rapidly degraded. To mimic this physiologic release of cytokines at the effector-target site, retroviral vectors were used to transduce melanoma cells with the IL-2 or IFN-γ cDNA. Five melanoma cell lines were transduced with IL-2 or IFN-γ containing vectors and secreted IL-2 at 1-40 U/ml/10⁶ cells/48hrs or IFN-γ 1-8 U/ml/10⁶ cells/48hrs respectively. Following gamma irradiation, these cells continued to secrete cytokines for about 3-4 weeks. Secretion of IFN-γ induced upregulation of major histocompatibility complex (MHC) class I molecules in a subset of melanoma cell lines. IL-2 production by human melanoma xenografts induced tumor rejection in BALB/c *nu*/*nu* mice demonstrating the in vivo effect of this cytokine. This study shows that a) human melanoma cells can be stable transduced with cytokine-containing retroviral vectors, b) cytokines are secreted constitutively by the transduced tumor cells and have the expected biological effects in vitro and in vivo, and c) after gamma irradiation, cytokines continue to be secreted for several weeks. These data suggest that irradiated cytokine secreting allogenic or autologous tumor cells can be used in vaccination protocols for cancer patients.

### Second series of experiments

### A PILOT STUDY OF IMMUNIZATION WITH HLA-A2 MATCHED ALLOGENEIC MELANOMA CELLS THAT SECRETE INTERLEUKIN-2 IN PATIENTS WITH METASTATIC MELANOMA

The objective of this series of experiments is to immunize HLA-A2-positive melanoma patients with HLA-A2 matched allogeneic melanoma cells that have been altered to secrete interleukin-2.

### Background

### Malignant Melanoma

Annually more than 32,000 cases of melanoma are diagnosed in the United States, resulting in greater than 8,000 deaths (American Cancer Society, 1989). Current systemic therapy for metastatic melanoma is inadequate. Dacarbazine (DTIC) is the most active single chemotherapeutic agent and is widely employed in the treatment of patients with metastatic disease, with response proportions ranging from 14-20% (Comis, 1976; Carbone et al., 1976; Luikart et al., 1984; Costanza et al. 1977; Bellet et al. 1979). Clinically meaningful regression of visceral metastases is uncommon. The nitrosoureas have also been used with equally disappointing results (Comis, 1976; Carbone et al., 1976; Luikart et al., 1984; Costanza et al. 1977; Bellet et al. 1979). Combination chemotherapy has not clearly improved the response proportion observed with single agent therapy, and has greater toxicity (Luikart et al., 1984; Costanza et al. 1977; Bellet et al., 1979).

The failure of conventional chemotherapeutic agents to impact on the treatment of metastatic melanoma has prompted the use of biological response modifiers in clinical trials. Studies have used interleukin-2 (IL-2) with or without LAK cells (Rosenberg, 1988; Parkinson et al., 1990), interferon (Creagan et al., 1984), and monoclonal antibodies (Vadhan-Raj et al., 1988), with response proportions ranging from 19-23%. These response proportions are modest in current treatment regimens employed, with sometimes considerable toxicity.

### Vaccination Studies

Every decade since the turn of the century has seen efforts to develop cancer vaccines, and these have taken the form of autologous or allogeneic fresh tumor tissues, cultured tumor cells, or tumor cells modified by chemicals, enzymes or viral infection. Malignant melanoma has been the leading type of cancer by far in these studies. In general, these trials did not produce convincing evidence of therapeutic efficacy when they were appropriately controlled (Oettgen et al., 1991).

The testing of human cancer vaccines involves uncertainties ranging from appropriate type, dose, route, and frequency of vaccine to suitable patient selection and evaluation of clinical response. Given the array of variables, a sequential analysis of effectiveness, if it were evaluated on clinical tumor response alone, would be extremely time consuming. What is required for the development of an immunogenic cancer vaccine are methods to assess effectiveness that are both rapid and objective and that guide the step-by-step process of vaccine construction and testing. With regard to vaccines against infectious diseases, serologic responses to bacterial and viral antigens have been an essential step in their development. The lack of comparable tests of humoral and cellular immunity to monitor the immunogenicity of cancer vaccines in humans has been a major impediment to investigating this approach to cancer therapy.

Beginning with the 1970's, attempts were made by many groups to demonstrate a specific immune response to vaccination in patients receiving cancer vaccines. Most of these studies were done in patients with melanoma, and involved tests for delayed cutaneous hypersensitivity, lymphocyte cytotoxicity, lymphocyte migration inhibition, and a variety of serologic assays. Because of the difficulties that were encountered in the analysis of the specificity of allogeneic reactions (in most cases the sera or lymphocytes tested and the melanoma cells used for vaccine construction or target cell preparation did not come from the same patient), those studies did not provide unequivocal evidence of a tumor specific immune response to vaccines (Oettgen et al., 1991).

A clearer picture emerged when the analysis was restricted to autologous serologic reactions. Although this approach depended on establishing tumor cell lines from each patient to be studied, the testing of autologous combinations of tumor cells and sera eliminated the confusing reactions to normal allogeneic cell-surface antigens. Autologous typing defined three classes of surface antigens on melanoma cells. Class I antigens are restricted to the autologous tumor. Class II antigens are shared tumor antigens that are found on autologous as well as allogeneic tumor cells; some Class II antigens are found on a restricted set of normal cell types and therefore, can be considered autoantigenic differentiation antigens. Class III antigens are widely distributed on malignant as well as normal cells (Old, 1981). With the development of autologous typing systems for defining cell-surface antigens of melanomas, serologic tests of requisite sensitivity and specificity were available that could be used to gauge the immunogenicity of melanoma vaccines. A series of trials with vaccine containing class I or II melanoma antigens showed, however, that an antibody response to the relevant class I or II melanoma antigens were induced by vaccination only in exceptional cases, although there was no lack of a humoral immune response to other vaccine components (Livingston et al., 1985). Speculation regarding the reasons for the difficulties encountered in these attempts to induce an antibody response to class I or class II melanoma antigens spawned a range of possibilities including insufficient sensitivity of serologic tests, unfavorable antigen presentation in the vaccines, genetic restriction of reactivity with melanoma antigens, and suppression of antibody production by suppressor cells or anti-idiotypic antibodies.

With the advent of hybridoma technology, a number of proteins, glycoproteins, and glycopeptides were identified as cell-surface constituents of human melanoma and candidates for vaccine construction. Prominent among them are the gangliosides GM2, GD2, and GD3. Vaccines containing purified GM2 have been shown to elicit production of GM2 antibodies in patients with melanoma (Livingston et al. 1987; Tai et al., 1985) when administered with BCG as adjuvant after pre-treatment with low dose cyclophosphamide to counteract suppressor activity.

Cellular immunity to cancer antigens, as opposed to humoral immunity, has been of much interest ever since it was shown in the early 1960's that specific immunity to challenge with chemically induced sarcomas can be transferred in the mouse with lymphocytes from immunized donors, but not serum. Defining the specificity of cellular immune reactions to cancer has been far more difficult than defining the specificity of humoral immune reactions to cancer antigens. To this regard, the discovery of the T cell growth factor, interleukin-2, which permits culture and cloning of T lymphocytes, has been a major step forward and opened a new era in the study of T cell immunity. Cytotoxic T lymphocyte clones with restricted reactivity for autologous melanoma have been isolated from the blood of patients in several laboratories, and similar results were obtained in studies of clones derived from tumor-infiltrated-studies of clones derived from tumor-infiltrated-lymphocytes (TIL) (Hainaut et al., 1990). In some cases these clones have shown exquisite specificity for autologous melanoma (Oettgen et al., 1991). In some instances, the presenting MHC molecules could be identified as class I molecules by inhibition of lysis with antibodies directed against HLA determinants. In the case of the melanoma cell line SK-MEL-29 (patient AV from Livingston et al., 1979), established at this institution, at least those antigens are recognized by autologous and cytotoxic T cell clones in association with HLA-A2 (Wolfel et al., 1989).

Both HLA-A2 and HLA-A1 have been identified as restriction elements in other melanomas and also sarcomas (Knuth et al., 1991). HLA-A2 expressed on human melanoma cells has been shown to present shared melanoma antigens recognized by T cells derived from peripheral blood, regional lymph node and tumor infiltrates. Thus, HLA-A2 is capable of presenting antigens that are shared among melanoma cells, but not apparently expressed by several other cell types. The outstanding challenge confronting the field of cellular immunity is the structural definition of the targets recognized by cytotoxic cells on human tumor cell. A major advance in this area has been the cloning of the gene that encodes a mouse tumor antigen recognized by cytotoxic T cells and the identification of its mutation (Deplaen et al., 1988). The same approach is now being applied to human melanoma, and a gene encoding an antigen recognized by cytolytic T cells on a human melanoma has been identified. In this case, the antigen appears to be presented by HLA-A1 (T. Boon, personal communication H. Oettgen) (Van Der Bruggen, 1991).

### Biologic effects in vitro of Interleukin-2

Interleukin-2 is a lymphokine that is produced by T helper cells and stimulates T cells, B cells and natural killer (NK) cells. In in vitro studies, IL-2 has been shown to induce proliferation of antigen-specific T cells. Helper, cytotoxic, and suppressor T cells from melanoma patients have been grown in vitro by culturing in the presence of IL-2 and antigen-presenting cells (Glasebrook et al., 1982). B cell responses in vitro have also been enhanced by IL-2 (Roehm et al., 1983). Growth of T cells in IL-2 can also lead to secretion by T cells of other lymphokines, including interferon-γ, IL-1, IL-4, IL-6, GM-CSF and TNF-a.

### Interleukin-2 in Human Studies

A number of investigators have studied the systemic administration of IL-2 patients with advanced cancer (Rosenberg, 1985; Rosenberg et al., 1987; Rosenberg et al., 1989). Systemic IL-2 has shown modest activity in patients with advanced melanoma and other tumors, with a 15-20% response rate reported (Rosenberg et al., 1987). The major effect of IL-2 appears to be the expansion of effector cell populations that have the capability to kill the tumors in vitro. However, because of the short serum half-life of IL-2, high doses have been used that are very toxic. Hypotension and capillary leak syndrome with decreased systemic vascular resistance has led to interstitial pulmonary edema, prerenal azotemia, cardiac arrhythmias, and myocardial infarction (Rosenberg, 1985; Rosenberg et al., 1987; Rosenberg et al., 1989).

### Gene Transfer In Animal Models

Several laboratories have shown that the introduction of cytokine genes, such as IL-2, IL-4 (Tepper et al., 1989), IFNγ (Watanabe et al., 1989; Gansbacher et al., 1990), TNF-α (Asher et al., 1991) and G-CSF (Colombo et al., 1991) into tumor cells leads to their rejection by an immunologically competent host in specific animal model systems. Secretion of G-CSF or IL-4 by tumor cells has led to localized inflammatory responses at the tumor site without apparent induction of immunological memory (Tepper et al., 1989; Colombo et al., 1991). Initial studies by Tepper et al. (Tepper et al., 1989) demonstrated that localized IL-4 secretion at the tumor site was characterized by accumulation of eosinophiles and macrophages. IL-2, interferon-γ, and TNF-α on the other hand have induced long lasting cellular immune responses that led to rejection of potentially lethal tumor challenges at later time points (Gansbacher et al., 1990a; Gansbacher et al., 1990b; Tepper et al., 1989; Watanabe et al., 1989). Fearon et al. (Fearon et al., 1989) introduced the IL-2 gene into a poorly immunogenic murine colon carcinoma. The IL-2-secreting tumors were rejected, and subsequently induced an effective cytolytic T cell response. Depletion of CD8-positive cells in vivo abrogated the cellular antitumor response, implicating a pivotal role for Class I MHC restricted CD8-positive cells.

To approximate more closely a physiologic mode of cytokine secretion generated in the course of an immune response, the IL-2 gene was directly introduced into a murine fibrosarcoma cell line (CMS5). In this way a localized secretion of low levels of cytokine was achieved that led to the regression of injected tumor cells (Gansbacher et al., 1990a; Gansbacher et al., 1990b). When control mice were challenged by tumor, they died within 45 days. Mice challenged with IL-2 secreting tumor cells rejected implanted tumor cells and were cured. These mice developed specific immunological memory. Rechallenge with potentially lethal doses of CMS5 parental tumor cells after several months led to tumor rejection, while an unrelated tumor grew and killed immune mice, demonstrating the specificity of the response. Lymphocytes taken from spleens of immune animals were capable of killing the tumor in vitro throughout the life span of mice. Of note, only weak cytolytic responses were generated against CMS5 tumor cells after challenge with parental tumor cells; these responses were transient and disappeared within three weeks after tumor implantation. However, mice injected with IL-2 secreting tumor cells generated tumor specific cytolytic activity that lasted more than 75 days.

Rosenberg et al. (unpublished data) have reproduced these results in the poorly immunogenic tumor MCA-102. Despite many attempts, they were never able to generate a cellular antitumor response using unmodified tumor cells. Introduction of the IL-2 gene into these cells increased their immunogenicity to a level which generated tumor rejection and memory. In addition, co-injection of IL-2 producing tumor cells with unmodified, parental tumor cells produced an immune response strong enough to reject not only the lymphokine secreting tumor cells but also unmodified tumor cells.

These finding have also been reproduced at MSKCC in different mouse tumor types, including B16 melanoma, 38C13 B cell lymphoma, and bladder carcinoma MBT2. Toxicity related to IL-2 has been observed in any of the animals challenged with IL-2 secreting tumor cells. Additional experiments using irradiated, IL-2 secreting CMS5 cells have demonstrated that there was no difference in their ability to induce immunological memory in vivo as compared to non-irradiated IL-2 secreting tumor cells.

In summary, the introduction and constitutive expression of IL-2 by tumor cells themselves has increased the immunogenicity of a variety of histologically different cancers. It appears from these studies that the localized, persistent expression and secretion of low levels of IL-2 may have an impact on the host anti-tumor response in the absence of any detectable toxicity.

### Gene Transfer in Humans

Most recently, Rosenberg et al. have taken genetically altered autologous tumor infiltrating lymphocytes and injected them into patients with refractory metastatic tumors (Rosenberg et al., 1990). A retroviral vector containing the neomycin-resistance gene was introduced into autologous lymphocytes in order to monitor cells reinfused into patients. Of five patients reported, cells from four were successfully grown in tissue cultures with G418, a neomycin analogue. These cells that showed neomycin resistance were given intravenously to patients along with systemic IL-2. Peripheral blood was recovered periodically and circulating mononuclear cells with neomycin resistance were detected. There were no additional toxicities seen as a result of the genetic alteration. No intact virus was recovered from any patient. Rosenberg concluded that this procedure was both feasible and safe (Rosenberg et al., 1990).

Prior safety studies have shown that exposure of primates to large infusion of infectious murine amphotrophic virus produces no acute pathologic effects (Cornetta et al., 1990). Twenty one primates transplanted with retroviral gene-modified bone marrow showed no evidence of toxicity related to the gene transfer as long as five years after infusion (Kantoff et al., 1987). The first adenosine deaminase-deficient patient with Severe Combined Immunodeficiency (SCID) underwent successful retroviral mediated adenosine deaminase gene transfer approximately one year ago at the NCI (Kantoff et al., 1987). So far, investigators observed partial reconstitution of cellular immune function after infusion without any detectable adverse effects on the patient.

To investigate whether findings from the murine system could be extended, human tumor cell lines with cytokine genes using retroviral vectors were infected. To date, the human IL-2 gene, human IFN-γ gene and the human epidermal growth factor gene have all been successfully transduced into a variety of human cell lines at MSKCC. In collaboration with Dr. Ray Taetle (University of Arizona), the epidermal growth factor receptor gene has been introduced into the Burkitts lymphoma cell line, Namalwa; the receptor was shown to be expressed on the cell surface and fully functional (Oval et al., 1991). Stimulation of these transduced cells with the epidermal growth factor led to the appropriate binding of ligand and signal transduction (Oval et al., 1991). In addition, the IL-2 or the IFN-γ gene have been introduced and expressed in primary and established human melanoma cell lines. These genetically modified human melanoma cells secreted constitutively up to 20 U/ml recombinant IL-2 into the supernatant. Constitutive IFN-γ expression led to upregulation of MHC class I and class II antigens on the cell surface of melanoma cells. Injection of the IL-2 secreting tumor cells into nude mice led to tumor rejection while unmodified parental cells were not rejected. Irradiation of the genetically altered melanoma does not immediately abrogate IL-2 secretion (see appendix F). IL-2 was continuously secreted for 2-3 weeks after lethal irradiation in amounts comparable to non-irradiated transduced cells.

### Rationale

A study to determine whether immunization with allogeneic melanoma cells expressing a recombinant human IL-2 gene can be well tolerated without evident toxicity is proposed. There is substantial experience at MSKCC and at other institutions that has established the general safety of immunization using irradiated allogeneic tumor cells. The choice of allogeneic HLA-A2-positive tumor cells is based on two considerations. First, the use of a single established immunizing cell line bypasses the necessity to infect autologous tumor cells for each patient. Growing autologous tumor cells in tissue culture can be a difficult and time-consuming procedure. Since approximately 40% of North Americans are HLA-A2 positive, a tumor vaccine produced from HLA-A2 matched melanoma cell lines could be used in a substantial subpopulation of melanoma patients. Preclinical studies have been performed with a single cell line, SK-MEL-29. This cell line expresses a number of restricted melanocyte differentiation antigens, as determined by typing with monoclonal antibodies, and class II MHC antigens. In addition, SK-MEL-29 was derived from a patient (AV) with extensive stage III melanoma who was shown to have cytotoxic CD8-positive T cells in his peripheral blood that lysed autologous tumor in an HLA-A2 restricted fashion (Roehm et al., 1983; Basham et al., 1983) (see Vaccination Studies and Clinical Protocol). Despite extensive and recurrent melanoma, patient AV is alive and free of disease more than 15 years after his initial treatment. Secondly, there is substantial evidence that melanoma cells can present restricted shared antigens (i.e. antigens expressed by autologous and allogeneic melanoma tumors but not certain non-melanoma cell types) to autologous T cells. The protocol will be restricted to patients who are HLA-A2 positive.

### Melanoma Cell Line

A well characterized and established melanoma cell line, SK-MEL-29, has been used for expression of IL-2. SK-MEL-29 is a well characterized melanoma cell line that is known to express a number of melanocyte differentiation and gangliosides antigens (Wolfel et al., 1989; Houghton et al., 1982). SK-MEL-29 was established from a patient, AV, in 1975 from regional lymph node metastases (see Livingston et al., 1979). Patient AV originally was diagnosed with melanoma Clark's level IV of the anterior chest wall in 1974 at age 18. In March 1975, melanoma recurred in multiple left axillary lymph nodes with deep invasion into soft tissue. This disease was resected and he received experimental adjuvant immunotherapy with Corynebacteria parvum. In August 1975, disease recurred in the left supraclavicular and infraclavicular area. He had surgical resection and started vaccine treatment with irradiated autologous melanoma cells and BCG adjuvant. In December 1975, the patient had a third recurrence of tumors in the left neck with invasion of the axillary vein and brachial plexus. He was treated with surgical resection followed by radiation therapy, then dacarbazine adjuvant chemotherapy in 1976-1977. In 1977, he restarted vaccination with autologous tumor cells and BCG. He developed a recurrent melanoma in the left neck in February 1978 that was resected. The vaccine was continued up to 1980. As of 1991, he has had no further recurrence of melanoma. CD8-positive T cells were derived from peripheral blood after vaccination that efficiently lysed autologous SK-MEL-29 tumor cells in an HLA-A2 restricted fashion, and have been extensively reported by Knuth and colleagues (Wolfel et al., 1989; Knuth et al., 1984)

The SK-MEL-29 melanoma cell line has been infected with the retroviral vector, NAP-AD/IL-2 (DC/AD/R/IL2). Those tumor cells constitutively and stably secrete IL-2. Cells will be irradiated with 10,000 rads prior to injection, to assure their inability to proliferate. Two dose levels of melanoma cells will be used for the vaccines.

### Clinical Protocol

This is a pilot study of immunization with SK-MEL-29 allogeneic melanoma cells that have bene altered to secrete IL-2, given as a vaccine to patients with stage IV metastatic melanoma, in order to test the safety of this procedure. Patients will be HLA tissue typed. Patients who are HLA-A2 positive will receive vaccinations of allogenic SK-MEL-29 cells that secrete IL-2. The expected accrual for this study is twelve patients. The expected duration for the trial will be 6-12 months.

Tissue culture of SK-MEL-29 (DC/AD/R/IL2) cells will be grown in the laboratories of Drs. Bernd Gansbacher and Alan Houghton. The melanoma cells have been infected with the retroviral vector NAP-AD/IL2. Cells that secrete of IL-2 will be irradiated (10,000 rad) and given as a series of four vaccinations. If a patient has a response as defined in section Criteria For a Therapeutic Response, then additional booster will be given up to one year in duration or until progression of disease. Six patients will receive a low dose vaccine and the additional six will receive higher dose.

### Criteria for Patient Eligibility

Patients must fulfill the following criteria to be eligible for study admission:
1. Patients with histologic confirmation of metastatic malignant melanoma at MSKCC.
2. Stage IV disease that is not curable by surgical resection.
3. Patients must have measurable or evaluable disease.
4. Karnofsky performance status greater than or equal to 70% and life expectancy greater than 4 months.
5. Adequate hematologic function with WBC ≥ 3000/mm³, absolute lymphocyte count > 1000/mm³, hemoglobin ≥ 9, and platelet ≥ 100,000/mm³.
6. Adequate renal and hepatic function (serum creatinine ≤ 2.5 mg/dl and bilirubin ≤ 1.7 mg/dl).
7. At least 4 weeks since any prior radiation therapy, immunotherapy, or chemotherapy (6 weeks for nitrosoureas), and full recovery from such treatment.
8. The ability to give written informed consent.
9. Age ≥ 18 years.
10. No history of other concurrent malignancies except basal cell carcinoma, squamous cell carcinoma of the skin, or carcinoma-in-situ of the cervix.
11. No systemic steroids for at least one week prior to treatment.
12. No intracranial or multiple hepatic metastases.
13. Patients must be HLA-A2 positive.

### Exclusion criteria

The following conditions/situations will render patients ineligible for this study:
1. Serious intercurrent medical illnesses, to include significant cardiac disease (New York Heart Association Class III or IV), angina pectoris, or myocardial infarction within the previous 6 months.
2. The presence of an active infection, including urinary tract infection.
3. The presence of active intracranial or multiple hepatic metastases.
4. Pregnant or lactating women.
5. Patients who have received more than two regimens of systemic chemotherapy. Use of low dose cyclophosphamide as part of a previous tumor vaccination program shall not constitute a prior systemic chemotherapy regimen.
6. Patients requiring continuing therapy with corticosteroids will not be eligible.

### Concurrent Therapy

The following therapies are prohibited while patients are being treated on this protocol: radiation therapy, chemotherapy, corticosteroids (except as administered for life-threatening circumstances), or other immunotherapy.

### Pretreatment Evaluation

1. History and physical examination, including documentation of all measurable disease.
2. Height, weight, and performance status.
3. Screening profile, BUN, and creatinine.
4. CBC, differential, and platelet count.
5. Chest X-ray.
6. EKG
7. Cranial CT scan.
8. An abdominal CT scan will be obtained if the patient has abnormal liver function tests or an elevated LDH.
9. Peripheral blood will be obtained for immune assays (six green top tubes and two red top tubes). These will be stored int he laboratories of Drs. Gansbacher and Houghton.
10. Additional tests, x-rays and scans as indicated by the patient's presenting signs and symptoms and required to define the extent of the patient's disease.

### Treatment Plan

Patients will receive at least four vaccinations. The first six patients will receive a low dose vaccination (defined below). The subsequent six will receive the higher doe level of vaccine. The vaccine will consist of irradiated, allogenic SK-MEL-29 tumor cells that have been engineered to secrete IL-2.
- Low Dose:: Approximately 10 million tumor cells
- High Dose:: Approximately 50 million tumor cells

A series of four vaccinations will be given either subcutaneously into the thigh or the arm of the patient. The sites will vary with each vaccination. A limb that has undergone a lymph node dissection will not be used for vaccination sites. One vaccination will be given every two weeks for four weeks. A booster vaccination will be given two months following the third vaccination. If a patient has a clinical major response (PR or CR as defined in Criteria For a Therapeutic Response), additional boosters will be given every three months for one year or until progression of disease. Vaccination may continue beyond one year in responding patients at the discretion of the Principal Investigators. Patients with minor responses or stable disease will receive boosters at the discretion of the principal investigators up to one year. Blood work will be obtained prior to each vaccination (see Evaluation During Study).

If a patient develops a severe reaction as defined as grade 4 toxicity (see Criteria for Toxicity) to the vaccine or deteriorating performance status necessitating hospitalization, that patient will not receive subsequent vaccination.

### Criteria for removal of study:

A. Progressive disease other than appearance of small (< 2 cm diameter) superficial cutaneous, subcutaneous or soft tissue metastases. If small superficial disease appears, the patient may continue with vaccination at the discretion of the investigator.
B. Severe intercurrent illness.
C. General or specific alteration in the patient's condition which would render further treatment as unacceptable in the judgment of the principal investigators.

### Formulation of vaccine

The established HLA-A2 melanoma cell line SK-MEL-29 infected with the retrovirus NAP-AD/IL2 (DC/AD/R/IL2) has been shown to secrete IL-2 in excess of 20 U/m. The stock IL-2-infected cell line is frozen in liquid nitrogen and stored in the laboratory of Dr. Bernd Gansbacher. Approximately one week before vaccination date, vials containing 10 million tumor cells (one vial for low dose and five vials for high dose) will be thawed, expanded in tissue culture, and reanalyzed for IL-2 production. These cultures will be shown to be free of helper virus, mycoplasma, bacteria, and fungus.

On the day of the vaccination, the cells will be trypsinized, counted, resuspended in 1 ml of sterile phosphate buffered saline (USP) into a sterile syringe. The syringe and its contents will be irradiated with 10,000 rads and delivered to the outpatient department for subcutaneous injection by a Principal Investigator or a designated co-investigator.

### Evaluation During study

1. Physical examination prior to each vaccination.
2. CBC, platelet count, and differential will be done prior to each vaccine.
3. Chemical profile including liver function tests, bilirubin, creatinine, and LDH will be done every four weeks for two months, then prior to each vaccine.
4. Peripheral blood will be obtained for immune assays (see section 6.9). Six green top tubes and two red top tubes will be obtained prior to the first vaccination and one to two weeks after the fourth vaccination.
5. Chest X-ray will be repeated every 4 weeks for two months, then prior to each vaccination.
6. If moderate toxicity (grade 3) is observed, pertinent tests will be performed more frequently than indicated in the table.

### EVALUATION OF IMMUNE RESPONSE

### Serological Studies

The most direct methods to measure immune responses to melanoma is detection of antibodies. Approximately one-third of patients with metastatic melanoma have antibodies that react with autologous tumor cells (Oettgen et al., 1991). Most of these autoantibodies are IgM but occasional antibodies are IgG class. Several antigens recognized by these autoantibodies have been defined at the molecular level, including the acidic glycoplipids GM2 and GD2, and the melanosomal glycoprotein gp75. The immunizing cell line SK-MEL-29 expresses GD2 and gp75. The detection of IgG antibodies induced by immunization is also a possible indirect measure of helper T cell responses, because induction of high affinity, mature IgG responses requires T cell help by CD4-positive T cells. All patients will have peripheral blood sera obtained for serologic analysis of antibodies directed against the SK-MEL-29 cell line (to include antibodies against GD2 and gp75) and autologous tumor cells when available. In addition, a control for a positive immune response with antibodies against allogeneic MHC class I and II antigens expressed by SK-MEL-29 will be done. It is expected that most patients will develop strong IgG responses against class I and II alloantigens expressed by SK-MEL-29. The development of IgG antibodies will provide an indirect measure of induction of a T cell help response against SK-MEL-29. Antibodies against cell surface and intracellular antigen will be detected by:
A. Immunoprecipitation of 1% NP40 cell lysates of SK-MEL-29 metabolically labeled with ³⁵- methionine.
B. Mixed hemadsorption assays for IgG and IgM antibodies directed cell surface antigens (23-27). Specificity of responses to MHC antigens will be tested using autologous AV Epstein-Barr virus transformed B lymphocytes or AV fibroblasts.

### Cellular Immune Responses

In selected patients where autologous tumor cells are available, MHC-restricted cellular immune responses can be evaluated. An attempt will be made to enter patients who have established cultures of autologous melanoma cell but these are not available for most patients. Although cellular immune responses are a critical measure of immune responses to melanoma, there are the following limitations: a) autologous tumor cells are necessary to detect responses; b) autologous normal cells and MHC-matched allogeneic cells are necessary to evaluate the specificity of responses; c) evidence of cytotoxic or helper responses are not evident without in vitro stimulation by autologous tumor cells - this requirement makes it difficult to discern T cells that have responded to tumor from precursor T cells that are capable of recognizing tumor without responding in vivo. Mononuclear cells from peripheral blood will be purified by Ficoll-Hypaque gradient centrifugation. Cells will be cultured with autologous tumor cells, interleukin-2 20-100 units/ml and autologous or allogeneic stimulator cells as described for 7-14 days (Wolfel et al., 1989). The following assays to assess cellular immune function will be done when autologous tumor cells are available.

### A. Helper T cell assays

Peripheral blood lymphocytes will be cultured with autologous tumor cells, autologous normal cells and allogeneic tumor cells (e.g. SK-MEL-29) for 48 hours. Proliferation will be measured by uptake of ³H-thymidine in a 4 hour pulse at the end of the assay. Additional measures are secretion of cytokines during co-cultivation with target cells. Interleukin-2 production is measured by enzyme-linked immunoassay (ELISA) and stimulation of the reference CTL cell line. Interferon-γ production is measured by ELISA. Release of additional cytokines can be measured by ELISA, including interleukin-4, interleukin-6, tumor necrosis factor-α, and granulocyte-monocyte colony stimulating factor.

### B. cytotoxic T cell assay

Cytotoxicity is measured in either a 4 hour or 18 hour ⁵¹-chromium release assay (as described in reference Wolfel et al., 1989).

### CRITERIA FOR A THERAPEUTIC RESPONSE

1. Complete Response (CR): Disappearance of all clinical evidence of tumor on physical examination, x-ray, and biochemical evaluation, for a minimum of 4 weeks.
2. Partial Response (PR): Greater than 50% decrease on physical examination or radiography (including CT scan and/or ultrasound) of the summed products of the perpendicular diameters of all measured lesions maintained for a minimum of 4 weeks. No simultaneous increase in size of any lesion or the appearance of any new lesion may occur.
3. Minor Response (MR): 25-49% decrease in the summed products of diameters of measured lesions for a minimum of 4 weeks.
4. Stable Disease (STAB): Less than 25% decrease or increase in tumor size for at least 3 months.
5. Progression (PROG): Greater than 50% increase in the sum of all measured lesions or appearance of new lesions. Note that if there is the appearance of superficial cutaneous, subcutaneous or soft tissue disease < 2 cm diameter, the patient may continue with vaccination at the discretion of the investigator.
6. Duration of response: measured form initiation of therapy until a 50% or greater increase from the smallest sum of all tumor measurements obtained during best response (CR, PR, MR or STAB).

### Criteria for Toxicity

Toxicity will be graded and classified according to Common Toxicity Criteria (Appendix) by the following outline:
- 0: No toxicity
- 1+: Mild toxicity, usually transient, requiring no special treatment and generally not interfering with usual daily activities.
- 2+: Moderate toxicity ameliorated by simple maneuvers.
- 3+: Severe toxicity which requires therapeutic intervention and interrupts usual activities. Hospitalization may or may not be required.
- 4+: Life-threatening toxicity which requires hospitalization. A toxicity which causes a drug-related death will be called a 4F.

### Adverse Experience

To date there have bene minimal side effects from vaccinations with allogenic tumor cells (Livingston, 1991). Patients have had induration and erythema at the site of the vaccination. There have been no reports of anaphylaxis to allogenic tumor cells in vivo. In the initial study of administration with genetically altered human cells to patients, no additional side effects were seen as a result of the procedure and no viable retrovirus was transmitted (Rosenberg et al., 1990).

Serious adverse effects of treatment will be reported to the Memorial Sloan-Kettering Cancer Center IRB as well as the NIH Office for Protection from Research Risks within 24 hours and a written report will follow within ten days. A copy will also e sent to the NIH Office of Recombinant DAN Activities.

### Biostatistical Considerations

The endpoint of this pilot study has been defined as clinical toxicity. As indicated in Treatment Plan, six patients are planned at each dose level. If three patients have grade III or IV toxicity is seen (as defined by the Common Toxicity Criteria), then a maximum tolerated dose will be defined and the study will be ended. All patients will be evaluated for immune response as described in Evaluation of Immune Response, although this evaluation will not be a measurable endpoint.

### Design and Sample Size

Approximately twelve patients will be enrolled in this pilot study to tumor cell vaccinations. Two dose levels of tumor cells will be uses.
- low dose:: 10 million tumor cells
- high dose:: 50 million tumor cells

The maximum tolerated dose will be defined as the dose of vaccine for which three or more patients have grade III or IV toxicity. The trial will be stopped at the determination of maximum tolerated dose or the completion of both dose levels. It is anticipated that an MTD may not be established in this study.

### Consent Procedures

All patients will be required to sign a statement of informed consent which must conform to IRB guidelines. This research study has been reviewed by the IRB of Memorial Sloan-Kettering Cancer Center as well as the NIH Recombinant DNA committee. The investigator will report to the IRB and the RAC changes in the research protocol and all unanticipated problems involving risks to human subjects or others, and no changes will be made in the research activity without IRB approval.

### Third series of experiments

### Retroviral Vector-mediated Lymphokine Gene Transfer into Human Renal Cancer Cells

### Introduction

The administration of cytokines can induce regression of primary and metastatic tumors in experimental animal models and patients (Malkovska et al. 1989). Growing evidence indicates that certain cytokines may demonstrate increased antitumor efficacy if they are delivered directly at the tumor site (Forni et al. 1988), and tumor cell-targeted cytokine gene therapy represents a novel approach to achieve this goal (Russel et al. 1990). Transfer of cytokine genes into tumor cells, leading to highly localized release of cytokines, approximates more closely the physiological topology of cytokine secretion in the course of an immune response. In fact, murine tumor cells genetically engineered to produce IL-2, (Gansbacher et al., 1990; Fearon et al., 1990b; Karasuyame et al., 1988), IL-4 (Tepper et al., 1989), IL-7 (Hock et al., 1991), IFN-γ (Gansbacher et al., 1990a; Myatake et al., 1990), tumor necrosis factor-a (Asher et al., 1991), or granulocyte colony stimulating factor (Colombo et al., 1991) demonstrated retarded or inhibited growth in vivo.

Retroviral-mediated gene transfer is an efficient method to introduce genes into mammalian cells, resulting in stable integration into the host cell genome and persistent expression of the encoded protein (McLachlin et al., 1990). Using this methodology, it was recently demonstrated that local secretion of human IL-2 or murine IFN-γ abrogated the tumorigenicity to the cytokine-producing murine CMS-5 fibrosarcoma (Gansbacher et al., 1990a; Gansbacher et al., 1990b), a weakly immunogenic, methylcholanthrene-induced tumor derived from BALB/c mice. Moreover, a long-lasting protective immunity against a subsequent tumor challenge was induced (Gansbacher et al., 1990a; Gansbacher et al., 1990b).

RCC remains a chemotherapeutically resistant tumor, and the reported median survival for patients with metastatic RCC is approximately 10 months (Maldazys et al., 1986). Recently, experimental and clinical evidence has shown that RCC is relatively responsive to various forms of immunotherapy (Graham 1989), including treatment with IL-2 with or without LAK cells (Rosenberg et al., 1987), and IFN-γ (Aulitzky et al., 1989). However, systemic administration of therapeutically effective cytokine doses is frequently associated with severe toxicity (Rosenberg et al., 1987). A possible means to improve immunotherapy of RCC would be to target cytokine release directly to tumor cells in vitro. To achieve this, human RC cell lines was transduced with the cDNAs for human IL-2 or IFN-γ by using retroviral vectors. In this report, it was demonstrated that the successful expression of IL-2 and IFH-γ in human RC cells. Using these cells, the influence of lymphokine gene transfer on the antigen profile of the tumor cells and the effect of high-dose γ-irradiation on tumor cell viability, cell surface phenotype, and lymphokine production in vitro was examined. Finally, the tumorigenicity of xenografted parental and lymphokine-releasing RC cells in a nude mouse model was investigated.

### Materials and Methods

### Cell Lines and Tissue Culture

Seven human RC cell lines were selected for this study. The cell lines SK-RC-1, -9, -28, -29, -39, -44, and -49 were established from primary nephrectomy specimens or metastatic lesions as described previously (Ebert et al., 1990). Several cell surface markers for these cell lines are summarized in Table 4. Cells were maintained in Eagle's minimum essential medium supplemented with 2mM glutamine, 1% nonessential amino acids, 100 units/ml of streptomycin, 100 units/ml of penicillin, and 10% FBS.

### Retroviral Vector Design and Infection of Tumor Cells.

DC/AD/R/IL2 was generated by cloning a minigene containing the human ADA promoter, IL-2 cDNA, poly-A sequences into the 3' long terminal repeat in reverse orientation. The ADA promotor is a 832-base pair fragment derived from the human ADA gene by digestion with restriction enzymes SspI and NcoI (Wingington et al., 1983). The poly-A signal containing a 275-base pair-long SmaI-AluI fragment was derived from plasmid PBC/CMV/IL (Cullen 1986). A 528-base pair-long DNA fragment encoding the human IL-2 cDNA was obtained from the plasmid PBCII/RSV/ΔT (Cullen, 1988) by digestion with restriction enzymes RamHI and HindIII. An 852-base pair-long DNA fragment encoding the herpes simplex virus thymidine kinase promotor was obtained from plasmid PHSV106 (McKnight, 1980) by digestion with restriction enzymes RamHI and BglII. A 794-base pair-long DNA fragment encoding the major immediate early human cytomegalovirus promotor was obtained from plasmid PRR23 (Wano et al., 1987) by digestion with restriction enzymes BalI an SmaI. N2 is a retroviral vector derived from the genome of Moloney murine leukemia virus, containing the bacterial neomycin resistance (neo) gene, which is used as a selectable marker (Armentano et al., 1987). The TKIL2 and the TKIFNγ fusion products were cloned into the MluI site present in the polylinker within the 3' long terminal repeat of a modified N2 vector as described by Hantzopoulos et al. (Hantzopoulos et al., 1989), to generate the vector constructs DC/TKIL2 and DC/TKIFNγ, respectively. The CMVIFNγ fusion product was cloned into a unique XhoI site present downstream from the neo gene coding sequences to generate the vector construct N2/CMVIFNγ, respectively (Gansbacher et al., 1990a; Gansbacher et al., 1990b). A schematic diagram presenting the various vector constructs used in this study is shown in Fig. 7.

Retroviral constructs were converted to the corresponding virus, using established procedures. Briefly, vector DNA was transfected into the helper-free amphotropic packaging cell line AM12 (Markowitz et al., 1988). Colonies were isolated by G418 selection and expanded to cell lines, and cell-free supernatants were tested for the presence of virus. Cell lines secreting a high titer of virus (10⁵-10⁶ neo colony-forming units/ml) were isolated by G418 selection and expanded to cell lines, and secretion of IL-2 or IFN-γ into the cell supernatant was measured by bioassay and immunoassay. Absence of replication-competent virus in the cytokine-producing tumor cells was demonstrated by the inability to transfer G418 resistance to HIH 3T3 cells.

### Replication competent Murine Retrovirus Detection Assay.

The detect the presence of replicating murine retroviruses in cell culture supernatants of gene-modified RC cells, the NIH3T3 amplification assay was used. This assay is based on the finding that replication competent ecotropic and amphotropic retrovirus proliferates in NIH3T3 cells. Briefly, NIH3T3 cells were maintained in Dulbecco's modified Eagle's medium with 10% FBS and 5 mM L-glutamine; cells were grown at 1 x 10⁵ cells/well in 6-well dishes 12 h before sample exposure. One ml of the supernatant samples taken from gene-modified human RC cells was then added per well; polybrene (8 µg/ml) was used to enhance viral infection. Spent media from NIH3T3 cells and the packaging cell line AM12 as well as Dulbecco's modified Eagle's medium alone were included as negative controls; supernatants from helper virus producer cell lines SAX-2 and SAX-3, and the producer cell line AM12/ETAR29, were used as positive controls. After infection, the media were removed and 5 ml of fresh medium were added. Cells were split 1:5 when confluent and carried for 3 weeks. When plates were confluent at the end of the third week, fresh medium was added to the wells and collected 12 h later for determination of reverse transcriptase activity.

Reverse transcriptase activity was determined by incubating 10 µl of sample media with 40 µl 50 mM Tris, pH 8.0, 20 mM dithiothreitol, 0.6 mM MnCl₂, 60 mM NaCl, 0.05% Nonidet P-40, 5 µg/ml oligo dT, 10 µg/ml poly a, 62 µM dTTP, and 10 µM [³²P]dTTP (3000 Ci/m; ICN Biochemicals, Inc., Costa Mesa, CA) for 1 h at 37°C. Negative controls with added media alone were used to determine backgrounds. Ten µl of the reaction mixture were then spotted on a DEAE DE81 filter disk and washed twice with 1 x standard saline-citrate (0.15 M sodium citrate, pH 7.4) at 25°C for 30 min. After rinsing with 95% ethanol, the filter disks were air dried, and filter radioactivity was then detected by exposure to X-ray film for 3 to 5 h and quantitated by scintillation counting.

**Human IL-2 Assay.** Supernatants of semiconfluent parental or lymphokine-secreting tumor cells in a 6-cm plate were collected after 48-72 h and assayed for human IL-2 using IL-2-dependent human primary lymphoblasts in a proliferation assay as described previously (Gansbacher et al. 1990b). The results obtained from the bioassay were confirmed by enzyme-linked immunosorbent assay (Genzyme, Boston, MA). DMS-1, a neutralizing mAB to human IL-2 (Smith et al., 1983), was used to inhibit IL-2 activity in the bioassay.

**Human IFN-γ Assay.** Supernatants tested by the human IL-2 assay were also analyzed for human IFN-γ. the bioassay for human IFN-γ was based on the antiviral activity in the cell culture supernatant, as determined by te reduction of the cytopathic effects of vesicular stomatitis virus determined by the reduction of the cytopathic effects of vesicular stomatitis virus on WISH cells (Rubinstein et al. 1987). Results were confirmed using a commercially available radioimmunoassay of human IFN-γ (Centocor, Malvern, PA). Humans IFN-γ bioactivity in the supernatant was blocked by a neutralizing murine mAB specific for human IFN-γ (Olympus, Lake Success, NY).

**Cell Surface Antigen Analysis by Indirect Immunofluorescence and FACS.** Expression of kidney-associated antigens, MHC class I and class II determinants, ICAM-1(CD54), and VLA-β1 on RC cell lines was determined by indirect immunofluorescence (Ebert et al. 1990; Finstad et al., 1985). The following murine mAB were used as primary antibodies: URO-2, URO-3, URO-4, URO-8, and G250, all detecting kidney-associated antigens (Finstad et al., 1985; Bander, 1989); W6/32 (anti-HLA-A,B,C) (Brodsky and Parham, 1982); NAMB-1 (anti-β2-microglobulin; Boehringer Mannheim, Indianapolis, IN); anti-HLA-DR, -DP, and -DQ (Olympus); OKT 27 (anti-ICAM-1; Ortho Diagnostics, Raritan, NJ); and AJ2 (anti-VLA-B1) (Cairncross et al., 1982). Cells either plated on 60-well Terasaki plates or suspended in PBS, were incubated with primary antibody for 60 min, washed in PBS, and then incubated with affinity-purified fluorescein isothiocyanate-conjugated rabbit anti-mouse IgG (1:50 dilution; Dako Corp., Santa Barbara, CA) for 45 min. Cells attached to Terasaki plates were evaluated using a fluorescence microscope, and cells in suspension were analyzed using a FACS 440 system (Becon Dickinson FACS Division, Sunnyvale, CA). As a control, PBS was substituted for the mAB reagent.

**Irradiation of Cultured Cells.** Parental and lymphokine-producing SK-RC-29 cells (0.25 x 10⁶ cells/well) were plated 24 h prior to irradiation in Falcon 3045 multiwell tissue culture plates (Falcon Labware, Oxnard, CA) and irradiated at room temperature with a 6-MV Varian CL6-100 linear accelerator at a dose rate of 100 rad/min. Test doses of 5,000 or 10,000 rad were applied. Irradiated cells were cultured for 22 days at 37°C in a 5% CO₂ atmosphere. Three, 12, and 22 days after irradiation culture supernatants were collected and the cells were refed every third day with 1 ml/well of fresh minimal essential medium containing 10% FBS. Spent media were frozen at -20°C until assaying. In parallel, cells from triplicate cultures were trypsinized 1, 12, and 22 days following irradiation, and the number of viable cells was assessed by light microscopy using trypan blue exclusion. Irradiated cells cultured in separate wells were harvested 1, 7, and 14 days after irradiation for FACS analysis.

**Tumor Formation in Nude Mice.** SK-RC-29 cells (0.005 x 10⁶, 0.05 x 10⁶, or 0.5 x 10⁶) were injected s.c. into 4-to 6-week-old male nude mice of BALB/c origin. This RC cell line has been reported to form nonmetastasizing s.c. tumors in BALB/c nude mice (Ebert et al., 1990). Five weeks after injection, the animals were sacrificed and the tumor weight was determined.

**Immunohistochemical Analysis.** Five weeks after s.c. injection of parental or gene-modified SK-RC-29 cells into BALB/c nude mice, tumors were removed, snap-frozen in liquid nitrogen, embedded in OCT compound (Miles, Inc. Eklhart, IN), and stored at -70°c until used. Cryostat tissue sections (6 µm) were air-dried, fixed in cold acetone, and treated with 0.03% H₂O₂ to remove endogenous peroxidase activity. For detection of endogenous peroxidase-positive cells, pretreatment with H₂O₂ and staining with primary mAB were omitted and diaminobenzidine was immediately added onto tissue sections. In parallel, tissue sections were stained by the indirect immunoperoxidase method described previously (Finstad et al., 1985). Briefly, sections were incubated for 90 min with a biotinylated mouse mAB specific for HLA-DR (Olympus), or with a biotinylated rat mAB detecting murine tissue macrophages (clone AL-21; Pharmingen, San Diego, CA). As a control, PBS substituted for primary mAB. After washing sections with PBS, biotinylated antibodies were detected by incubation with peroxidase-conjugated avidin (1:400 dilution; Dako Corp.) for 45 min. Antigen-antibody complexes were visualized using diaminobenzidine in the presence of H₂O₂ as the chromogen and Harris' hematozylin as counterstain.

### Experimental Results

### Analysis of Gene-Modified RC Cells for the Presence of Replication Competent Murine Retrovirus

Replication competent murine retrovirus may arise during the experimental procedures by recombination in human cells with endogenous retroviral-like particles. Therefore, the presence of replication competent murine retrovirus was assayed in spent media from RC cell lines SK-RC-9, -28, -29, and -39, which had been transduced 21 to 28 days before with DC/AD/R/IL2. No replication competent murine retrovirus wa detected using the NIH/3T3 amplification assay (Figure 8).

**Expression and Release of IL-2 or IFN-γ from Gene-modified RC Cells.** Two retroviral vector-constructs containing the human IL-2 cDNA and 2 constructs carrying the human IFN-γ cDNA were used to transfer these lymphokine genes into human RC cells (Figure 7). seven human RC cell lines expressing various kidney-associated antigens were chosen for this study (Table 4).

**TABLE 4 -**

| Cell surface antigen phenotype of human RC cell lines^{a} | | | | | | | | | | |
|---|---|---|---|---|---|---|---|---|---|---|
| **Cell line** | **UR 02** | **UR 03** | **UR 04** | **UR 08** | **G2 50** | **HLA ABC** | **β-2M** | **HLA DR** | **ICAM- 1** | **VLA β1** |
| SK-RC-1 | + | + | + | + | + | + | + | - | + | + |
| SK-RC-9 | - | - | + | - | + | + | + | - | - | + |
| SK-RC-28 | + | - | + | - | + | + | + | - | + | + |
| SK-RC-29 | - | + | + | + | - | + | + | - | + | + |
| SK-RC-39 | - | - | + | + | - | + | + | - | + | + |
| SK-RC-44 | - | + | + | + | - | + | + | - | + | + |
| SK-RC-49 | - | ± | ± | + | - | + | + | - | + | + |

| | | | | | | | | | | |
|---|---|---|---|---|---|---|---|---|---|---|
| ^{a} As determined by indirect immunofluorescence (29). Cells were evaluated for cell-surface staining by fluorescence microscopy using a panel of mouse mAB. Staining was evaluated as follows: +, strong homogenous reactivity; ±, heterogenous reactivity: -, negative | | | | | | | | | | |

Data on the infected cell lines and the vector-constructs used are summarized in Table 5.

**Table 5**

| Lymphokine release by vector-transduced human RC cell lines^{a} | | | |
|---|---|---|---|
| Cell line | Viral Construct | IL-2 (units/10⁶ cells/48 h) | IFN-γ (units/10⁶ cells/48h) |
| SK-RC-1 | DC/AD/R/IL2 | 7 | |
| SK-RC-9 | DC/AD/R/IL2 | 29 | |
| SK-RC-28 | DC/AD/R/IL2 | 10 | |
| SK-RC-44 | DC/AD/R/IL2 | 20 | |
| SK-RC-49 | DC/AD/R/IL2 | 4 | |
| SK-RC-29 | DC/TKIL2 | 10 | |
| SK-RC-29 | N2/CMVIFNγ | | 10 |
| SK-RC-29 | DC/TK1FNγ | | <1 |

| | | | |
|---|---|---|---|
| ^{a} IL-2 and IFN-γ levels in cell culture supernatants represent values (mean of duplicates) obtained from the IL-2 bioassay and IFN-γ radioimmunoassay. The parental RC cell lines did not release detectable amounts of IL-2 or IFN-γ | | | |

Secretion of IL-2 or IFN-γ was determined by measuring lymphokine concentrations in cell culture supernatants using an appropriate bioassay. The results were then confirmed with an enzyme-linked immunosorbent assay for IL-2 or a radioimmunoassay for IFN-γ. RC cell lines infected with the retroviral constructs carrying the IL-2 cDNA were found to release variable amounts of bioactive IL-2 consistent with previous findings (Gansbacher et al., 1990b). SK-RC-29 cells modified with the construct N2/CMVIFNγ released moderate amounts of IFN-γ. Thus, in agreement with the previous findings (Gansbacher et al., 1990a; Gansbacher et al., 1990b), target cell type as well as the design of the vector construct are important parameters when determining the efficiency of expression of transferred lymphokine genes.

**Effect of Lymphokine Secretion by Tumor Cells on Their Antigen Profile.** Tumor-associated antigens. MHC, and adhesion molecules are critical determinants of tumor cell immunogenicity. In order to evaluate the effect of lymphokine secretion on such markers expressed by human RC cells, the antigen profile of parental RC cell lines and their lymphokine secreting variants was examined by indirect immunofluorescence and FACS analysis. As a representative example, the findings with cell line SK-RC-29 cells will be described in detail (Table 6).

**Table 6 -**

| Cell surface expression of MHC antigens and adhesion molecules on parental and lymphokine-screening SK-RC-29 cell^{a} | | | | | | | |
|---|---|---|---|---|---|---|---|
| Vector construct in SK-RC-29 cells | HLA -ABC | 6-2 Micro-globulin | HLA -DR | HLA -DP | HLA -DQ | ICAM -1 | VLA-B1 |
| None^{b} | 141 | 117 | 0 | 0 | 0 | 74 | 94 |
| DC/TKIL2 | 141 | 117 | 0 | 0 | 0 | 77 | 97 |
| N2/CMVIFNγ | 161 | 140 | 20 | 35 | 0 | 92 | 97 |
| DC/TKIFNγ | 140 | 121 | 6 | 7 | 0 | 76 | 93 |

| | | | | | | | |
|---|---|---|---|---|---|---|---|
| ^{a} As determined by indirect immunofluorescence using a FACS; as negative control, PBS was substituted for the primary antibody reagent. | | | | | | | |
| ^{b} Unmodified parental SK-RC-29 cells ^{c} Delta mean fluorescence intensity in arbitrary units on a logarithmic scale (test sample - negative control) | | | | | | | |

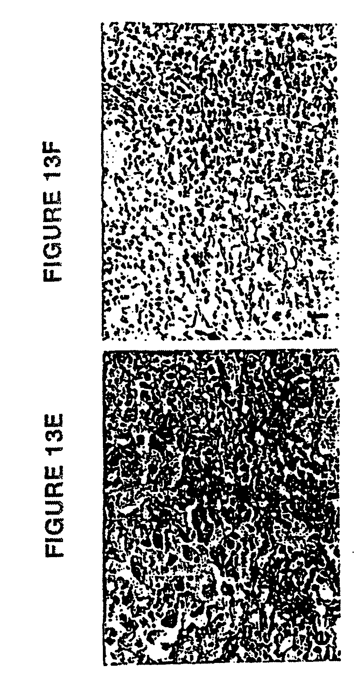

express constitutively various kidney-associated antigens, MHC class I antigens, and cell adhesion molecules, but do not express MHC class II determinants (Table 4). The expression of kidney-associated antigens on SK-RC-29 cells was not affected by expression of either lymphokine (data not shown). Similarly, IL-2 releasing SK-RC-29 cells carrying the CD/TKIL2 construct had no detectable effect on the expression of MHC antigens and adhesion molecules (Table 6). However, efficient expression of IFN-γ by SK-RC-29 cells carrying the N2/CMVIFNγ construct was associated with increased expression of HLA-ABC, β2-microglobulin, and ICAM-1, as well as neo-expression of HLA-DR and -DP molecules, HLA-DR expression was detectable on IFN-γ producing SK-RC-29 cells grown in vitro and in vivo (see Figure 13e). The marker profile of SK-RC-29 cells harboring the DC/TKIFNγ construct and releasing only minimal amounts of IFN-γ was indistinguishable from that of the parental cells (Table 3).

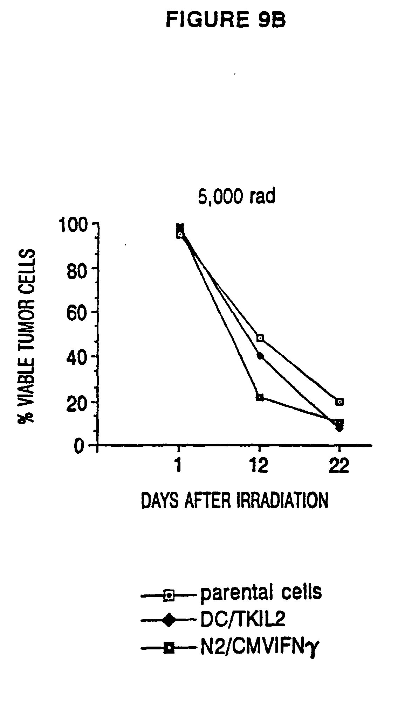

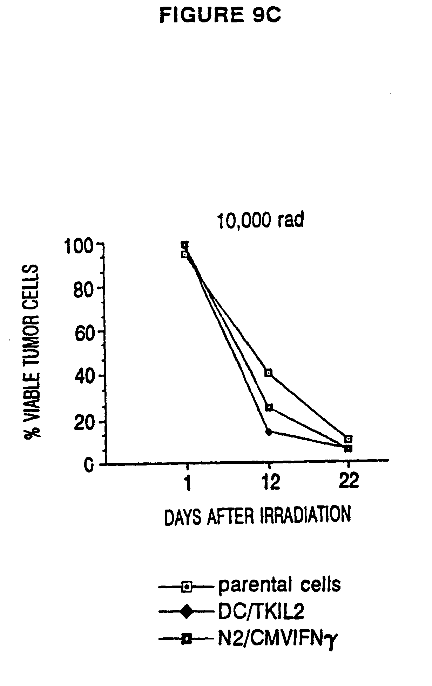

**Survival, Lymphokine secretion, and immunogenicity of RC Cells Following High-Dose γ-Irradiation.** Future vaccination of patients with lymphokine-secreting allogeneic or autologous tumor cells will require methods to prevent tumor formation by gene-modified tumor cells in vivo. Such safeguard procedures should, however, not diminish the capability of gene-modified tumor cells to secrete lymphokines and should not abolish their immunogenicity. High-dose γ-irradiation offers an approach to achieve this goal. Therefore the effect of γ-irradiation on survival, lymphokine production, and HLA-DR antigen expression of lymphokine gene-modified SK-RC-29 cells was examined. Parental and lymphokine-releasing SK-RC-29 cells were plated at high cell density (0.25 x 10⁶ cells/ml) in multiwell plates and irradiated with 5,000 or 10,000 rad. The survival curves of irradiated parental and lymphokine-producing SK-RC-29 cells are illustrated in Fig. 9. Twenty-two days after irradiation, 13% (means range, 6-20%) of the lymphokine-secreting tumor cells were still viable as determined by trypan-blue staining, but these surviving cells remained growth-arrested and died within 5 weeks after irradiation (data not shown).

To determine the effect of γ-radiation on lymphokine release, the concentration of IFN-γ in cell supernatants collected before and 3, 12 and 22 days following irradiation were measured. As shown in Fig. 10, secretion of IFN-γ was even enhanced at days 3 and 12 (after exposure to 5,000 rad) and decreased until day 22, suggesting ongoing biosynthesis and release of this lymphokine in growth-arrested tumor cells surviving γ-irradiation.

In order to evaluate the effect of irradiation on the expression of lymphokine-induced cell-surface antigens, expression of HLA-DR antigen on parental and IFN-γ producing SK-RC-29 cells was examined before and 1, 7, and 14 days after γ-irradiation. In parallel with sustained release of IFN-γ from irradiated SK-RC-29 cells carrying the N2/CMVIFNγ construct, more intense staining for HLA-DR was found on these cells after irradiation (Fig. 11B). γ-Irradiation, however did not induce neo-expression of HLA-DR on parental (Fig. 11A) or IL-2-secreting cells (data not shown).

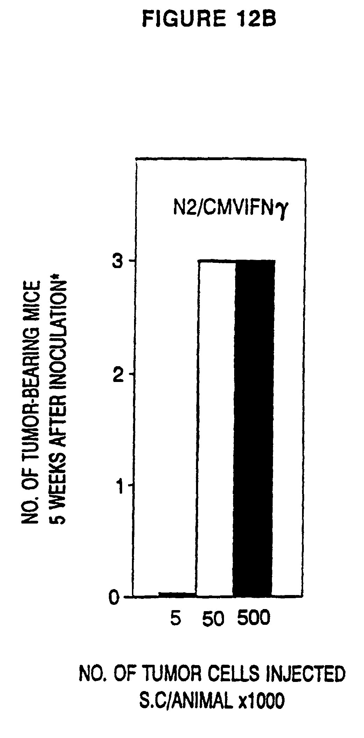

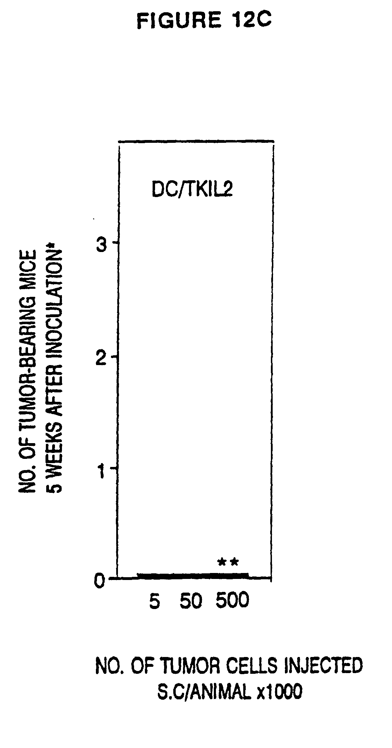

**Effect of Retroviral-mediated Gene Transfer on Tumorigenicity of RC Cells in Nude Mice.** Parental SK-RC-29 cells have been shown to form tumors in BALB/c nude mice (Ebert et al., 1990). Therefore, this cell line was chosen to study the effect of lymphokine production by tumor cells on the tumorigenicity of parental and lymphokine-secreting SK-RC-29 cells. Four- to 6-week-old male BALB/c nu/nu mice were given s.c. injections of various numbers of parental and vector-modified SK-RC-29 cells, and tumor formation was evaluated after 5 weeks. The results of one set of experiments ar summarized in Fig. 12. Parental as well as lymphokine-producing SK-RC-29 cells administered at the lowest dose level (0.005 x 10⁶ cells/mouse) failed to generate tumors, and during 12 weeks of observation these mice remained tumor-free.

However, injection of 0.05 x 10⁶ or 0.5 x 10⁶ or 0.5 x 10⁶ tumor cells/mouse let to a striking difference in tumor formation by animals receiving either parental or lymphokine-producing SK-RC-29 cells. Whereas parental and human IFN-γ secreting SK-RC-29 cells formed large s.c. tumors, mice given injections of IL-2 releasing SK-RC-29 cells showed no visible tumors macroscopically. This finding was reproducible in many other experiments during which different RC or melanoma cell lines transduced with IL-2 vector were infected into nude mice (Gansbacher et al., 1992). Microscopic examination of the injection site in animals inoculated with the highest dose of IL-2 producing tumor cells revealed small tumor cell aggregates, which by immunohistology showed a strong, peritumoral infiltrate of peroxidase-positive mononuclear cells (Fig. 13b). Using a rat mAB specific for murine tissue macrophages, a major proportion of these tumor-infiltrating host cells stained positive (Fig. 7d). In contrast, such peri- or intratumoral accumulations of tumor-infiltrating cells were not observed in animals developing tumor nodules derived from parental or IFN-γ releasing SK-RC-29 cells (Fig. 13, a and c). Mice inoculated with SK-RC-29 cells carrying the DC/TKIL2 construct when observed for 12 weeks remained macroscopically tumor-free.

### Experimental Discussion

Several approaches may be utilized in an attempt to stimulate host antitumor immunity. One possibility uses the systemic administration of cytokines or the adoptive transfer of LAK cells into the tumor-bearing host (Rosenberg et al., 1987). Alternatively, the tumor cells themselves and the host immune system can be manipulated in situ to elicit a host antitumor response (Forni et al., 1988; Russel et al., 1990). Following the second approach, it was recently reported on a strategy to induce specific cell-mediated antitumor immunity in experimental animals by tumor-cell targeted cytokine therapy. Constitutive secretion of IL-2 (Gansbacher et al., 1990b; Fearson et al., 1990). IL-4 (Tepper et al., 1989), IL-7(Hock et al., 1991), and IFN-γ (Gansbacher et al., 1990a; Myatake et al., 1990) from genetically modified murine tumor cells abrogated their tumorigenicity in syngeneic animals and induced a specific and persistent T cell-mediated antitumor response in vivo. In clinical studies, lymphokines, IL-2, and IFN-γ, proved to induce remissions in a subset of patients with advanced RCC (Graham, 1989; Rosenbert et al., 1987; Aulitzky et al., 1989, Topalian et al., 1988). In the majority of patients, however, systemic high-dose lymphokine therapy was ineffective and associated with severe toxicity (Graham, 1989; Rosenberg et al., 1987; Topalian et al., 1988).

Since local delivery of lymphokines by tumor cells provides an attractive, alternative approach for tumor immunotherapy, RCC was chosen as a model disease to determine the feasibility of introducing lymphokine genes into human RC cells. In this report, retroviral vector mediated transfer of cDNAs for IL-2 or IFN-γ into human RC cells is demonstrated to be feasible and results in the elaboration of biologically active lymphokines. Both cytokines, IL-2 (Rees and Wiltrout, 1990) and IFN-γ (Trinchieri et al., 1985), are physiologically produced by activated helper T lymphocytes and play a critical role for eliciting cell-mediated immune responses. IL-2 stimulates the proliferation and lytic capacity of NK (Trinchieri, 1989) and T lymphocytes (Erard, 1985), and induces the differentiation of NK cells into LAK cells (Grimm et al., 1982). Beldegrun et al. (Belldegrun et al., 1988) were able to demonstrate strong tumoricidal activity against autologous RC cells in tumor-infiltrating lymphocytes activated with IL-2 in-vitro, IL-2, however, has no direct cytotoxic or cytostatic effect on neoplastic cells. IFN-γ is a pleiotropic lymphokine that promotes the activation and differentiation of cytotoxic T lymphocytes (Chen et al., 1986; Catalon et al., 1981) and amplifies the tumoricidal activity of NK cells (Catalona et al., 1981) and macrophages (Adams and Hamilton, 1987). In addition, IFN-γ induces or enhances the expression of MHC class I and class II antigens (Wallach et al., 1982) and cell adhesion molecules (Tomita et al., 1990; Mortarini et al., 1990). The up-regulation of these cell surface molecules can enhance the antigen-presenting capacity of malignant cells (Ostrand-Rosenbert et al., 1990; Alexander et al., 1989) and increase their susceptibility to lysis by cytotoxic T lymphocytes (Hayashi et al., 1985; Vanky et al., 1988). Moreover, IFN-γ mediates a direct antiproliferative effect on some types of tumor cells (Wadler and Schwartz, 1990).

Two important aspects of lymphokine and tumor biology are considered in this study: (a) regulation of expression of immunoregulatory cell surface molecules on tumor cells; and (b) lymphokine-induced modulation of tumor growth in vivo. MHC class I antigen expression in IFN-γ secreting murine CMS-5 cells was reported recently to be upregulated and possibly responsible for their reduced tumorigenicity in syngeneic mice, since tumor rejection was associated with a strong tumor-specific cytotoxic T cell response (Gansbacher et al., 1990a). As shown by this study, IFN-γ secreted by SK-RC-29 cells not only amplifies MHC class I antigenexpression but also induces expression on MHC class II molecules (HLA-DR and -DP) and up-regulates ICAM-1 expression. The modulation of MHC class II and ICAM-1 antigens on tumor cells appears to be particularly important since: (a) human tumor cells expressing MHC class II antigens can acquire the capacity for presenting soluble antigens to helper T lymphocytes (Vanky et al., 1988); and (b) more efficient antigen presentation has been obtained with antigen-presenting cells coexpressing HLA-DR and ICAM-1 (Altmann et al., 1989). These studies suggest that, if tumor-specific neo-antigens are expressed by human tumor cells, the concomitant expression of MHC class I and class II determinants as well as ICAM-1 could make them accessible for MHC-restricted helper (Ostrand-Rosenberg et al., 1990; Alexander et al., 1989) and cytotoxic T lymphocytes (Hayashi et al., 1985; Vanky et al., 1988). This could enable the host immune system to generate a cell-mediated antitumor response in vivo.

The effect of local lymphokine release on tumor formation by xenografted human RC cells was examined in a nude mouse model. In vitro studies revealed that under no circumstances was the growth rate of human RC cells reduced by retroviral transfer and expression of the human IFN-γ or IL-2 gene (data not shown). However, the tumorigenicity of human SK-RC-29 cells s.c. injected into BALB/c nude mice was abrogated by local release of human IL-2 (Fig. 12). Notably, secretion of even low amounts of human IL-2 from s.c. injected SK-RC-29 cells was sufficient to locally attract macrophges and to suppress tumor formation (Fig. 13, b and d). In this context, it is noteworthy that IL-2 has previously been shown to stimulate the cytolytic activity of human (Malkovsky et al., 1987) and murine (Verstovsek et al. 1992) macrophages in vitro, and to enhance the proliferaton and differentiation of murine macrophage precursors in vivo (Baccharini et al., 1989).

In nude mice inoculated with parental or IFN-γ releasing SK-RC-29 cells, large tumors devoid of tumor-infiltrating host cells developed (Fig. 13, a and c). This finding is consistent with the previous observation that parental SK-RC-29 cells are tumorigenic when injected into nude mice (Ebert et al., 1990), and that human IFN-γm unlike IL-2, is species-specific and not capable of exerting immunoregulatory functions in mice (Langer and Pestka, 1988).

For the initial clinical study of lymphokine-secreting human RC cells as a live-cell vaccine, safeguards are required to abolish any tumorigenic capacity. High-dose γ-irradiation offers a feasible method to abolish the growth potential of RC cells while retaining their metabolic activity and antigenicity over a limited time. In vitro γ-irradiation with 5,000 or 10,000 rad killed 100% of SK-RC-29 cells within 5 weeks. However, although growth-arrested, a significant proportion of lymphokine-releasing tumor cells survived for 3 weeks (Fig. 9). As reported in a separate paper (Gansbacher et al., 1992), the pattern of IL-2 or IFN-γ secretion by lymphokine gene-modified human tumor cells following γ-irradiation was almost identical. The lymphokine release from irradiated tumor cells lasted for approximately 3 to 4 weeks. This time may be sufficient to provide local lymphokine release and immunostimulation in vivo.

Tests were performed to ensure that the transduced RC cell lines do not release replication competent helper viruses. Using the NIH3T3 amplification assay, which is capable of detecting a single replication competent viral particle per ml test medium, no replication competent virus was found to be produced by gene-modified human RC cell lines has been shown to be free of human immunodeficiency virus, cytomegalovirus, and hepatitis viruses.

These data demonstrate that retroviral vector-mediated gene transfer represents a feasible and safe approach to introduce lumphokine genes into human RC cells. While preserving cell surface phenotypic and functional characteristics of lymphokine-gene modified tumor cells, high-dose γ-irradiation offers a simple means to generate a safe live-cell vaccine for in vivo immunization. The effect of IL-2 or IFN-γ released locally from RC cells on host immune cells and the up-regulation of immunoregulatory cell surface molecules on RC cells could trigger a local as well as a systemic antitumoral immune response in vivo. In view of recent data showing that untreated cancer patients already possess circulating tumor-specific T cells (Coulie et al., 1992), active immunization with such lymphokine gene modified tumor cells may increase the number of these effector cells. this assumption is strongly supported by recent experimental data obtained from P815 mastocytoma bearing mice, where the frequency of circulating tumor-specific precursor and mature cytotoxic T lymphocytes could be significantly enhanced by vaccination with tumor cells transfected with the murine IL-2 cDNA (Ley et al., 1991). Moreover, in immunized animals this cellular immune response was closely correlated with tumor rejection and long-term tumor-specific immunity.

In summary, progress has been made in tumor vaccine development. Cytokine-secreting tumor cells are now available for clinical use in vaccination protocols.

Thus, phase I studies determining the feasibility and safety of this novel therapeutic approach are warranted.

### Fourth series of experiments

### A PILOT STUDY OF IMMUNIZATION WITH INTERLEUKIN-2 SECRETING ALLOGENEIC HLA-A2 MATCHED RENAL CELL CARCINOMA CELLS IN PATIENTS WITH ADVANCED RENAL CELL CARCINOMA

To immunize HLA-A2 positive renal cell carcinoma patients with HLA-A2 matched allogeneic renal cell carcinoma cells that have been altered to secret interleukin-2. Toxicity of immunization, evidence for induction of humoral and cellular immunity and Anti-tumor effects will be evaluated.

### Background of Invention

### Renal Cell Carcinoma

Renal cell carcinoma (RCC) is the fourth most common genitourinary cancer in the United States, with an annual incidence of 20,000 cases and death rate of 8,000 to 10,000. The natural history and clinical manifestations of RCC are variable (Javadpour, 1982; Lang et al., 1982; Marshall and Powell, 1982; Robson et al., 1964; Van Der Wef-Messing, 1973). Approximately one-half of the new cases are localized to the kidney, and 30% of patients present with distant metastases. A radical nephrectomy is a curative therapy for localized RCC, with a five year survival of 66% for patients with stage I-II disease and 35% for stage III disease (Robson, et al. 1964; Skinner, et al. 1972; Boxer, et al. 1979). Selected patients with state IV disease, those with solitary metastases, appear to show a survival benefit from surgical intervention.

Systemic therapy for metastatic RCC has limited effectiveness. Hormonal therapy with antiandrogens, antiestrogens, androgens, and progestins has been investigated, with reported response proportions of less than 2% (Bloom, 1973; Hrushesky, et al. 1977; Yogada, et al. 1982). Chemotherapy is generally ineffective, with more than 30 drugs investigated, alone or in combination, and none achieving a response proportion greater than 15-20% (Hrushesky, 1977; Yagoda, 1982). Of note, spontaneous regressions are well-recognized occurrence in as many as 7% of patients with advanced RCC (Oliver, et al. 1988). This phenomenon has prompted the investigation of immunotherapy. The most promising interventions so far have been immunologic, in particular the cytokines. The interferons and interleukin-2 have been reported to elicit antitumor effects in renal cell carcinoma (Quesada, 1988). However, the majority of trials have reported response proportions of less than 20% (Krown, 1987).

### Vaccination Studies

For many years it has been hoped that vaccination against cancer may specifically sensitize the host immune system and induce an antitumor response. At MSKCC, a variety of tumor vaccine studies aimed at inducing or augmenting a humoral or cellular antitumor response have been completed. Most of these studies have been done in patients with melanoma. Since there is more experience on the use of vaccines for melanoma, this trial will be based in part on the observations that have been made for melanoma.

In the initial trials with autologous and allogenic melanoma cell vaccines, Livingston et al. (1979) tested for augmentation of cell mediated cytotoxicity against cultured melanoma cells. No augmentation was seen and the specificity analysis of preexisting cell mediated cytotoxicity was found to be difficult. Consequently, emphasis was placed on serologic analysis. In the analysis of reactions of patient's sera with autologous cultured melanoma cells, three classes of cell surface antigens have been defined (Livingston, 1979). Class I are unique antigens detected only on autologous cells. Class II are restricted differentiation antigens shared by many melanoma cell lines. Class III are nonspecific antigens seen on normal cells and other non-melanoma tumor cells.

Cytotoxic T cells that kill allogenic tumor cells have been found to be class I restricted. These cytotoxic T lymphocytes could kill HLA matched but not non-HLA matched allogenic melanoma cells (Hoon, et al. 1991; Hayashi, et al. 1991). Further evidence for shared tumor antigens is growing. Houghton (unpublished data) has noted that CD 4+ cells can kill both autologous and allogeneic tumor cells and these recognize shared antigens restricted HLA-DR 15. (Crowley et al. 1990, 1991) demonstrated that autologous tumor-specific cytotoxic T-lymphocytes (CTL's), when generated by repeated stimulation with autologous melanoma cells and expanded in the presence of interleukin-2, were major histocompatibility restricted (Crowley et al. 1990; Crowley et al. 1991).

Additional experiments suggested that these CTL's recognized a tumor-associated antigen in the presence of HLA-A2. Allogeneic HLA-A2 matched melanoma cells were substituted for autologous tumor cells in the generation of CTL's. These lymphocytes lysed the autologous tumor cells to the same degree the CTL's generated by stimulation with autologous tumor cells. Thus, tumor specific CTL's could be generated using HLA-A2 matched allogeneic melanoma cells. Since growing autologous tumor cells in culture is a time intensive procedure, allogenic HLA-A2 cells could be used to generate autologous cytotoxic T cells.

### Biologic Effects in vitro of Interleukin-2

Interleukin-2 is a lymphokine that is produced by T helper cells and stimulated T cells and natural killer (NK) cells. In vitro IL-2 has been shown to cause proliferation of antigen specific T cells. Helper, cytotoxic, and suppressor T cells have been immortalized by repetitive culturing in vitro in the presence of IL-2 (Roehm, et al. 1983). B cell responses in vitro are also enhanced by IL-2 (Basham, et al. 1983). Interleukin-2 can also stimulate secretion by T cells of other lymphokines including gamma interferon.

### Interleukin-2 in Human Studies

Within the last ten years, Rosenberg et al. have conducted several studies with systemic IL-2 in order to enhance lymphocyte function (Rosenberg, 1985; Rosenberg, et al. 1987; Rosenberg, et al. 1989). Indeed, tumors such as renal cell carcinoma and melanoma have shown activity to intravenous IL-2 with a 20-30% response rate (Rosenberg, et al. 1987). The major effect of IL-2 was on the expansion of effector cell populations which have the capability to kill the tumor cells in vitro. However, because of the short serum half life of IL-2, high doses have to be used which are very toxic. Hypotension and capillary leak syndrome with decreased systemic vascular resistance can lead to interstitial pulmonary edema, pre-renal azotemia, cardiac arrhythmias, and myocardial infarction (Rosenberg, 1985; Rosenberg, et al. 1987; Rosenberg, et al. 1989).

### Gene Transfer in Animal Models

Several laboratories have shown that the introduction of cytokine genes, such as IL-2, IL-4 (Tepper, et al. 1989), IFN-gamma (Watanabe, et al. 1989; Gansbacher, et al. 1990), TNF (Asher, et al. 1991) and G-CSF (Colombo, et al. 1991) into tumor cell leads to their rejection in an immunologically competent host. Secretion of G-CSF or IL-4 leads to a localized inflammatory response at the tumor site without induction of immunological memory (Tepper, et al. 1989; Colombo, et al. 1991). IL-2, interferon-gamma, and tumor necrosis factor on the other hand induce a long lasting cellular immune response which leads to the rejection of potentially lethal tumor challenges at a later time point (Gansbacher, et al. 1990; Watanabe, et al. 1989; Asher, et al. 1991). Initial studies by Tepper, et al (1989) demonstrated the potent effect of localized IL-4 secretion at the tumor site characterized by an accumulation of eosinophils and macrophages. Fearon et al. (1990) introduced the IL-2 gene into a poorly immunogenic murine colon carcinoma. The IL-2 secreting tumors were rejected and induced an effective cytolytic T cell response. Depletion of CD 8+ cells in vivo abrogated the cellular antitumor response implication the pivotal role of class I restricted CD 8+ cells.

To approximate more closely the physiologic mode of cytokine secretion in the course of an immune response, the IL-2 gene was directly introduced into a murine fibrosarcoma cell line (CMS5). In this way a localized secretion of low levels of cytokine was achieved which led to the regression of the tumor (Gansbacher, et al. 1990; Gansbacher, et al. 1990). While control mice were killed by tumor within 45 days, mice carrying the IL-2 secreting tumor not only rejected the implanted tumor and were cured, but also developed immunological memory. Lethal doses of tumor cells implanted after several months were rejected, while unrelated tumor grew and killed these mice, demonstrating the specificity of the response. In addition, lymphocytes taken from the spleens were capable of killing the tumor in vitro through the life span of the immunized mouse. Of note, weak cytolytic responses were generated by unmodified tumor cells; these were transient and disappeared three weeks after tumor implantation. However, mice injected with IL-2 secreting tumor cells generated tumor specific cytolytic activity lasting for over 75 days.

Several of these findings have been reproduced at MSKCC in different tumor types such as murine melanoma B16, murine B cell lymphoma 38C13, and murine bladder carcinoma MBT 2. Additional experiments using irradiated IL-2 secreting CMS5 cells demonstrated that there was no difference in their ability to induce immunological memory in vivo as compared to non-irradiated IL-2 secreting tumor cells. Rosenberg et al. (unpublished data) reproduced these results in the poorly immunogenic tumor MCA-102. Despite many attempts they were never able to generate a cellular antitumor response using unmodified tumor cells. Introduction of the IL-2 gene into these cells increased their immunogenicity to a level which generated tumor rejection and memory. In addition, coinjection of the IL-2 producing tumor cells with unmodified tumor cells produced an immune response strong enough to reject not only the lymphokine secreting tumor cells but the unmodified tumor cells as well.

In summary, the introduction and constitutive expression of IL-2 by tumor cells themselves increased the immunogenicity of a variety of histologically different cancer cells. It appears from these studies that the localized persistent expression and secretion of low levels of IL-2 may have an impact on the host antitumor response in the absence of any detectable toxicity.

### Gene Transfer in Humans

Most recently, Rosenberg et al. have taken genetically altered autologous tumor infiltrating lymphocytes and injected them into patients with refractory metastatic tumors (Rosenberg, et al. 1990). A retroviral vector containing the neomycin resistance gene was introduced into autologous lymphocytes in order to monitor them when reinfused into patients. Of the five patients, cells from four were successfully grown in tissue cultures with G418, a neomycin analogue. Those cells that showed neomycin resistance were given intravenously to patients along with systemic IL-2. Peripheral blood was recovered periodically and circulating mononuclear cells with neomycin resistance were found. There were no additional toxicities seen as a result of the genetic alteration. No intact virus was recovered from any patient. Rosenberg concluded that this procedure was both feasible and safe (Rosenberg, et al. 1990).

Prior safety studies have shown that exposure of primates to large infusion of infectious murine amphotrophic virus produces no acute pathologic effects (Cornetta, et al. 1990). Twenty-one primates transplanted with retroviral gene modified bone marrow showed no evidence of toxicity related to the gene transfer as long as five years after infusion (Kantoff, et al. 1987). The first adenosine deaminase-deficient patient with Severe Combined Immunodeficiency (SCID) underwent successful retroviral-mediated adenosine deaminase gene transfer approximately one year ago at the NCI. So far, investigators observed partial reconstitution of cellular immune function without any ill effect on the patient.

To investigate whether the findings from the murine system could be extended, human tumor cell lines were infected with cytokine-containing retroviral vectors. To date, the human IL-2 gene, human IFN-gamma gene and the human epidermal growth factor receptor gene have bene successfully transduced into a variety of human cell lines. In collaboration with Dr. Raymon Taetle (University of Arizona), the epidermal growth factor gene has been introduced into the Burkitts lymphoma cell line, Namalwa; the receptor was shown to be expressed on the cell surface and fully functional. Stimulation of these transduced cells with the epidermal growth factor leads to the appropriate binding of the ligand and signal transduction (Oval, et al. 1991). In addition, the IL-2 or the IFN-gamma gene have been introduced and expressed in primary and established renal cell carcinoma lines. These genetically modified cells secreted constitutively up to 20 U/ml recombinant IL-2 into the supernatant. Constitutive IFN-gamma expression led to the upregulation of RLA class I and class II on the cell surface of renal cell carcinoma cells (Gansbacher, unpublished data). Injection of the IL-2 secreting tumor cells into nude mice led to tumor rejection in contrast to unmodified cells. Irradiation of the genetically altered renal cell carcinomas does not abrogate IL-2 secretion. After lethal irradiation, IL-2 was continuously secreted for 2-3 weeks in amounts comparable to nonirradiated transduced cells.

### Rationale

Studies of whether immunization with allogenic renal carcinoma cells expressing a recombinant human IL-2 gene can be well tolerated without evidence toxicity were proposed. There is substantial experience at MSKCC and other institutions that has established the general safety of immunization using irradiated allogeneic tumor cells (Livingston et al. 1985; Livingston et al. 1983). The choice of allogeneic HLA-A2-positive tumor cells is based on two considerations. First, the use of a single established immunizing cell line bypasses the necessity to infect autologous tumor cells for each patient. Growing autologous tumor cells in tissue culture is a difficult and time intensive procedure. The success rate in growing long term cultures with renal carcinoma cells is 10-20% (Ebert et al. 1990). since approximately 40% of patients are HLA-A2 positive, a tumor vaccine produced from previously established HLA matched renal cell carcinoma cell lines could be widely used. Additionally, evidence is mounting that shared tumor antigens exist (Livingston et al. 1991; Ebert et al. 1990). HLA class I antigens are the restricting element for a cytotoxic T cell response. Allogeneic HLA-A2 matched tumor cells can induce cytotoxic T cell responses. The protocol will be restricted to patients who are HLA-A2-positive.

### Renal Carcinoma Cell Line

Two cell lines, SK-RC-28 and SK-RC-39, will be used in combination for expression of IL-2. The combination will allow presentation of more renal associated antigens and allow greater allogenic stimulation. The cell line SK-RC-28 was derived from the 1979 primary nephrectomy specimen of a 51 year old woman with a solitary humeral metastasis. The parental cell line is moderately well differentiated carcinoma that grows on soft agar but not in nude mice. The cell in SK-RC-39 was derived from the 1980 resection of local recurrence that occurred in a 55 year old woman five years after her primary nephrectomy. The parental line grows on soft agar and in nude mice; it has an in vitro doubling time of 42 hours. The antigenic phenotype of each cell line is shown below.

| Cell line | gp160 | gp140 | gp120 | URO-8 | G250 | HLA-A2 | ABH/Rh |
|---|---|---|---|---|---|---|---|
| SK-RC-28 | + | - | + | - | + | + | A+ |
| SK-RC-39 | - | + | + | + | - | + | O+ |

Both cell lines have been infected with the retroviral vector NAP-AD/IL-2 (DC/AD/R/IL2). The transfected cell lines constitutively secrete IL-2: SK-RC-28 secretes 20 U/ml and SK-RC-39 secretes 40 U/ml. These tumor cells will be irradiated with 10,000 rads prior to injection, to assure their inability to proliferate. Two dose levels of tumor cells will be used for the vaccines.

### Clinical Protocol

This will be a pilot study of immunization with SK-RC-28 and SK-RC-39 allogeneic tumor cells that have been altered to secrete IL-2, given to patients with renal cell carcinoma in order to test the safety of this procedure. Patients will be HLA tissue typed. Those who are HLA-A2 positive will receive vaccinations of allogenic HLA-A2 matched renal carcinoma cells that secrete IL-2. The expected accrual for this study is twelve patients. The expected duration for the trial will be twelve months.

The tissue cultures will be grown in the laboratories of Drs. Bernd Gansbacher and Neil Bander. The tumor cells have been infected with the retroviral vector, NAP-AD/IL2 (DC/AD/R/IL2). Cells that secrete IL-2 will be irradiated (10,000 rads) and given as a series of four vaccinations. If a patient has a clinical response as defined in section Criteria For a Therapeutic Response, then additional boosters will be given up to one year in duration or until progression of disease. Six patients will receive low dose vaccine and the additional six will receive high dose (see Treatment Plan).

### Criteria for Patient Eligibility

Patients must fulfill the following criteria to be eligible for study admission:
1. Patients will histologic confirmation of metastatic renal cell carcinoma at MSKCC.
2. Karnofsky performance status greater than or equal to 70% and life expectancy greater than 4 months.
3. Adequate hematologic function with WBC ≥ 3,000/mm³, absolute lymphocyte count > 1,000/mm³, hemoglobin ≥ 9, and platelets ≥ 100,000/mm³.
4. Adequate renal and hepatic function (serum creatinine ≤ 2.5 mg/dl and bilirubin ≤ 1.7 mg/dl).
5. At least 4 weeks since any prior radiation therapy, immunotherapy, or chemotherapy (6 weeks for nitrosoureas), and full recovery from such treatment.
6. The ability to give written informed consent.
7. Age 2 18 years.
8. No history of other concurrent malignancies except basal carcinoma, squamous cell carcinoma of the skin, or carcinoma-in-situ of the cervix.
9. No systemic steroids for at least one week prior to treatment.
10. Selection of patients with minimal tumor burden is encouraged. Therefore, bulky tumors should be removed if possible prior to entry.
11. Patients must be HLA-A2 positive.
12. Patients must have measurable or evaluable disease.

### Exclusion Criteria

The following conditions/situations will render patients ineligible for this study:
1. Serious intercurrent medical illnesses, to include significant cardiac disease (New York Heart Association Class III or IV), angina pectoris, or myocardial infarction within the previous 6 months.
2. The presence of an active infection, including urinary tract infection.
3. The presence of active CNS metastases.
4. Pregnant or lactating women.
5. Patients who have received more than one form of immunosuppressive therapy to include radiotherapy or chemotherapy.
6. Patients requiring continuing therapy with corticosteroids will not be eligible.

### Concurrent Therapy

The following therapies are prohibited while patients are being treated on this protocol: radiation therapy, hormonal therapy, chemotherapy, corticosteroids (except as administered for life-threatening circumstances), or other immunotherapy.

### Pretreatment Evaluation

1. History and physical examination, including documentation of all measurable disease.
2. Height, weight, and performance status.
3. Screening profile, BUN, and creatinine.
4. CBC, differential, and platelet count.
5. Chest X-ray.
6. EKG
7. Additional tests, x-rays and scans as indicated by the patient's presenting signs and symptoms and required to define the extent of the patient's disease.
8. Peripheral blood will be obtained for immune assays. Six green top tubes and two red top tubes.
9. Biopsy of any clinically asscessible site for confirmation of diagnosis. Please note this is not an absolute requirement for entry into this protocol.

### Treatment Plan

Patients will receive at lease four vaccinations. The first six patients will receive low dose vaccination (defined below). The subsequent six will receive the higher dose level. The vaccine will consist of irradiated, allogeneic RC tumor cells that have bene engineered to secrete IL-2.
- Low Dose:: Approximately 10 million tumor cells
- High Dose:: Approximately 50 million tumor cells

A series of four vaccinations will be given either subcutaneously into the thigh or the arm of the patient. The sites are to be rotated with each vaccination. One vaccine will be given every two weeks for four weeks. A booster vaccine will be given two months following the third. If a patients has a clinical response (CR or PR as defined in Criteria for a Therapeutic Response), additional boosters will be given every three months for one year or until progression of disease. Patients with minor response or stable disease will receive boosters at the discretion of the principal investigators. Blood work will be obtained prior to each vaccination (see section Evaluation During Study).

### Vaccinate: Day 1, 15, 29, 85

If patient develops a severe reaction as defined as grade 4 toxicity (see Criteria for Toxicity) to the vaccine or deteriorating performance status necessitating hospitalization, that patient will not receive subsequent vaccinations.

Criteria for removal from study:
A) Progressive Disease
B) Intercurrent Illness
C) Unacceptable Grade 4+ toxicity
D) General or specific alteration in the patient's condition which would render further treatment as unacceptable in the judgment of the investigators.

### Formulation of Vaccine

The established HLA-A2 matched renal cell carcinoma cell lines SK-RC-28 and SK-RC-39 infected with the retrovirus NAP-AD/IL2 (DC/AD/R/IL2), have been shown to secrete IL-2 in excess of 20 U/ml. The stock IL-2 infected cell lines are frozen in liquid nitrogen and stored in the laboratory of Drs. Bernd Gansbacher and Neil Bander. Approximately one week before the vaccination date, vials containing 10 million tumor cells (one for the low dose and five for the high dose) will be thawed, expanded in tissue culture, and reanalyzed for IL-2 production. These cultures will be shown to be free of helper virus, mycoplasma, bacteria, and fungus.

### Evaluation During Study

1. Physical examination while on therapy prior to each vaccine.
2. CBC, platelet count, and differential will be done prior to each vaccine.
3. Chemical profile including liver function tests, bilirubin, creatinine, and LDH will be done every four weeks for two months, then prior to each vaccine.
4. Peripheral blood will be obtained for immune assays (see section 6.8). Two red top tubes and six green top tubes will be obtained prior to beginning vaccinations and one to two weeks after the fourth vaccine. These will be stored in Dr. Gansbacher's laboratory.
5. Chest X-ray will be repeated every 4 weeks for two months then prior to each vaccine.
6. If moderate to severe toxicity is observed, pertinent tests will be performed more frequently than indicated in the table.

### Evaluation of Immune Response

The most direct method to measure immune response to renal carcinoma is detection of antibodies. The detection IgG antibodies induced by immunization is also a possible indirect measure of helper T cell responses, because induction of high affinity, mature IgG responses requires T cell help by CD-4 positive T cells. All patients will have peripheral blood sera obtained for serologic analysis of antibodies directed against the SK-RC-28 and SK-RC-39 cell lines (to include gp160, g120, gp 140, and Uro-8) and autologous tumor cells whenever available. In addition, a control for positive immune response with antibodies against allogeneic MHC class I and II antigens expressed by SK-RC-28 and SK-RC-39 will be done. It is expected that most patients will develop strong IgG responses against class I and class II alloantigens expressed by the two cell lines. Antibodies against cell surface and intracellular antigen will be detected by:
A. Immunoprecipitation of 1% NP40 lysates of SK-RC-29 and SK-RC-39 metabolically labeled with ³⁵methionine.
B. Mixed hemadsorption assays for IgG and IgM antibodies directed against cell surface antigens (as stated above).

In selected patients where autologous tumor cells are available, MHC-restricted cellular immune responses can be evaluated. Although cellular immune responses are a critical measure of immune response, there are the following limitations: a) autologous tumor cells are necessary to detect responses, b) autologous normal cells and MHC-matched allogeneic cells are necessary to evaluate the specificity of responses, and c) evidence of cytotoxic or helper responses are not evident without in vitro stimulation by autologous tumor cells. Mononuclear cells from peripheral blood will be purified by Ficoll-Hypaque gradient centrifugation. Cells will be cultured with autologous tumor cells, interleukin-2 (20-100 u/ml) and autologous or allogeneic stimulator cells as described for 7-14 days (Wolfel et al., 1989). The following assays to assess cellular immune response will be done when autologous tumor cells are available:
A. Helper T cell Assays. Peripheral blood lymphocytes will be cultured with autologous tumor cells, autologous normal cells and allogeneic tumor cells (e.g. SK-MEL-29) for 48 hours. Proliferation will be measured by uptake of ³H-thymidine in a 4 hour pulse at the end of the assay. Additional measures are secretion of cytokines during co-cultivation with target cells. Interleukin-2 production is measured by enzyme-linked immunoassay (ELISA) and stimulation of the reference CTL cell line. Interferon-γ production is measured by ELISA. Release of additional cytokines can be measured by ELISA, including interleukin-4, interleukin-6, tumor necrosis factor-a, and granulocyte-monocyte colony stimulating factor.
B. Cytotoxic T Cell Assay. Cytotoxicity is measured in either a 4 hour or 18 hour ⁵¹-chromium release assay (as described in Wolfel et al., 1989).

### Criteria For a Therapeutic Response

1. Complete Response (CR): Disappearance of all clinical evidence of tumor on physical examination, x-ray, and biochemical evaluation, for a minimum of 4 weeks.
2. Partial Response (PR): Greater than 50% decrease on physical examination or radiography (including CT scan and/or ultrasound) or the summed products of the perpendicular diameters of all measured lesions maintained for a minimum of 4 weeks. No simultaneous increase in size of any lesion or the appearance of any new lesion may occur.
3. Minor Response (MR): 25-49% decrease in the summed products of diameters of measured lesions for a minimum of 4 weeks.
4. Stable Disease (STAB): Less than 25% decrease or increase in tumor size for at least 3 months.
5. Progression (PROG): Greater than 25% increase in the sum of all measured lesions or appearance of new lesions.
6. Duration of response: Measured from initiation of therapy until a 25% or greater increase from the smallest sum of all tumor measurements obtained during best response (CR, PR, MR, or STAB).

### Criteria for Toxicity

Toxicity will be graded and classified according to Common Toxicity Criteria by the following outline:
- 0: No toxicity
- 1+: Mild toxicity, usually transient, requiring no special treatment and generally not interfering with usual daily activities.
- 2+: Moderate toxicity ameliorated by simple maneuvers.
- 3+: Severe toxicity which requires therapeutic intervention and interrupts usual activities. Hospitalization my or may not be required.
- 4+: Life-threatening toxicity which requires hospitalization. A toxicity which causes a drug-related death will be called a 4F.

### Adverse Experienoe

To date there have been minimal side effects from vaccinations with allogeneic tumor cells (Livingston, 1991). Patients have had induration and erythema at the site of the vaccination. There have been no response of anaphylaxis to allogeneic tumor cells in vivo. In the initial study of giving genetically altered human cells to patients, no additional side effects were seen as a result of the procedure and no viable retrovirus was transmitted (Rosenberg et al., 1990)

Serious adverse effects of treatment (defined as grade III or IV toxicity) will be reported to the Memorial Sloan-Kettering IRB as well as the NIH Office for the Protection from Research Risks within 24 hours and a written report will follow in ten days to the Office of Recombinant DNA Activities, Building 31, Room 4B11, Bethesda, MD.

### Biostatistical Considerations

The endpoint of this pilot study has been defined as clinical toxicity. As indicated in Treatment Plan, six patients will be treated at each dose level. If three patients develop grade III or IV toxicity (as defined by the Common Toxicity Criteria), then a maximum tolerated dose will not be a measurable endpoint.

Design and Sample Size. Approximately twelve patients will be enrolled in this pilot study of tumor cell vaccinations. Two dose levels of tumor cells will be used.
- low dose:: 10 million tumor cells
- High dose:: 50 million tumor cells

The maximum tolerated dose will be defined as the dose of vaccine for which three or more patients have grade IV toxicity. The trial will be stopped at the determination of maximum tolerated dose or the completion of both dose levels. It is anticipated that a maximum tolerated dose may not be established in this study.

### Consent Procedures

All patients will be required to sign a statement of informed consent which must conform to IRG guidelines. This research study has been reviewed by the IRB of Memorial Sloan-Kettering Cancer Center as well as the NIH Recombinant DNA Committee. The investigator will report to the IRB and the RAC changes in the research protocol and all unanticipated problems involving risks to human subjects or others, and no changes will be made in the research activity without IRB approval.

### Fifth series of experiments

### INCREASED EFFECTIVENESS OF THE ALLOGENEIC VACCINE WHEN MORE THAN ONE CYTOKINE IS EXPRESSED

Figure 14 shows retroviral constructs of vectors, N/CIL2/TIFNγ and N/CIFNγ/TIL2, which contains both IL-2 and IFN-γ genes. These constructs were used to make producer cells as described hereinabove. The retroviral vector produced was then used to transduce cells. Figure 15 illustrate the effectiveness of tumor cells which express both IL-2 and IFN-γ. The solid square is a control showing that tumor are formed when 250 thousands of tumor cells, CMS5 were innoculated to Balb/c mice. Solid circle indicates a small tumor was formed when the tumor cells are expressing IL-2, DC/TKIL2/CMS5 and innoculated in four time in amount, that is one million cells. The solid rhombus indicates that the smaller tumor could be formed even if the tumor cells are expressing IFN-γ, SVIFNγ/CMS5 and when four time as much inoculum were used, that is a million cells. When a million was used and the cells are expressing both IL-2 and IFN γ, N/CIL2/TIFNγ/CMS5, no tumor could form. This experiment clearly indicates that the effectiveness of the allogeneic vaccine could be increased by expression of more than one cytokine.

### REFERENCES

Adams, D.O., and Hamilton, T.A. Molecular transduction mechanism by which IFN-gamma and other signals regulate macrophage development. Immunol. Rev., 97: 5-27, 1987.

Alexander, J., Rayman, P., Edinger, M., Connelly, R., Tubbs, R., Bukowski, R., Pontes, E., Finke, J., TIL from renal-cell carcinoma: Restimulation with tumor influences proliferation and cytolytic activity. Int. J. Cancer 45:119, 1990.

Alexander, M.A., Bennicelli, J., and Guerry, D. Defective antigen presentation by human melanoma cell lines cultured from advanced, but not biologically early disease. J. Immunol., 142: 4070-4078, 1989.

Altmann, D.M., Hogg, N., Trowsdale, J., and wilkinson, D. Cotransfection of ICAM-1 and HLA-DR reconstitutes human antigen-presenting cell function in mouse L cells. Nature (Lond.). 338: 512-514, 1989.

Anichini, A., Fossati, G., Parmiani, G., Clonal analysis of cytotoxic T-lymphocyte response to autologous human metastatic melanoma. Int. J. Cancer 35:683, 1985.

Armentano, D., Yu, S.F., Kantoff, P.W., von Ruden, T., Anderson, W.F., and Gilboa, E. Effect of internal viral sequences on the utility of retroviral vectors, J. Virol., 61: 1647-1653, 1987.

Asher, A.L., Mule, J.J., Kasid, A., Restifo, N.P., Salo, J.C., Reichert, C.M., Jaffe, G., Fendly, B., Kriegler, M., and Rosenberg, S.A. Murine tumor cells transduced with the gene for tumor necrosis factor-alpha. Evidence for paracrine immune effects of tumor necrosis factor against tumors. J. Immunol., 146: 3227-3234, 1991.

Asher, A.L., Mule, J.J., Kasid, A., Restifo, N.P., Salo, J.C, Reichert, C.M., Jaffe, G., Fendly, B., Kriegler, M., and Rosenberg, S.A. Murine tumor cells transduced with the gene for tumor necrosis factor-alpha. Evidence for paracrine immune effects of tumor necrosis factor against tumors. J. Immunol., 146: 3227-3234, 1991.

Aulitzky, W., Gastl, G., Aulitzky, W.E., Herold, M., Kemmler, J., Mull, B., Frick, J. and Huber, C. Successful treatment of metastatic renal cell carcinoma a with biologically active dose of recombinant interferon-gamma, J. Clin. Oncol., 7: 1875-1884, 1989.

Baccharini, M., Schwinzer, R., and Lohmann-Matthes, M-L. Effect of human recombinant IL-2 on murine macrophage precursors, Involvement of a receptor distinct from the p55(Tac) protein. J. Immunol., 142: 118-125, 1989.

Bander, N.H., Monoclonal antibodies to renal cancer antigens, Semin. Urol, 7: 264-270, 1989.

Basham, T.Y., Merrigan, T.C., Recombinant gamma interferon increases HLA-DR synthesis and expression. J. Immunol. 130:1494-4, 1983.

Belldegrun, A., Kasid, A., Uppenkamp, M., Rosenberg, S.A., Lymphokine mRNA profile and functional analysis of a human CD4+ clone with unique antitumor specificity isolated from renal cell carcinoma ascitic fluid. Cancer Immunol. Immunotherapy 31:1, 1990.

Belldegrun, A., Kasid, A., Uppenkamp, M., Topalian, S.L., Rosenberg, S.A., Human tumor infiltrating lymphocytes. Analysis of lymphokine mRNA expression and relevance to cancer immunotherapy. J. Immunol. 142:4520, 1989.0

Belldegrun, A., Muul, L.M., Interleukin-2 expanded tumor-infiltrating lymphocytes in human renal cell carcinoma: Isolation, characterization and antitumor activity. Cancer Res. 48:206, 1988.

Belldegrun, A., Muul, L.M., and Rosenberg, S.A. Interleukin-2 expanded tumor-infiltrating lymphocytes in human renal cell carcinoma: isolation, characterization, and antitumor activity. Cancer Res., 48: 206-214, 1988.

Bellet, R.E., Mastrangelo, M.J., Berd, D., et al. Chemotherapy of metastatic malignant melanoma. In: Clark W.H. Jr, Goldman L.L., and Mastrangelo M.J. (eds.). Human malignant melanoma. New York: Grune and Stratton, 1979, p. 235.

Bernd, D., Maguire, H., McCue, P., et al. Treatment of metastatic melanoma with autologous tumor-cell vaccine: clinical and immunologic results in 64 patients. J. Clin. Onc. 8:1858-67, 1990.

Bloom, H.J., Hormone-induced and spontaneous regression of metastatic renal cancer. Cancer 32:1066, 1973.

Borberg, H., Oettgen, H.F., Coudry, D., Beattie, E.J., Jr, Inhibition of established transplants chemically induced sarcomas in syngeneic mice by lymphocytes from ummunized dones. Cancer 10:539, 1972.

Borystewicz, L.K., Graham, S., Hickling, J.K., Mason, P.D., Sissons, J.G.P., Human cytomegalovirus-specific cytotoxic T cells: Their precursor frequency and stage specificity. Eur. J. Immunol. 18:269, 1988.

Boxer, R.J., Waisman, J, Lieber, M.M., Renal carcinoma: computer analysis of 96 patients treated by nephrectomy. J. Urol. 122:598, 1979.

Brodsky, F.M., and Parham, P. Monomorphic anti-HLA-A.B.C. monoclonal antibodies detecting molecular subunits and combinatorial determinants, J. Immunol., 128: 129, 1982.

Cairncross, G.J., Mattes, M.J., Beresford, H.R., Albino, A.P., Houghton, A.N., Lloyd, K.O., and Old, L.J. Cell surface antigens of human astrocytoma defined by mouse monoclonal antibodies identification of astrocytoma subsets. Proc. Natl. Acad. Sci. USA, 79: 5641, 1982.

Carbone, P.P., Costello, W. Eastern Cooperative study groups with DTIC (NSC-45388). Cancer Treat. Rep. 60:193, 1976.

Castle, G., Finstad, C.L., Guanni, A., Bosl, G., Gilboa, E., Bander, N.H. and Gansbacher, B., Retroviral vector-mediated lymphokine gene transfer into human renal cancer cells. Can. Res. 52:6229, 1992.

Catalona, W.J., Ratliff, T.L., and McCool, R.E. Gamma-Interferon induced by S. Aureus protein A augments natural killing and ADCC. Nature (Lond.), 291: 77-79, 1981.

Cheever, M.A., Greenberg, P.D., Fefer, A., Specificity of adoptive chemoimmunotherapy of established syngeneic tumors. J. Immunol. 125:711, 1980.

Chen, L.K., Tourvieille, B., Burns, G.F., Bach, F.H., Matieu-Mahul, D., Sasportes, M., and Bensussan, A. Interferon-gamma: a cytotoxic T lymphocyte differentiation signal. Eur. J. Immunol. 16: 767-770, 1986.

Chen, L., S. Ashe, W.A. Brady, I. Hellstrom, K.E. Hellstrom, J.A. Ledbetter, P. McGowan and P.S. Linsley, Costimulation of Antitumor Immunity by the B7 Counterreceptor for the T Lymphocyte Molecules CD28 and CTLA-4. Vol. 71 1093-1102, 1992.

Collins, T., Korman, A.J., Wake, c.T., Boss, J.M.,, Kappes, D.J., Fiers, W., Ault, K., Bimbrone, M.A., Fr., strominger, J.L., Pober, J.S., Immune interferon activates multiple class II major histocompatibility complex genes and the associated invariant chain gene in human endothelial cells and dermal fibroblasts. Proc. Natl. Acad. Sci. U.S.A., 81:4917, 1984.

Colombo, M.P., Ferrari, G., Stoppachiaro, A., Parenza, M., Rodolfo, M., Mavilio, F., and Parmiani, G. Granulocyte colony-stimulating factor gene transfer suppresses tumorigenicity of a murine adenocarcinoma in vivo, J. Exp. Med., 173: 889-897, 1991.

Comis, R.L. DTIC (NSC-45388) in malignant melanoma: a perspective. Cancer Treat. Rep. 609:165, 1976). Costanza, M.E., Nathanson, L., Schoenfeld, D., et al. Results with methyl-CCNU and DTIC in metastatic melanoma. Cancer 40:1010, 1977.

Cornetta, K., Moen, R.C. Amphotropic murine leukemia retrovirus is not an acute pathogen for primates. Human Gene Therapy 1:13-26, 1990.

Coulie, P.G., Somville, M., Lehmann, F., Hainaut, P., Brasseur, F., Devos, R., and Boon, T. Precursor frequency analysis of human cytolytic T lymphocytes directed against autologous melanoma cells. Int. J. Cancer, 50: 289-297, 1992.

Creagan E.T., Ahmann D.L., Green S.I. et al. Phase II study of recombinant leukocyte A interferon in disseminated malignant melanoma. Cancer 54:2844, 1984).

Crowley, N.J., Slinghuff, C.L., Darrow, T.L., Siegler, H.F. Generation of human autologous tumor specific cytotoxic T cells using HLA-A2 matched allogenic melanoma. Cancer Res. 50:492, 1990.

Crowley, N.J., Darrow, T.L., Quinn-Allen, M.A. et al. MHC-restricted recognition of autologous melanoma by tumor-specific cytotoxic T cells. Evidence for restriction of a dominant HLA-A allele. J. Immunol. 146:1692-9, 1991.

Crowley, N.J., Vervaert, C.E., Seigler, H.F., Human xenograft-nude mouse model of adoptive immunotherapy with human melanoma-specific cytotoxic T-cells. Cancer Res. 52:394, 1992.

Cullen, B.R., Trans-activation of human immunodeficiency virus occurs via bimodal mechanisms, Cell, 46: 973-982, 1986.

Cullen, B.R., Expression of a cloned human interleukin-2 cDNA in enhanced by the substitution of a heterologous mRNA leader region. DNA (NY) 7: 645-650, 1988.

Darrow, T.L., Sligluff, C.L., Seigler, R. et al. The role of HLA class I antigens in recognition of melanoma cells by tumor-specific cytotoxic T lymphocytes: Evidence for shared antigens. J. Immuno. 142:3329-35, 1989.

Deplaen, E., Lurguin, C., Van pel, A. Cloning of the gene of tumor antigen P91A and identification of the tumor mutation. Proc. Natl. Acad. Sci. USA 85:1988, 1988.

De Vries, J.E., Spits, H., Cloned human cytotoxic T lymphocytes (CTL) lines reactive with autologous melanoma cells. J. Immunol. 132:510, 1984.

Ebert, T., Bander, N.H., Finstad, C.L., Ramsawak, R.D., and Old, L.J. Establishment and characterization of human renal cancer and normal kidney cell lines, Cancer res., 50: 5531-5536, 1990.

Erard, F., Corthesy, P., Nabholz, M., Lowenthal, J.W., Zaech, P., Plaetinck, G., and MacDonald, H.R. Interleukin-2 is both necessary and sufficient for th growth and differentiation of lectin-stimulated T lymphocyte precursors. J. Immunol., 134: 1644-1649, 1985.

Fearon, E.R., Pardoll, D.M., Itaya, T., Golumbek, P., Levitsky, H.I. Simons, J.W., Karasuyama, H., Vogelstein, B., and Frost, P., Interleukin-2 production by tumor cells bypasses T helper function in the generation of an antitumor response. Cell. 60: 397-403, 1990.

Feinberg, A.P., Vogelstein, B., A technique for radiolabeling DNA restriction ribonuclease fragments to high specific activity. Anal. Biochem. 132:6, 1983.

Fernandez-Crus, E., Halliburton, B., Feldmann, J.D., In vivo elimination by specific effector cells of an established syngeneic rat moloney virus induced sarcoma. J. Immunol. 123:1772, 1979.

Finstad, C.L., Cordon-Cardo, C., Bander, N.H., Whitmore, W.F., Melamed, M.R., and Old, L.J. Specificity analysis of mouse monoclonal antibodies defining cell surface antigens of human renal cancer. Proc. Natl. Acad. Sci. USA, 82: 2955-2959, 1985.

Forni, G., Cavallo, G.P., Giovarelli, M., Benetton, G., Jemma, C., Barioglio, M.G., De Stafani, A., Forni, M., Santoni, A., Modesti, A., Cavallo, G., Menzio, P., and Cortesina, G. Tumor immunotherapy by local injection of interleukin-2 an non-reactive lymphocytes, Experimental and clinical results. Prog. Exp. Tumor Res. 32: 187-212, 1988.

Gansbacher, B., Zier, K., Cronin, K., Houghton, A., and Giboa, E. Retroviral gene transfer induced constitutive expression of IL-2 or IFN-gamma in irradiated melanoma cells, Blood, in press, 1992.

Gansbacher, B., Bannerji, R., Daniels, B., Zier, K., Cronin, K., and Giboa, E. Retroviral vector-mediated gamma-interferon gene transfer into tumor cells generates potent and long-lasting antitumor immunity, Cancer Res., 50: 7820-7825, 1990a.

Gansbacher, B., Zier, K., Daniels, B., Cronin, K., Bannerji, R., and Gilboa, E. Interleukin-2 gene transfer into tumor cells abrogates tumorigenicity and induces protective immunity, J. Exp. Med., 172: 1217-1224, 1990b.

Gansbacher, B., Rosenthal, F., Guarini, A., Golde, D., Retroviral vectors carrying both the IL-2 and the IFN-gamma gene induce potent antitumor responses in murine tumors. Proc. Am. Assoc. Cancer Res. 33:351, 1992 (abstract).

Gansbacher, B., Levinson, A.I., Effect of PHA-activated T-cells on B-cell differentiation. Immunology 58:191, 1986.

Gansbacher, B., Zier, S., The accumulation of HLA class II mRNA by cloned human T-cell following growth in conditioned medium. Clin. Exp. Immunol. 76:440, 1989.

Glasebrook, A.L., Fitch, F.W. Murine T-cell clones reactivity with alloantigens: An overview. In: Fathman CG. Isolation, Characterization, and Utilization of T Lymphocyte Clones. New York, Academic Press 1982, pp. 265-284.

Graham, S.D. Immunotherapy of renal cell carcinoma, Semin. Urol., 7: 215-227, 1989.

Graham, S.D. Immunotherapy of renal cell carcinoma, Semin. Urol., 7: 215-227, 1989.

Grimm, E.A., Mazumbder, A., Zhang, H.Z., and Rosenberg, S.A. Lymphokine-activated killer cell phenomenon. Lysis of natural killer cell resistant fresh solid tumor cells by interleukin-2 activated cytotoxic T cells. J. Exp. Med., 155: 1823-1841, 1982.

Hainaut, R., Weynants, P., Coulie, P.G., Boon, T. Antitumor T lymphocyte responses. In Oettgen HF. Human Cancer Immunology I. Immunology and Allergy Clinics of North America. 10:639, 1990.

Hantzopoulos, P.A., Sullenger, B.A., Ungers, G., and Giboa, E. Improved gene expression upon transfer of the adenosine deaminase minigene outside the transcriptional unit of a retroviral vector. Proc. Natl. Acad. Sci, USA, 86: 3519-3523, 1989.

Harding, F.A., McArthur, J.G., Gross, J.A., Raulet, D.H., Allison, J.P., CD28-mediated signalling co-stimulates murine T cells and prevents induction of energy in T-cell clones. Nature 356:607, 1992.

Hayashi, H., Tanaka, K., Jay, F., Khoury, G., and Jay, G. Modulation of the tumorigenicity of human adenovirus-12-transformed cells by interferon. Cell, 43:263-267, 1985.

Hayashi, Y., Hoon, D. Cytotoxic T cell (CTL) restricted recognition of common antigens on HLA-A2 matched allogenic human melanomas. Proc. Amer. Assoc. Cancer Res. 32:238, 1991.

Hickling, J.K., Borysiewicz, L.K., Sissons, J.G.F., Varicella-zoster virus specific cytotoxic T-lymphocytes (tc): Detection and frequency analysis of HLA class I restricted Ic in human peripheral blood. J. Virol. 61:3463, 1987.

Hock, H., Dorsch, M., Diamanstein, T., and Blankenstein, T. Interleukin 7 induces CD4+ T cell-dependent tumor rejection, J. Exp. Med., 174: 1291-1298, 1991.

Hom, S.S., Toplian, S.L., Simonis, et al. Common expression of melanoma tumor-associated antigens recognized by human tumor infiltrating lymphocytes.

Analysis by human lymphocyte antigen recognition. J. Immunother. 10:153-164, 1991.

Hoon, D., Hayashi, Y. Specific cytotoxic T cells (CTL's) to human uveal melanoma. Proc. Amer. Assoc. Cancer Res. 32:236, 1991.

Houghton, A.N., Eisinger, M., Albino, A. et al. Surface antigens of melanocytes and melanomas: Markers of melanocyte differentiation and melanoma subsets. J. Exp. Med. 156:1755, 1982.

Houghton, A.N., Thomson, T.M., Gross, D., Oettgen, H.F., Old, L.J., Surface antigens of melanoma and melanocytes. Specificity of induction of 1a antigens by human a-interferon. J. Exp. Med. 160:255, 1984.

Houghton, A.N., Real. F.X., Davis, L.J., Cordon-Cardo, C., Old., L.J., Phenotypic heterogeneity of melanoma. Relation to the differentiation program of melanoma cells. J. Exp. Med. 164:812, 1987.

Hrushesky, W.J., and Murphy, G.P. Current status of the therapy of advanced renal carcinoma. J. Surg. Oncol. 9:277, 1977.

Ikemoto, S., Wada, S., Kamizuru, M., Hayahara, N., Kishimoto, T., Maekawa, M., Clinical studies on cell-mediated immunity in patients wtih renal cell carcinoma: Interleukin-2 and interferon-gamma production of lymphocytes. Cancer Immunol. Immunotherapy 34:289, 1992.

Inge, T.H., Hoover, S.K., Susskind, B.M., Barrett, S.K., Bear, H.D., Inhibition of tumor-specific cytotoxic T-lymphocyte responses by transforming growth factor β.

Cancer Res. 52:1386, 1992.

Javadpour, N. Management of renal cancer. In Cater, Glatstein, and Livingston (eds.), Principles of Cancer Treatment. New York: McGraw Hill Book Company, 1982, p. 571.

Kantoff, P.W., Gillio, A.P., McLachlin, J.R. et al. Expression of human adenosine deaminase in nonhuman primates after retrovirus-mediated gene transfer. J. Exp. Med. 166:219-34, 1987.

Karasuyama, H., and Melchers, F., Establishment of mouse cell lines which constitutively secrete large quantities of interleukin-2, -3, -4, or -5, using modified cDNA expression vectors, Eur. J. Immunol., 18: 97-104, 1988.

Karasuyama, H., and Melchers, F., Establishment of mouse cell lines which constitutively secrete large quantities of interleukin-2, -3, -4, or -5, using modified cDNA expression vectors, Eur. J. Immunol., 18: 97-104, 1988. Knuth, A., Wolfel, T., Meyer, Z. Cellular and humoral immune responses against cancer vaccines. Current Opinion in Immunol. 3:659, 1991.

Kawakami, Y., Zakut, R., Topalian, S.L., Stotter, H., Rosenberg, S.A., Shared human melanoma antigens: Recognition by tumor-infiltrating lymphocytes in HLA-A2.1-transfected melanomas. J. Immunol. 148:638, 1992.

Knuth, A., Danowski, B., Oettgen, H. et al. T-cell mediated cytotoxicity against autologous malignant melanoma: Analysis with interleukin-2 dependent T-cell cultures. Proc. Natl. Acad. Sci. USA 81:3511, 1984.

Knuth, A., Wolfel, T., Klebmann, E., Boon, T., Meyer zum Buschenfelde, K., cytolytic T-cell clones against an autologous human melanoma: Specificity study and definition of three antigens by immunoselection. Proc. Natl. Acad. Sci. U.S.A., 86:2804, 1989.

Koo, A.S., Tso, C., Shimabukuro, T., Peyret, C., de Kernion, J.B., Belldegrun, A., Autologous tumor-specific cytotoxicity of tumor-infiltrating lymphocytes derived from human renal cell carcinoma. J. Immunol. Ther. 10:347, 1991.

Krown, S.E. Interferon treatment of renal cell carcinoma: current status and future prospects. Cancer 59:647, 1987.

Lang, E.K., Sullivan, J., and Apell, R. Staging of renal cell carcinoma by computed tomography and dynamic computed tomography. Proc. Am. Urol. Acco. 1982, Kansas City, Missouri.

Langer, J.A., and Pestka, S. Interferon receptors. Immunol. Today, 9: 393-399, 1988.

Ley, V., Langlade-Demoyen, P., and Larsson-Sciard, L. Interleukin 2-dependent activaton of tumor-specific cytotoxic T lymphocytes in vivo. Eur. J. Immunol., 21: 851-854, 1991.

Liu, Y., Janeway, C.A., Fr., Cells that present both specific ligand and costimulatory activity are the most efficient inducers of clonal expansion of normal CD4 T cells. Proc. Natl. Acad. Sci. U.S.A. 89:3845, 1992.

Livingston, P. Active specific immunotherapy in treatment of patients with cancer. In Immunology and Allergy Clinics of North America. 11:3329-35, May 1991.

Livingston, P.O., Old, L.J., Oettgen, H.F. Approaches to augmenting the immunogenicity of tumor antigens. In Reisfeld R.A., Sell S. Monoclonal Antibodies and Cancer Therapy, UCLA Symposia on Molecular and Cellular Biology. New York, Alan Liss, 27:537, 1985.

Livingston, P.O., Natoli, E.J., Calves, M.J. Vaccines containing purified GM2 ganglioside elicit GM2 antibodies in melanoma patients. Proc. Natl. Acad. Sci. USA 84:2911, 1987.

Livingston, P.O., Kaelin, K., Pinksy, C. et al. Serologic response of patients with Stage II melanoma to allogenic melanoma cell vaccines. Cancer 56:2194-2200, 1985.

Livingston, P.O., Takeyama, A.P., Pollack, M.S. et al. Serologic response of melanoma patients to vaccines derived from allogenic cultured melanoma cells. Int. J. Cancer 31:567-75, 1983.

Livingston, P.O., Albino, A., Chung, T.S.C. et al. Serologic response of melanoma patients to vaccines prepared from VSV lysates of autologous and allogenic cultured melanoma cells. Cancer 55:713-20, 1985.

Livingston, P.O., Watanabe, T., Shiku, H. et al. Serologic response of melanoma patients receiving melanoma vaccines. Autologous cultured melanoma cells. Int. J. Cancer 30:413-22, 1982.

Livingston, P.O., Shiku, H. Cell-mediated cytotoxicity for cultured autologous melanoma cells. Int. J. Cancer 24:34-44, 1979.

Luikart, S.D., Kennealey, G.T., and Kirkwood, J.M. Randomized phase III trial of vinblastine, bleomycin and cisdichlorodiammine-platinum versus dacarbazine in malignant melanoma. J. Clin. Oncol. 2:164, 1984.

Maldazys, J.D., and DeKernion, J.B. Progenostic factors in metastatic renal carcinoma, J. Urol, 136: 376-379, 1986.

Malkovska, V., Sopndel, P.M., and Malkovsky, M. Tumour Immunotherapy, Curr. Opin. Immunol., 1: 883-890, 1989.

Malkovsky, M., Loveland, B., North, M., Asherson, G.L., Gao, L., and Ward, P. Recombinant interleukin-2 directly augments the cytotoxicity of human monocytes. Nature (Lond.), 325: 262-265, 1987.

Markowitz, D., Goff, S., Bank, A., A safe packaging line for gene transfer: separating viral genes on two different plasmids. J. Virology 62:1120, 1988.

Markowitz, D., Goff, S., and Bank, A. Construction and use of a safe and efficient amphotropic packaging cell line. Virology, 167: 400-406, 1988.

Marshall, F.F. and Powell, K.C. Lymphadenectomy for renal cell carcinoma: anatomical and therapeutic consideration. J. Urology. 128:677, 1982.

McKnight, S.L. The nucleotide sequence and transcript map of the herpes simplex thymidine kinase gene. Nucleic Acids Res., 8: 5949-5955, 1980.

McLachin, J.R., Cornetta, K., Eglitis, M.A., and Anderson, W.F. Retroviral mediated gene transfer, Prog. Nucleic Acid Res. Mol. Biol., 38: 91-135, 1990.

Mortarini, R., Belli, F., Parmiani, G., and Anichini, A. Cytokine-mediated modulation of HLA-class II, ICAM-1, LFA-3 and tumor-associated antigen profile of melanoma cells. Comparison with anti-proliferative activity by rIL-1β, rTNF-alpha, rIFN-gamma, rIL-4 and their combinations. Int. J. Cancer, 45: 334-341, 1990.

Mukherji, B., MacAlister, T.J., Clonal analysis of cytotoxic T-cell response against human melanoma. J. Exp. Med. 158:240, 1983.

Myatake, S., Nishibra, K., Kikuchi, H., Yamashita, J., Yuzirou, N., Hanoaka, M., and Watanabe, Y. Efficient tumor suppression by glioma-specific murine cytotoxic T lymphocytes transfected with interferon-gamma gene. J. Natl. Cancer Inst., 82: 217-220, 1990.

Oettgen, H., Old, L.J. The history of cancer immunotherapy. In Devita VT, Hellman S, Rosenberg S. Biological Therapy of Cancer. Philadelphia, Lippincott, 1991, p. 87.

Old, L.J. Cancer Immunotherapy: The search for specificity. Cancer Res. 41:361, 1981.

Oliver, R.T.D., Miller, R.M., Mehta, A. A phase II study of survillence in metastatic renal cancer. J. Urol. Bioth 1:14-20, 1988.

Ostrand-Rosenberg, S., Thakur, A., and Clements, V. Rejection of mouse sarcoma cells after transfection of MHC-class-II genes. J. Immunol. 144: 4068-4071, 1990.

Oval, J., Hershberg, R., Gansbacher, B. et al. Expression of functional epidermal growth factor receptors in human hematopoietic cell lines. Cancer Res. 51:150-6, 1991.

Pandolfini, F., Boyle, L.A., Trentin, L. et al. Expression of HLA-A2 antigen in human melanoma cell lines and its role in T-cell recognition. Cancer Res. 51:3164-70, 1991.

Parkinson, D.R., Fisher, R.I., Rayner, A.A. et al. Therapy of renal cell carcinoma with interleukin-2 and lymphokine-activated killer cells: phase II experience with a hybrid bolus and continuous infusion interleukin-2 regimen. J. Clin. Oncol. 8:1630, 1990.

Porgador, A., Tzeboval, E., Katz, A., Vadai, E., revel. M., Fedman, M., Eisenbach, L., Interleukin-6 gene transfection into Lewis lung carcinoma tumor cells suppresses the malignant phenotype and confers immunotherapeutic competence against parental metastatic cells. Cancer Res. 52:3679, 1992.

Quesada, J.R., Biologic response modifiers in therapy of metastatic renal cell carcinoma. Seminars Onc. 15:396-407, 1988.

Radrizzani, M., GanbacortiPasserini, C., Parmiani, G., Fossati, G., Lysis by interleukin-2 stimulated tumor-infiltrating lymphocytes of autologous and allogeneic tumor target cells. Cancer Immunol. Immunotherapy 28:67, 1989.

Rees, R.C., and Wiltrout, R.H. The biology and clinical applicaitons of interleukin-2, Immunol. Today, 11: 11-13, 1990.

Reilly, E.B., Antognetti, G. Increased tumor-specific CTL activity in human tumor-infiltrating lymphocytes stimulated with autologous tumor lines. Cell Immunol 135:526-33, 1991.

Restifo, N.P., Spiess, P.J., Karp, S.E., Mule, J.J., Rosenberg, S.A., A nonimmunogenic sarcoma transduced with the cDNA for interferon-γ elicits CD8 T cells against the wildtype tumor: Correlation with antigen presentation capability. J. Exp. Med. 175:1423, 1992.

Robson, D.J., Chruchill, B.M., and Anderson, W. The results of radical nephrectomy for renal cell carcinoma. J. Urology 101:297, 1964.

Roehm, N., Marrack, P., Kappler, et al. Helper signals in plaque-forming cell response to protein bound haptens. J. Exp. Med. 158:317-33, 1983.

Rosa., F., Hata, D., Abadie, A., Wallach, D., Revel, M., Fellous, M., Differential regulation of HLA-DR mRNAs and cell surface antigens by interferon. EMBO, 2:1585, 1983.

Rosenberg, S.A. Immunotherapy of patients with advanced cancer using interleukin-2 alone or in combination with lymphokine-activated killer cells. DeVita, V.T., Hellman, S., and Rosenberg, S.A. (eds). Important Advances in Oncology 1988. Philadelphia, PA: JP Lippincott, 1988, p. 217.

Rosenberg, S.A. Special Report: Observations on the systemic administration of autologous lymphokine-activated killer cells and recombinant interleukin-2 in patients with metastatic cancer. NEJM 313:1485-96, 1985.

Rosenberg, S.A., Lorze, M.T., Muul, L.M., Chang, A.E., Avis, F.P., Leitman, S., Linehan, W.M., Robertson, C.N., Lee, R.E., Rubin, J.T. Treatment of 157 patients with advanced cancer using lymphokine-activated killer cells and interleukin-2 or high-dose interleukin-2 alone, N. Engl. J. Med., 316: 889-883, 1987.

Rosenberg, S.A., Lorze, M.T., Muul, L.M., Chang, A.E., Avis, F.P., Leitman, S., Linehan, W.M., Robertson, C.N., Lee, R.E., Rubin, J.T. Treatment of 157 patients with advanced cancer using lymphokine-activated killer cells and interleukin-2 or high-dose interleukin-2 alone, N. Engl. J. Med., 316: 889-883, 1987.

Rosenberg, S.A., Lotze M.T., Yang, J.C. et al. Interleukin-2 gene and alpha interferon for treatment of patients with advanced cancer. J. Clin. Oncol. 7:1863-74, 1989.

Rosenberg, S.A., Aebersald, D., Cornetta, K. et al. Gene transfer into humans - Immunotherapy of patients with advanced melanoma, using tumor-infiltrating lymphocytes modified by retroviral gene transduction. NEJM 323:570-8, 1990.

Rubinstein, M., Novick, D., and Fischer, D.G. The human interferon-gamma geceptor system, Immunol. Rev., 97:28-49, 1987.

Russel, S.J. Lumphokine gene therapy of cancer, Immunol. Today, 11: 196-202, 1990.

Schwartz, R. H. Costimulation of T lymphocytes: The Role of CD28, CTLA-4, and B7/BB1 (CD80) or B70/B7-2 (Clark, E.A., et al. (Feb. 1994) Nature, 367:425-428) in Interleukin-2 Production and Immunotherapy. Cell, 71 1065-1068, 1992.

Shu, S., Rosenberg, S.A., Adoptive immunotherapy of newly induced murine sarcomas. Cancer Res. 45:1657, 1985.

Skinner, D.G., Vermillion, C.D., and Colvin, R.B. The surgical management of renal cell carcinoma. J. Urol. 107:705, 1972.

Smith, E.G., Harmel. R.P., Hann, M.G., Jr., Zwilling, B.S., Zbar, B., Rapp, H.J., Regression of established intradermal tumors and lymph node metastases in guinea pigs after systemic transfer of immune lymphoid cells. J. Natl. Cancer Inst. 58:1315, 1977.

Smith, K.A., Favata, M.F., and Oroszia, S. Production and characterizaton of monoclonal antibodies to human interleukin-2. strategy and tactics, J. Immunol., 131: 1808-1815, 1983.

Tai, T., Cahan, L.D., Tsuchida, T. Immunogenicity of melanoma associated gangliosides in cancer patients. Int. J. Cancer 35:607, 1985.

Tepper, R.I., Pattengale, P.K., and Leder,P. Murine interleukin-4 displays potent anti-tumor activity in vivo, Cell, 57: 503-512, 1989.

Timonen, T., Ortaldo, JR., Stadler, B.M. et al. Cultures of purified human natural killer cells; growth in the presence of interleukin-2. Cell Immuno. 72:178-85, 1982.

Tomita, Y., Nishiyama, T., Watanabe, H., Fujiwara, M., and Sato, S. Expression of intercellular adhesion molecule-1 (ICAM-1) on renal cancer: possible significance in host immune responses, Int. J. Cancer. 46: 1001-1006, 1990.

Topalian, S.L., Salomon, D., Avis, F.P., Chang, A.E., Freerksen, D.L., Linehan, W.M., Lotze, M.T., Robertson, C.N., Slepp, C.A., Simon, C.G., Simpson, C.G., and Rosenberg, S.A. Immunotherapy of patients with advanced cancer using tumor-infiltrating lymphocytes and recombinant interleukin-2; a pilot study, J. Clin. Oncol., 6: 839-853, 1988.

Trinchieri, G., and Perussia, B. Immune interferon; a pleiotropic lymphokine with multiple effects, Immunol. Today, 6: 131-136, 1985.

Trinchieri, G. Biology of natural killer cells, Adv. Immunol., 47: 187-232, 1989.

Vadhan-Raj, S., Cordon-Cardo, C., Carswell, E. et al. Phase I trial of a mouse monoclonal antibody against GD3 ganglioside in patients with melanoma: Induction of inflammatory responses at tumor sites. J. Clin. Oncol. 6:1636, 1988).

Van Der Bruggen, P., Traversari, C., Chomez, P., Lurquin, C., De Plaen, E., Van Den Eynde, B., Knuth, A., Boon, T., A gene encoding an antigen recognized by cytolytic T lymphocytes on a human melanoma. Science, 254:1643, 1991.

Van Der Wef-Messing, B. Carcinoma of the kidney. Cancer 32:1055, 1973.

Vanky, F., Stuber, G., Rotstein, S., and Klein, G. Auto-tumor recognition following in vitro induction of MHC antigen expression on solid human tumours: stimulaiton of lymphocytes and generaton of cytotoxicity against the original MHC-antigen-negative tumour cells. Cancer Immunol. Immunother., 28: 17-21, 1988.

Verstovesek, S., Maccubbin, D., Ehrke, M.J., and Mihich, E. Tumoricidal activation of murine resident peritoneal marophages by interleukin-2 and tumor-necrosis factor alpha, Cancer Res., 52: 3880-3885, 1992.

Vidard, L., K.L. Rock, and B. Benacerraf, Heterogeneity in antigen processing by different types of antigen-presenting cells. Effect of cell culture on antigen processing ability. J. Immunol. 149:1905-1911, 1992.

Wadler, S., and Schwartz, E.L. Anti-neoplastic activity of the combination of interferon and cytotoxic agents against experimental and human malignancies: a review, Cancer Res., 50: 3473-3486, 1990.

Wallach, D., Fellous, M., an Revel, M. Preferential effect of interferon-gamma on the synthesis of HLA-antigens and their mRNAs in human cells. Nature (Lond.), 299: 833-836, 1982.

Wano, Y., Cullen, B.R., Svetlik, P.A., Pfeffer, N.J., and Greene, W.C. Reconstitution of high-affinity IL-2 receptor expression in a human T-cell line using a retroviral cDNA expression vector. Mol. Biol. Med., 4: 95-101, 1987.

Watanabe, Y., Kuribayashi, K., Miyatake, S. et al. Exogenous expression of mouse interferon gamma cDNA in mouse neuroblastoma C1300 cells: results in reduced tumorigenicity by augmented anti-tumor immunity. Proc. Natl. Acad. Sci. USA 86:9456, 1989.

Watanabe, Y., Sakata, T., Highly efficient action of autocrine mouse interferon-gamma expressed via a retroviral vector. Eur. J. Immunol. 18:1627, 1988.

Wingington, D.A., Adrian, G.S., Friedman, R.L., Suttle, D.P., and Hutton, J.J. Cloning of cDNA sequences of human adenosine deaminase, Proc. Natl. Acad. Sci. USA, 80: 7481-7485, 1983.

Wolfel, T., Klehmann, E., Muller, C. Lysis of human melanoma cells by autologous cytolytic T cell clones. Identification of human histocompatibility leukocyte antigen A2 as a major restricting element for those different antigens. J. Exp. Med. 170:797, 1989.

Yagoda, A., Bosl, G. and Scher, H. Advances in chemotherapy. In Javadpour N (ed.) Recent Advances in Urologic Cancer. Baltimore, MD: The williams and Wilkins Company, 1982.

Zier, K., Functional and antigenic properties of cultured T-cells in the cell mediated lympholysis (CML) assay. Human Immunol. 4:147, 1982.

### Sixth Series of Experiments

Therapeutic models using gene transfer into tumor cells have pursued three objectives: (1) to induce rejection of the tumor transduced with therapeutic genes, (2) to induce immune mediated regression of metastatic disease, and (3) to induce long-lasting immunity to protect against challenge with tumor cells, or clinically regrowth of micro-metastatic disease. Since in *vivo* therapy of patients with cancer using gene transfer would as a first step attempt to eliminate the existing tumor, applicant has investigated whether antitumor immunity induced by tumor cells secreting a single cytokine could be increased by cotransfer of a second cytokine gene. To test this approach, CMS-5, a murine fibrosarcoma, was transduced with retroviral vectors carrying interleukin-2 (IL-2), interferon-γ (IFN-γ) or granulocyte-macrophage colony-stimulating-factor (GM-CSF) cDNA alone or IL-2 cDNA in combination with IFN-γ or GM-CSF cDNA. Single cytokine secreting clones were selected to match levels of cytokine production by double cytokine secreting clones so that similar amounts of cytokine are secreted. IFN-γ and IL-2/IFN-γ secreting CMS-5 cells showed increased levels of MHC class I expression compared to IL-2 and GM-CSF secreting or parental CMS-5 cells. IL-2/IFN-γ secreting CMS-5 cells were always rejected by syngeneic mice, whereas the same number of CMS-5 cells secreting only one of these cytokines, or mixtures of single cytokine secreting CMS-5 cells was not rejected. *In vivo* depletion of CD4⁺, CD8⁺ or NK effector cell subpopulations showed that CD8⁺ cytotoxic T cells were primarily responsible for rejection of IL-2/IFN-γ transduced tumor cells. Applicant's data shows the successful use of a single retroviral vector to stably transduce two cytokine genes into the same tumor cell, leading to an increased effect on the *in vivo* induction of antitumor immunity.

### Introduction

Improvements in the ability to target the delivery and expression of transgenes *in vivo* raise the possibility of exploring gene therapy for the treatment of human malignancies (1-6). Among several possible approaches to gene therapy of cancer, the introduction of cytokine genes into tumor cells as part of a vaccine strategy has shown efficacy in experimental models (7-24). Results vary, however, depending on the cytokine gene and tumor system chosen. The expression of certain cytokine genes by tumor cells has been shown to increase tumor immunogenicity and led to primary rejection of transfected tumor cells by syngeneic hosts as well as to the induction of a systemic cellular antitumor immune response (7-12, 15-17, 21, 23). Cytokines such as GM-CSF do not show an effect on tumor growth *in vivo* but can lead to potent systemic immunity upon challenge with parental tumor cells when the vaccine is applied as irradiated cytokine secreting tumor cells (24, 25). Tumor cells expressing these cytokines might prove to be important in adjuvant tumor therapy, however, appear unlikely to benefit patients with a primary tumor and metastatic disease. While in some tumor systems it has been possible to demonstrate rejection of preestablished disease (16, 17, 26-28), in most animal models this has not been possible. In initial steps toward in *vivo* gene therapy for cancer, it is necessary to develop strategies to optimize the stimulation of primary antitumor immunity by cytokine gene transduced tumor cells. In the present study applicant investigated the antitumor immune responses induced by single cytokine secreting tumor cells and the effect of having the tumor cell provide two cytokine stimulatory signals. Applicant and others have shown that transfection and expression of IFN-γ cDNA can lead to increased expression of cell surface molecules, including MHC molecules and tumor associates antigens (TAA), and render non-immunogenic tumors susceptible to destruction of cytotoxic T cells (9-14). Local secretion of IL-2 from IL-2 cDNA transfected tumor cells can activate and expand specific cytotoxic effector cells leading to tumor rejection (7, 8, 29). The synergistic interaction of cytokines has been shown in several biological systems. By transducing a tumor cell with the cDNA's for both IL-2 and IFN-γ applicant has attempted to induce increased antitumor immunity by activating the afferent as well as the efferent arm of the immune response: IFN-γ should act by enhancing the immunogenicity of the target cell and by activating nonspecific cytotoxic effector cells, while IL-2 should provide the costimulatory signal necessary to activate and expand cytotoxic T cells, presumably present in close proximity to the transduced tumor cells (30-34). Since mixing two tumor cell populations, each secreting one cytokine, could lead to preferential elimination of one of the populations (22) and thus to the premature cessation of the effects of one cytokine, applicant has decided to express both cytokines by the same tumor cell to increase cellular antitumor response *in vivo*. Because a single transduction step providing signals for optimum primary rejection would be desirable for *in vivo* use, applicant has developed double cytokine retroviral vectors carrying both, IL-2 and IFH-γ genes or IL-2 and GM-CSP genes and as controls single cytokine retroviral vectors carrying IL-2, IFN-γ or GM-CSF genes. These vectors were used to transduce the murine fibrosarcoma cell line CMS-5. The ability of single cytokine secreting CMS-5 clones to induce primary rejection of tumor cells was compared to tumor cells secreting similar amounts of two cytokines. The immunologically mechanisms involved in the rejection of cytokine secreting tumor cells and the potential to induce long-lasting systemic antitumor immunity were also investigated.

### Materials and Methods

### Retroviral Vector Design and Conversion of Vectors into Viruses.

The retroviral vector N2 is derived from the genome of Moloney murine leukemia virus (MLV) and contains the bacterial neomycin resistance (neo) gene as a selectable marker (35). The sources and restriction enzymes used to obtain DNA fragments encoding the human IL-2 cDNA, the mouse IFN-γ cDNA, the Herpes simplex virus thymidine kinase (TK) promoter, the major immediate early human CMV promoter, the adenosine deaminase (ADA) promoter and the poly-A signal, have been described previously (7, 9, 36). The murine GM-CSF cDNA was a gift of Genetics Institute, Inc. CMV, ADA and TK promoter encoding DNA fragments were fused to the human IL-2 cDNA, murine GM-CSF cDNA or murine IFN-γ cDNA fragment and cloned into different sites of the N2 vector. The TK-IL2 or TK-IFNγ fusion products were cloned into the SnaBI site present in the polylinker in the 3' long terminal repeat (LTR) of a modified N2 vector to generate vector constructs DC/TKIL2, N/CIFNγ/TIL2 or N/CIL2/TIFNγ. To complete cloning of the vector constructs N/CIL2/TIFNγ and N/CIFNγ/TIL2, the CMV-IL2 or CMV-IFNγ fusion products respectively were cloned into a XhoI site present downstream to the neomycin resistance gene. To generate the retroviral vectors DC/AD/R/IFNγ, DC/AD/R/GM-CSF and N/CGM-CSF/RIL2, the ADA promoter was cloned in reverse orientation into the Klenowed MluI site, the murine IFN-γ, human IL-2 or murine GM-CSF cDNA in reverse orientation into the SnaBI site and the poly-A signal into the Klenowed ApaI site of the 3' LTR polylinker. To complete cloning of N/CGM-CSF/RIL2 and CMV-GM-CSF fusion product was cloned into the XhoI site downstream the neomycin gene coding sequences of DC/AD/R/IL2 (36). N2/CMV-GM-CSF was generated by cloning the CMV-GM-CSF fusion product into the same XhoI site in the N2 vector. DCA is a previously described vector in which the human ADA minigene was cloned into the 3' LTR of N2 (37). Retroviral vector constructs were converted to the corresponding virus by electroporating vector DNA into a helper free, ecotropic (GP+E-86: DC/AD/R/IFNγ, DC/AD/R/GM-CSF, N2/CMVGM-CSF, N/CIL2/TIFNγ, N/CIFNγ/TIL2, N/CGM-CSF/RIL2) or amphotropic packaging cell line (GP+*env*AM12: DC/TKIL2, DCA) (38, 39). Colonies were isolated by G418 selection (0.7 mg/ml of Genticin; Gibco® Laboratories, Grand Island, NY), expanded to producer cell lines and cell free supernatant was tested for viral titer.

Tumor Cell Lines and Infection of Tumor Cells. CMS-5 and CMS-13 are methylcholanthrene-induced, non-metastatic fibrosarcoma cell lines of BALB/c origin (40, 41). Both cell lines reproducibly form tumors in syngeneic as well as in immunodeficient nude mice after intradermal (i.d.) injection. The minimum lethal dose in BALB/c mice is 2 x 10⁵ cells (7). Tumor cells were cultured in DMEM medium supplemented with 10% fetal calf serum, 100 units/ml of penicillin, 100 µg/ml of streptomycin and 2 mM L-glutamine. Virus producer cell lines secreting high titer of virus were used to infect CMS-5 cells. Clonal derivatives of CMS-5 cells were isolated by G418 selection, expanded to cell lines and used for further analysis. The *in vitro* growth rate of parental and transfected CMS-5 cells was evaluated by plating 0.25 x 10⁵ cells/ml, in triplicate for each day, and counting viable cells 2, 3, 4, and 5 days after seeding.

Cytokine Assays. Secretion of IL-2, GM-CSF and IFN-γ into supernatants by retrovirally infected tumor cells was determined using appropriate bioassays and confirmed by ELISA (Genzyme, Boston, MA; Endogen, Cambridge, MA). Supernatant from semiconfluent parental or cytokine-secreting CMS-5 cells was collected after 24 hours and assayed for human IL-2, murine GM-CSF and murine IFN-γ. IL-2 biological activity was measured by testing the ability of IL-2 containing preparations to induce ³H-thymidine incorporation into DNA of IL-2 dependent human primary lymphoblasts (42). concentration of murine GM-CSF was similarly determined in a bioassay using GM-CSF dependent murine 32DC13 cells. After overnight incubation without GM-CSF, 10⁴ cells were incubated with serial dilutions of test samples and cultured for 6 hrs. at 37°, 6% CO₂. After pulsing cultures with 1 µCi of ³H-thymidine, incubation was continued for another 15 hrs. at 37°C and cells counted by liquid scintillation. GM-CSF activity was expressed as cpm and calculated in ng/24h/10⁶ cells by comparison with a standard curve of recombinant murine GM-CSF. The mouse IFN-γ bioassay was based on the antiviral activity of supernatants as determined by the reduction of cytopathic effects of vesicular stomatitis virus on L-cells.

Fluorescence Activated Cell Analysis. Flow cytometry was performed from quantitative analysis of surface MHC class I and II expression. One million cells were harvested from tissue culture plates by treatment with 5 mM EDTA, washed with FACS buffer (PBS with 2% FCS and 0.02% Sodium azide), incubated for 30 min. at 4°C with culture supernatants of rat hybridoma cell lines M1/42.3.9.8.HLK (ATCC TIB 126) and B21-2 (ATCC TIB 229). These cell lines, obtained from American Type Culture Collection (Rockville, MD), produce monoclonal antibodies reactive with a monomeric determinant on the MHC class I molecule and a polymorphic determinant on the MHC class II molecules of I-A^{b,d} haplotypes, respectively. After washing twice, cells were incubated with a 1:50 dilution of fluorescein isothiocyanate-conjugated goat anti-rat IgG (Cappel Laboratories, Durham, NC) for 30 min. at 4°C in the dark. The cells were washed twice and then analyzed. Fluorescence originating from dead cells was excluded by propidium iodide staining and threshold gating of forward and 90° lighter scatter signals. Results were confirmed by fluorescence microscopy.

**Tumor Growth *in vivo*.** BALB/c mice were obtained from a colony bred at Sloan Kettering Institute. Tumor cell injections were carried out using freshly prepared tumor cells that were removed from culture plates by trypsinization and washed twice in PBS. Cells were injected i.d. in the back of 7-10 weeks old female animals. Tumor growth was measured in mm using a caliper and recorded as mean diameter (longest surface length plus width divided by two).

***In vivo* Depletion of Effector Cell Subpopulationa.** The rat anti-mouse MAb GK1.5 (IgG2b) and the rat anti-mouse MAb 2.43 (IgG2b) in ascites fluid were used to deplete for CD4⁺ helper cells and CD8⁺ cytotoxic cells, respectively (43, 44). The amount necessary for T cell depletion was determined by weekly i.p. injections of different dilutions of ascites into BALB/c mice. Splenocytes were analyzed 1 week after injection by flow cytometry. For *in vivo* studies weekly i.p. injections of 0.5 ml. of 1:50 (2.43) or 1:30 (GK 1.5) diluted ascites were given, starting 1 week before tumor cell injection. For NK cell depletion 30 µl of rabbit anti-asialo GM1 serum (Wako Chemicals USA, Richmond, VG) was injected i.p. every 5 days, starting 5 days before tumor cell injection. NK cell depletion were confirmed by *in vitro* analysis of spleen cell cytotoxicity against the NK target cell line YAC-1.

### Experimental Results

**Infection of Tumor Cells and Secretion of Cytokines** The structures of the retroviral vectors used to transduce the CMS-5 murine fibrosarcoma cells are shown in Figure 16. Virus-containing cell-free supernatants of infected packaging cell clones were used to infect NIH 3T3 fibroblasts to determine virus titer. Virus titers of different constructs varied and were correlated inversely with the complexity of the retroviral construct used (Table 7). Viral titers for double cytokine constructs ranged from 10³-10⁵ Neo CFU/ml, those of single cytokine constructs from 10⁵-10⁶ Neo CFU/ml. High titer clones were selected to infect CMS-5 fibrosarcoma cells. G418-resistant CMS-5 clones were screened for expression of transfected cytokine genes by measuring IL-2, GM-CSF and IFN-γ release into the culture supernatant using a bioassay. The results were then confirmed by ELISA. CMS-5 parental cells did not secrete any of the cytokines tested in this study. Clonal isolates of cytokine-gene transduced CMS-5 cells varied in their ability to express and secrete cytokines depending on the retroviral vector used. The range of cytokines produced by different clones were between 0-170 ng/24hrs/10⁶ cells for IL-2, 0-1600 pg/24hrs/10⁶ cells for IFN-γ and 0-400 ng/24hrs/10⁶ cells for GM-CSF. Single cytokine secreting clones were selected which after inoculation of one million cells showed delayed tumor growth, but not tumor rejection. IL-2 or IFN-γ transduced CMS-5 clones DC/TKIL2/CMS5 # 6 and DC/AD/R/IFNγ/CMS5 #13 secreted IL-2 at 53 ng/24h/10⁶ cells (160 U/24h/10⁶ cells) and IFN-γ at 268 pg/24h/10⁶ cells (2 U/24h/10⁶ cells), respectively. To investigate whether secretion of a second cytokine by the same cell would induce tumor rejection applicant selected tumor clone N/CIL2/TIFNγ/CMS5 #3 producing 58 ng/24h/10⁶ cells (174 U/24h/10⁶ cells) of IL-2 and 159 pg/24h/10⁶ cells (0.72 U/24h/10⁶ cells) of IFN-γ (Table 8). To analyze *in vivo* effects of GM-CSF on primary tumor rejection, CMS-5 bulk-transduced cells that secreted high (130 ng/24h/10⁶ cells) medium (80 ng/24h/10⁶ cells) or low (8 ng/24h/10⁶ cells) amounts of GM-CSF and clone N2/CMV-GM-CSF/CMS5 # 6.1 secreting 65 ng/24h/10⁶ cells were selected. Tumor cell clone N/CGM-CSF/RIL2 # 14.14 secreting 130 ng/24h/10⁶ cells of GM-CSF and 3.3 ng/24h/10⁶ cells (10 U/24h/10⁶ cells) of IL-2 was chosen as a control (Tab. 2). Secretion of IL-2, IFN-γ or GM-CSF had no discernible effect on cell morphology. No change in growth rate *in vitro* of GM-CSF or IL-2/GM-CSF secreting CMS-5 cells was observed as compared to parental CMS-5 cells. Growth rate in culture was slightly reduced by the secretion of IFN-γ (Figure 17).

**Table 7**

| **VIRAL TITERS FOR THE CONSTRUCTS USED IN THE EXPERIMENTS** | |
|---|---|
| **Construct** | **Titer (Neo CFU/ml)** |
| DC/TKIL2 | 1 x 10⁶ |
| DC/AD/R/IFNγ | 1 x 10⁵ |
| DC/AD/R/GM-CSF | 5 x 10⁵ |
| N2/CMVGM-CSF | 1 x 10⁶ |
| DCA | 1 x 10⁶ |
| N/CIL2/TIFNγ | 5 x 10³ |
| N/CIFNγ/TIL2 | 1 x 10⁴ |
| N/CGM-CSF/RIL2 | 1 x 10⁵ |

**Table 8**

| **CYTOKINE SECRETION PROM VECTOR-TRANSDUCED DMS-5 CLONES USED IN EXPERIMENTS** | | | |
|---|---|---|---|
| **Cells** | **IL2 (ng/24h 10**^{**6**} **cells)** | **IFNγ pg/24h 10**^{**6**} **cells)** | **GM-CSF (ng/ 24h/10**^{**6**} **cells** |
| CMS5 | 0 | 0 | 0 |
| DC/AD/R/IFNγ/ CMS5 #13 | 0 | 268 | 0 |
| DC/TKIL2/CMS5 #6 | 53 | 0 | 0 |
| N/CIL2/TINFγ/CMS5 #3 | 58 | 159 | 0 |
| N2/CMVGM-CSF/ CMS5 #6.1 | 0 | 0 | 65 |
| N/CGM-CSF/RIL2/ CMS5 #14.14 | 3.3 | 0 | 130 |

### MHC Expression of IFN-γ Transduced Tumor Cells.

Upregulation of MHC molecules and other molecules involved in antigen processing and presentation of IFN-γ has been shown and is believed to contribute to the observed antitumor effects of IFN-γ (12, 13, 45-49). To determine whether the constitutive expression of IFN-γ would lead to effects on MHC class I and II expression, IL-2, IFN-γ, GM-CSF and IL-2/IFN-γ transduced CMS-5 cells were assessed by indirect immunofluorescent staining and fluorescence activated cell analysis (Figure 18). Parental CMS-5 cells expressed MHC class I molecules, but no detectable MHC class II molecules. The level of MHC I expression was substantially elevated in cells secreting IFN-γ alone and modestly elevated in those secreting IFN-γ plus IL-2, compared to non-transduced fibrosarcoma cells. No *de novo* expression of MHC class II molecules in IFN-γ transfected cells was seen. As expected, no effects of IL-2 or GM-CSF secretion were observed on MHC expression (data not shown, 9).

**Augmentation of a Specific Antitumor Response by IL-2 and IFN-γ** To study the *in vivo* effects of IL-2, IFN-γ and GM-CSF secretion on primary tumor rejection, cytokine-secreting or parental CMS-5 cells were injected i.d. into the back of BALB/c mice and tumor growth was measured. Applicant previously showed that injection of 2 x 10⁵ unmodified CMS-5 cells into BALB/c mice results in tumor growth in 100% of mice (7). Applicant therefore used 2.5 x 10⁵ parental CMS-5 cells in CMS-5 control groups. Previous work showed that secretion of IL-2 or IFN-γ alone by CMS-5 cells lead to primary tumor rejection (7, 9). Applicant has now chosen single cytokine secreting CMS-5 clones that did not lead to rejection when one million cells were injected. However, one million CMS-5 cells secreting comparable amounts of both, IL-2 plus IFN-γ, were always rejected within 3-4 weeks. One representative experiment is shown in Figure 19. Co-injection of 10⁶ or 5 x 10⁵ IL-2 secreting CMS-5 cells with the same number of IFN-γ secreting CMS-5 cells did also not result in tumor rejection (Figure 20). Thus, the co-secretion of IL-2 and IFN-γ from the same tumor cells seem to augment the primary host antitumor responses compared to single cytokine secretion.

As GM-CSF has proven a potent inducer of immunity against a challenge of parental tumor cells, applicant has investigated the direct *in vivo* effects of GM-CSF secreting fibrosarcoma cells. CMS-5 bulk-transduced cells secreting high, medium or low amounts of GM-CSF did not regress at a dose of 2.5 x 10⁵ cells (Figure 21). Moreover, the results suggest that GM-CSF secretion of CMS-5 fibrosarcoma cells might even have promoted tumor growth. To demonstrate continuing cytokine secretion *in vivo*, GM-CSF secreting tumors were removed after 3-4 weeks, reestablished in culture and production of cytokine measured before and after 10 days culture with G418. GM-CSF secretion before injection corresponded in all cases to the amount secreted after resection of the tumor and after G418 selection (data not shown). These data suggest that GM-CSF production was responsible for the *in vivo* observed effects. Ongoing *in vivo* experiments using CMS-5 cells transduced with vectors carrying the IL-2 cDNA alone or GM-CSF plus IL-2 cDNAs show that the secretion of GM-CSF in addition to IL-2 has no further effect on tumor growth inhibition compared to IL-2 alone (data not shown).

To investigate whether the antitumor activity induced in N/CIL2/TIFNγCMS5 injected mice was a systemic, long lasting tumor-specific response, mice that previously had rejected cytokine-secreting CMS-5 cells were challenged with a lethal dose of parental tumor dells or with non-crossreacting methylcholantrene-induced tumors of the same genetic background (CMS-13). Tumors grew in mice challenged with CMS-13, but not with CMS-5 cells, confirming previous data (data not shown, 7, 9). CMS-5 cells that secreted IL-2 and IFN-γ thus induced both immediate antitumor responses as well as a lasting protective immunity against the parental tumor.

**Influence of *in vivo* Depletion of Effector Cell Subpopulations on Tumor Growth.** To determine the contribution of different effector cell subpopulations to the rejection of IL-2/IFN-γ secreting tumor cells, tumor growth was monitored in mice that were selectively depleted of CD4⁺, CD8⁺ or NK cells. One representative experiment is shown in Figure 7. Mice were injected with MAbs to CD4⁺, CD8⁺ or NK cells as described in Materials and Methods. The absence of CD8⁺ T cells prevented the rejection of IL-2/IFN-γ secreting CMS-5 cells. In contrast, depletion of CD4⁺ cells did not interfere with the ability of mice to reject transduced tumor cells. NK cell depletion retarded rejection of IL-2/IFN-γ secreting tumors compared to nondepleted mice. These results suggested that in the primary rejection of IL-2/IFN-γ secreting tumor cells CD8⁺ cytotoxic T cells were mainly responsible but that NK cells also were induced and that, while they were not necessary for elimination of the tumor, they may also influence tumor rejection (29). CD4⁺ cells did not seem to play a role in the antitumor activity mediated by IL-2/IFN-γ secreting CMS-5 cells.

### Experimental Discussion

In this work applicant investigates the mechanisms of primary rejection of tumor cells transduced with cytokine genes presented in single or double cDNA containing retroviral constructs. Although currently it is not possible to target therapeutic genes to tumor cells *in situ*, progress in the development of tissue-specific delivery and expression systems make this a realistic approach in the future (1-6). Since *in vivo* treatment of patients with cancer using gene transfer would try to achieve maximum induction of antitumor immunity by a single transduction step, applicant has investigated whether the co-transduction of two cytokines into one tumor cell by a single retroviral vector would lead to an increased host antitumor response.

Applicant chose IFN-γ and IL-2 in this study because these cytokines have previously shown efficacy in inducing primary tumor rejection (7-12) and because both act on different levels of the immune response: IFN-γ is a cytokine known to upregulate molecules that participate in processing and presentation of antigen and thus acts on the levels of the target cells (45, 47-52). IFN-γ also regulates induction of cytotoxic T cells and activation of macrophages, NK cells and LAK cells and can induce various other immune regulatory factors (29-33, 53, 54). IL-2, on the other hand, causes proliferation of cytotoxic T cells, NK and LAK cells and thus primarily acts on the level of the effector cells (55-57). Synergy of IL-2 and IFN-γ also might be anticipated as recent data show that activation of T cells can be enhanced, if an antigen-nonspecific costimulatory signal, like B7 or IL-2, is delivered by the APC (26, 27, 58-60). Vaccination with GM-CSF secreting irradiated tumor cells can stimulate a potent long-lasting immunity against a challenge with parental tumor cells (24). Applicant studied the effects of live GM-CSF and GM-CSF/IL-2 secreting tumor cells on induction of primary tumor rejection.

To this end, applicant constructed single cytokine retroviral vectors carrying the murine IFN-γ, murine GM-CSF or human IL-2 cDNA and double cytokine retroviral vector containing both the IL-2 and IFN-γ or IL-2 and GM-CSF cDNA and used those to infect CMS-5, a weakly immunogenic fibrosarcoma. our data demonstrate that retroviral vectors carrying two cytokine genes can be used to stably transfect tumor cells leading to secretion of biologically active proteins over prolonged periods of time. Secretion of low amounts of IFN-γ by fibrosarcoma cells resulted in the upregulation of MHC class I molecules on the cell surface. Immunogenicity thus could be enhanced because tumor associated antigens are presented to cytotoxic T cells in context of MHC class I molecules (61, 62). The secretion of IL-2 and IFN-γ from the same tumor cell led to the generation of cytotoxic cells capable of rejecting at least one million CMS-5 cells, a dose four times higher than the lethal dose of CMS-5 for BALB/c mice. The secretion of similar amounts of IFN-γ or IL-2 alone did not induce an immune response strong enough to cause rejection of one million tumor cells. Mixing the same numbers of IL-2 and IFN-γ secreting CMS-5 cells also did not result in tumor rejection. This phenomenon may be related to the circumstance that with the double cytokine vector both cytokines are secreted at a constant concentration/cell until all tumor cells are destroyed and that in the cell mixture circumstance one cytokine secreting population is preferentially destroyed before the other one. These data support the concept that IL-2 and IFN-γ can cooperatively activate cytotoxic functions of the immune system (46, 63-65) and suggest an advantage to having both cytokines secreted by the same cell. It is possible that *in vivo* IFN-γ induced upregulation of molecules involved in antigen processing and presentation is more efficacious in the autocrine situation and that in the paracrine situation more IFN-γ would be needed. Yet, it has to be considered that on a per cell basis only half as much cytokine is produced in a mixture of clones. The question of the advantage of co-secretion of both cytokines by one tumor cell is not definitely answered with the experiments performed here. Nonetheless secretion of both cytokines by a single cell should lead to simultaneous exposure of all effector cells to both cytokines. In contrast, secretion of both cytokines by different tumor cells would be expected to result in exposure of effector cells to variable concentration of the cytokines. Furthermore, the use of a single vector carrying both cytokine genes would simplify gene transfer for the treatment of tumors *in vivo* requiring only one transduction step.

Unirradiated GM-CSF secreting bulk-transduced CMS-5 cells and clonally selected GM-CSF and GM-CSF/IL-2 secreting CMS-5 cells were not rejected even at lower cell numbers. To the contrary, the more GM-CSF secreted, the faster the tumors grew, suggesting a growth promoting effect of GM-CSF on CMS-5 cells. Tumor explantation and reselection with G418 *in vitro* showed that outgrowth of none or low GM-CSF secreting CMS-5 cells could not be the reason for the observed tumor growth *in vivo.* In additional experiments the GM-CSF containing vector was used to transduce MBT-2, a bladder carcinoma, TS/A, an adenocarcinoma, and B16, a melanoma. In no case did GM-CSF secretion induce rejection of the transduced tumor cells. Applicant's data is in agreement with published data showing that GM-CSF exerts its potent immunostimulatory effects best if secreted by irradiated tumor cells incapable of proliferation. Dranoff et al. reported a systemic syndrome of fatal toxicity manifested by leukocytosis, hepatosplenomegaly, and pulmonary hemorrhage in mice injected with live GM-CSF secreting B16 cells (24). This phenomenon was attributed to the progressively increasing number of tumor cells expressing GM-CSF *in vivo*. Human tumors naturally secreting GM-CSF do not appear to be rejected either (66). These and our data underline the importance to carefully select the cytokine for each tumor and each gene therapy approach chosen.

In the absence of cytotoxic CD8⁺ T cells, IL-2/IFN-γ secreting CMS-5 tumors were not rejected, confirming the necessity of CD8⁺ CTLs for elimination of the tumor. These result suggest that despite the slight growth inhibition of IFN-γ secreting cells *in vitro,* direct inhibitory effects of IFN-γ on tumor growth were not responsible for tumor regression. Although IL-2/IFN-γ secretion by tumor cells activated NK cells, as shown by the delayed rejection of tumors in NK depleted animals, their presence was not necessary to eliminate tumors. CD4⁺ helper T cells did not seem to contribute to the rejection of IL-2/IFN-γ secreting CMS-5 cells, as tumor rejection was not impaired in CD4 depleted mice, probably because constitutive secretion of IL-2 and IFN-γ by target cells bypassed the requirement for T helper cells (8). From the experiments presented here applicant cannot exclude that other non-specific effector cells of the immune system contributed to the rejection of neoplastic cells. IFN-γ is a potent macrophage activator and thus antitumor activity in this system might be partially attributable to cytostatic or cytotoxic effects of activated macrophages. Moreover, IL-2/IFN-γ secretion not only leads to rejection of transduced tumor cells but also stimulates a specific, long-lasting antitumor immunity. This could be clinically important if after primary therapy not all tumor cells were eliminated. Then, immunity might prevent regrowth of micro-metastases. Whether the immune response induced by double cytokine transduced tumor cells might be sufficient to also eliminate pre-established disease or, if not, how this can be achieved, needs further investigation.

In summary, double cytokine gene carrying retroviral vectors can be used to stably transfect tumor cells and augmentation of *in vivo* antitumor activity can be obtained. Also, expression of both cytokines from one vector construct may be of benefit for future clinical applications to deliver both genes at the same time into one tumor cell.

### References of the Sixth Series of Experiments

1. Vile, R.G., Hart I.R., *In vitro* and *in vivo* targeting of gene expression to melanoma cells. Cancer Res., 53:962 (1993).
2. Culver, K.W., Ram, Z., Wallbridge, S., Ishii, H., Oldfield, E.H., Blaese, R.M., In vivo gene transfer with retroviral vector producer cells for treatment of experimental brain tumors. Science, 256:1550 (1992).
3. Nabel, E.G., Plaut, G., Nabel, G.J., Site-specific gene expression in vivo by direct gene transfer into the arterial wall. Science, 249:1285 (1990).
4. Nabel, E.G., Gordon, D., Yang, Z.Y., Xu, L., San, H., Plautz, G.E., Wu, B.Y., Gao, X., Huang, L., Nabel, G.J., Gene transfer in vivo with DNA-liposome complexes: lack of autoimmunity and gonadal localization. Human Gene Therapy, 3:649 (1992).
5. Ferry, N., Duplessis, O., Houssin, D., Danos, O., Heard, J-M., Retroviral-mediated gene transfer into hepatocytes in vivo. Proc. Natl. Acad. Sci. USA, 88:8377 (1991).
6. Roux, P., Jeanteur, P., Piechaczyk, M., A versatile and potentially general approach to the targeting of specific cell types by retroviruses: Application to the infection of human cells by major histocompatibility complex class I and class II antigens by mouse ecotropic murine leukemia virus-derived viruses. Proc. Natl. Acad. Sci. USA 86:9079 (1989).
7. Gansbacher, B., Zier, K., Daniels, B., Cronin, K., Bannerji, R., Gilboa, E., Interleukin 2 gene transfer into tumor cells abrogates tumorigenicity and induces protective immunity. J. Exp. Med., 172:1217 (1990).
8. Fearon, E.R., Pardoll, D.M., Itaya, T., Golumbek, P., Levitsky, H.I., Simons, J.W., Karasuyama, H., Vogelstein, B., Frost, P., Interleukin-2 production by tumor cells bypass T helper function in generation of an antitumor response. Cell, 60:397 (1990).
9. Gansbacher, B., Bannerji, R., Daniels, B., Zier, K., Cronin, K., Gilboa, E., Retroviral vector-mediated γ-interferon gene transfer into tumor cells generates potent and long lasting antitumor immunity. Cancer Res., 50:7820 (1990).
10. Watanabe, Y., Sakata, T., Highly efficient action of autocrine mouse interferon-γ expressed via a retroviral vector. Eur. J. Immunol., 18:1627 (1988).
11. Watanabe, Y., Kuribayashi, K., Miyatake, S., Nishihara, K., Nakayama, E-I., Taniyama, T., Sakata, T-A., Exogenous expression of mouse interferon gamma cDNA in mouse neuroblastoma C1300 cells: results in reduced tumorigenicity by augmented anti-tumor immunity. Proc. Natl. Acad. Sci. USA 86:9456 (1989).
12. Restifo, N.P., Spiess, P.J., Karp, S.E., Mulé, J.J., Rosenberg, S.A., A nonimmunogenic sarcoma transduced with the cDNA for interferon-γ elicits CD8⁺ T cells against wild-type tumor: correlation with antigen presentation capability. J. Exp. Med., 175:1423 (1992).
13. Gastl, G., Finstad, C.L., Guarini, A., Bosl, G., Bander, N., Gilboa, E., Gansbacher, B., Retroviral vector-mediated lymphokine gene transfer into human renal cancer cell lines. Cancer Res., 52:6229 (1992).
14. Ogasawara, M., Rosenberg, S., Enhanced expression of HLA molecules and stimulation of autologous human tumor infiltrating lymphocytes following transduction of melanoma cells with γ-interferon genes. Cancer REs., 53:3561 (1993).
15. Tepper, R.I., Pattengale, P.K., Leder, P., Murine interleukin-4 displays potent anti-tumor activity in vivo. Cell, 57:503 (1989).
16. Golumbek, P.T., Lazenby, A.J., Levitzky, H.I., Jaffé, L.M., Karasuyama, M., Baker, M., Pardoll, D.M., Treatment of established renal cancer engineered to secrete Interleukin-4. Science, 254:713 (1991).
17. Porgador, A., Tzehoval, E., Katz, A., Vadai, E., Revel, M., Feldman, M., Eisenbach, L., Interleukin 6 gene transfection into lewis lung carcinoma tumor cells suppresses the malignant phenotype and confers immunotherapeutic competence against metastatic cells. Cancer Res., 52:3679 (1992).
18. Hock, H., Dorsch, M., Diamantstein, T., Blankenstein, T., Interleukin-7 induces CD4⁺ T cell-dependent tumor rejection. J. Exp. Med., 174:1291 (1991).
19. Blankenstein, T., Qin, Z., Überla, K., Müller, W., Rosen, H., Volk H-D., Diamantstein, T., Tumor suppression after tumor cell-targeted tumor necrosis factor a gene transfer. J. Exp. Med., 173:1047 (1991).
20. Colombo, M.P., Ferrari, G., Stoppacciaro, A., Parenza, M., Rodolfo, M., Mavilio, F., Parmiani, G., Granulocyte colony-stimulating factor gene transfer suppresses tumorigenicity of a murine adenocarcinoma in vivo. J. Exp. Med., 173:889 (1991).
21. Cavallo, F., Giovarelli, M., Gulino, A., Vacca, A., Stoppacciaro, A., Modesti, A., Forni, G., Role of neutrophils and CD4⁺ T lymphocytes in the primary and memory response to nonimmunogenic murine mammary adenocarcinoma made immunogenic by IL-2 gene. J. Immunol., 149:3627 (1992).
22. Ohe, Y., Podack, E.R., Olsen, K.J., Miyahara, Y., Ohira, T., Miura, K., Nishio, K., Saijo, N., Combination effect of vaccination with IL2 and IL4 cDNA transfected cells on the induction of a therapeutic immune response against lewis lung carcinoma cells. Int. J. Cancer, 53:432 (1993).
23. Porgador, A., Gansbacher, B., Bannerji, R., Tzehoval, E., Gilboa, E., Feldman, M., Eisenbach, L., Anti-metastatic vaccination of tumor-bearing mice with IL-2-Gene-inserted tumor cells. Int. J. Cancer, 53:471 (1993).
24. Dranoff, G., Jaffee, E., Lazenby, A., Golumbek, P., Levitsky, H., Brose, K., Jackson, V., Hamada, H., Pardoll, D., Mulligan, R.C., Vaccination with irradiated tumor cells engineered to secrete murine granulocyte-macrophage colony-stimulating factor stimulated potent, specific, and long-lasting anti-tumor immunity. Proc. Natl. Acad. Sci. USA, 90:3539 (1993).
25. Jaffee, E.M., Dranoff, G., Cohen, L.K., Hauda, K.M., Clift, S., Marshall, F.F., Mulligan, R.C., Pardoll, D.M., High efficiency gene transfer into primary human tumor explants without cell selection. Cancer Res., 53:2221 (1993).
26. Chen, L., Ashe, S., Brady, W.A., Hellström, I., Hellström, K.E., Ledbetter, J.A., McGowan, P., Linsley, P.S., Costimulation of antitumor immunity by the B7 counterreceptor for the T lymphocyte molecules CD28 and CTLA-4. Cell, 71:1093 (1992).
27. Townsend, S.E., Allison, J.P., Tumor rejection after direct costimulation of CD8⁺ T cells by B7-transfected melanoma cells. Science, 259:368 (1993).
28. Connor, J., Bannerji, R., Saito, S., Heston, W., Fair, W., Gilboa, E., Regression of bladder tumors in mice treated with interleukin 2 gene-modified tumor cells. J. Exp. Med., 177:1127 (1993).
29. Alosco, T.R., Croy, B.A., Gansbacher, B., Wang, H.Q., Rao, U., Bankert, R.B., Tumor cells secreting IL-2 stimulate a local anti-tumor response independent of functional B or T lymphocytes. Cancer Immunology and Immunotherapy, 36:364 (1993).
30. Herbermann, R.B., Ortaldo, J.E., Mantovani, A., Hobbs, D.F., Kung, H-F., Pestka, S., Effect of human recombinant interferon on cytotoxic activity of natural killer (NK) cells and monocytes. Cell Immunol., 67:160 (1982).
31. Schreiber, R.D, Celada, A., Molecular characterization of γ-interferon as a macrophage activating factor. In: E. Pick (ed.), Lymphokines 11, pp. 87-118, New York, Academic Press, 1985.
32. Vil 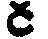 ek, J., Gray, P.W., Rinderknecht, E., Sevastopoulos, C.G., Interferon-γ: A lymphokine for all seasons. In: E. Pick (ed.), Lymphokines 11, pp. 1-32, New York, Academic Press, 1985.
33. Trinchieri, G., Perussia, B., Immune interferon: A pleiotropic lymphokine with multiple effects. Immunol. Today 6:131 (1985).
34. Maraskovsky, E., Chen, W., Shortman, K., IL-2 and IFN-gamma are two necessary lymphokines in the development of cytolytic T cells. J. Immunol., 143:1210 (1989).
35. Armentano, D., Yu, S-F., Kantoff, P.W., von Ruden, T., Anderson, W.F., Gilboa, E., Effect of internal viral sequences on the utility of retroviral vectors. J. Virol., 61:1647 (1987).
36. Gansbacher, B., Zier, K., Cronin, K., Hantzopoulos, P.A., Bouchard, B., Houghton, A., Gilboa, E., Golde, D., Retroviral gene transfer induced constitutive expression of interleukin-2 or interferon-γ in irradiated human melanoma cells. Blood 80:2817 (1992).
37. Hantzopoulos, P.A., Sullenger, B.A., Ungers, G., Gilboa, E., Improved expression upon transfer of the adenosine deaminase minigene outside the transcriptional unit of the retroviral vector. Proc. Natl. Acad. Sci. USA 86:3519 (1989).
38. Markowitz, D., Goff, S., Bank, A., A safe packaging line for gene transfer: Separating viral genes on two different plasmids. J. Virol., 62:1120 (1988).
39. Markowitz, D., Goff, S., Bank, A., Construction and use of a safe and efficient amphotropic packaging cell line. Virology, 167:400 (1988).
40. Srivastava, P.K., DeLeo, A.B., Old, L.J., Tumor rejection antigens of chemically induced sarcomas of inbred mice. Proc. Natl. Acad. Sci. USA, 83:3407 (1986).
41. DeLeo, A.B., Shiku, H., Takahashi, T., John, M., Old, L.J., Cell surface antigens of chemically induced sarcomas of the mouse. I. murine leukemia virus-related antigens and alloantigens on cultured fibroblasts and sarcoma cells: description of a unique antigen on BALB/c Meth A sarcoma. J. Exp. Med., 146:720 (1977).
42. Zier, K., Functional and antigenic properties of cultured T-cells in cell mediated lympholysis (CML) assay. Human Immunol., 4:147 (1982).
43. Dialynas, D.P., Quan, Z.S., Wall, K.A., Pierres, A., Qintanás, J., Loken, M.R., Pierres, M., Fitch, F.W., Characterization of the murine T cell surface molecule, designated L3T4, identified by monoclonal antibody GK1.5: Similarity of L3T4 to the human Leu-3/T4 molecule. J. Immunol. 131:2445 (1983).
44. Sarmiento, M., Glasebrook, A.L., Fitch, F.W., IgG or IgM monoclonal antibodies reactive with different determinants on the molecular complex bearing Lyt 2 antigen block T cell-mediated cytolysis in the absence of complement. J. Immunol. 125:2665 (1980).
45. Basham, T.Y., Merrigan, T.C., Recombinant gamma interferon increases HLA-DR synthesis and expression. J. Immunol. 130:1492 (1983).
46. Weber, J.S., Jay, G., Tanaka, K., Rosenberg, S.A., Immunotherapy of a murine tumor with interleukin-2. Increased sensitivity after MHC class I gene transfection. J. Exp. Med., 166:1716 (1987).
17. Hämmerling, G.J., Klar, D., Pulm, W., Momburg, F., Moldenhauer, G., The influence of major histocompatibility complex class I antigens on tumor growth and metastasis. Biochem. Biophys. Acta., 907:245 (1987).
48. Skoskiewicz, M.J., Colvin, R.B., Schneeberger, E.E., Russel, P.S., Widespread and selective induction of major histocompatibility complex-determined antigens in vivo by γ Interferon. J. Exp. Med. 162:1645 (1985).
49. Carrel, S., Schmidt-Kesse A., Guiffrè, L., Recombinant interferon-gamma can induce the expression of HLA-DR and -DC on DR-negative melanoma cells and enhanced the expression of HLA-ABC and tumor-associated antigens. Europ. J. Immunol. 15:118 (1985).
50. Houghton, A.N., Thomson, T.M., Gross, D., Oettgen, H.F., Old, L.J., Surface antigens of melanoma and melanocytes. Specificity and induction of Ia antigens by human gamma interferon. J. Exp. Med., 160:255 (1984).
51. Lindahl, P., Leary, P., Gresser, I., Enhancement by interferon of the expression of surface antigens on murine leukemia L1210 cells. Proc. Natl. Acad. Sci. (Wash), 70:2785 (1973).
52. Yang, Y., Waters, J.B., Früh, K., Peterson, P.A., Proteasomes are regulated by interferon gamma: implications for antigen processing. Proc. Natl. Acad. Sci. USA 88:4928 (1992).
53. Birmingham, J.R., Chestnut, R.W., Kappler, J.W., Marrack, P., Kubo, P., Grey, H.M., Antigen presentation to T cell hybridomas by a macrophage cell line: an inducible function. J. Immunol. 128:1491 (1982).
54. Baker, J.N., Allen, M.H., MacDonald, D.M., The effect of *in vivo* interferon gamma on the distribution of LFA-2 and ICAM-1 in normal human skin. J. Invest. Dermatol. 93:439 (1989).
55. Farrar, W.L., John, H.M., Farrar, J., Regulation of the production of immune interferon and cytotoxic T-lymphocytes by IL-2. J. Immunol., 126:1120 (1981).
56. Malkovsky, M., Loveland, B., North, M., Asherson, G.L., Gao, L., Ward, P., Fiers, W., Recombination interleukin-2 directly augments the cytotoxicity of human monocytes. Nature, 325:262 (1987).
57. Erard, F., Corthesy, P., Nabholz, M., Lowenthal, J.W., Zaech, P., Plaetinck, G., McDonald, H.R., Interleukin 2 is both necessary and sufficient for the growth and differentiation of lectin-stimulated cytolytic T lymphocyte precursors. J. Immunol. 134:1644 (1985).
58. Liu, Y., Janeway, C.A., Cells that present both specific ligand and costimulatory activity are the most efficient inducers of clonal expansion of normal CD4 T cells. Proc. Natl. Acad. Sci. USA, 89:3845 (1992).
59. Heath, W.R., Allison, J., Hoffman, W.M., Schönrich, G., Hämmerling, G., Arnold, B., Miller, J.F.A.P., Autoimmune diabetes as a consequence of locally produced interleukin-2. Nature, 359:547 (1992).
60. Norton, S.D., Zuckerman, L., Urdahl, K.B., Shefner, R., Miller, J., Jenkins, M.K., The CD28 ligand, B7, enhances IL-2 production by providing a costimulatory signal to T cells. J. Immunol. 149:1556 (1992).
61. Van der Bruggen, P., Traversari, C., Chomez, P., Lurguin, C., de Plaen, E., Van den Eynde, B., Boon, T., A gene encoding an antigen recognized by cytolytic T lymphocytes on a human melanoma. Science, 254:1643 (1991).
62. Crowley, N.J., Darrow, T.L., Quinn-Allen, M.A., MHC-restricted recognition of autologous melanoma by tumor-specific cytotoxic T cells. Evidence for restriction by a dominant HLA-A allele. J. Immunol., 146:1692 (1991).
63. Nakajima, I., Chu, T.M., Enhanced cell-mediated cytotoxicity by interferon-gamma and interleukin-2 against syngeneic murine mammary adenocarcinoma. Mol. Biother., 4:47 (1992).
64. Itoh, K., Shiiba, K., Shimizu, Y., Suzuki, R., Kumagai, K., Generation of activated killer (AK) cells by recombinant interleukin 2 (rIL 2) in collaboration with interferon-γ (IFN-γ). J. Immunol., 134:3124 (1985).
65. Shalaby, M.R., Svedersky, L.P., McKay, P.A., Finkle, B.S., Palladino, M.A., *In vivo* augmentation of natural killer activity by combined treatment with recombinant gamma interferon and interleukin-2. J. Interferon Res., 5:571 (1985).
66. Hocking, W., Goodman, J., and Golde, D.W., Granulocytosis associated with tumor cell production of colony-stimulating activity. Blood, 61:600 (1983).

## Claims

1. A genetically manipulated cell which co-expresses interleukin-2 and interferon-gamma and which cell is allogenic relative to a patient to whom the cell is administered.

2. The genetically manipulated cell of claim 1, which expresses another immunomolecule selected from the group consisting of:
a cytokine, an adhesion molecule, a costimulatory factor, a tumor associated antigen and a tumor specific antigen.

3. The genetically manipulated cell of claim 1, wherein the cell is a nonprofessional antigen presenting cell.

4. The genetically manipulated cell of claim 1, wherein the cell expresses tumor associated antigen and tumor specific antigen.

5. The genetically manipulated cell of claim 1, wherein the cell expresses either a tumor associated antigen or a tumor specific antigen.

6. The genetically manipulated cell of claim 1, wherein the cell is selected from a group consisting of a melanoma cell, a renal carcinoma cell, a breast tumor cell and a brain tumor cell.

7. The genetically manipulated cell of claim 1, wherein the cell comprises a retroviral vector which comprises:
(a) a 3' long terminal repeat (LTR); and
(b) an insert comprising:
(i) a promoter,
(ii) DNA encoding interleukin-2 and interferon-gamma, and
(iii) a poly A signal,
wherein the promoter, the DNA encoding interleukin-2 and interferon-gamma, and the poly A signal are aligned in frame in a 5' to 3' orientation, and
wherein the insert is positioned within the 3' LTR in an orientation opposite to the orientation of the 3' LTR.

8. The genetically manipulated cell of claim 7, wherein the promoter is selected from the group consisting of a promoter of the Herpes simplex virus thymidine kinase gene, a promoter of the major immediate early human CMV gene, and a promoter of the adenosine deaminase gene.

9. The genetically manipulated cell of claim 7, wherein the vector is N2.

10. The genetically manipulated cell of claim 1, wherein the cell is selected from the group consisting essentially of SK-MEL-29, SK-MEL-131, SK-RC-28 and SK-RC-39.

11. A composition comprising a plurality of the genetically manipulated cell of any one of claims 1 to 10.

12. Use of the genetically manipulated cell of any one of claims 1 to 10 for the preparation of a pharmaceutical composition for inhibiting tumor growth in a mammalian subject.

13. The use of claim 12, wherein the cell further expresses the tumor associated antigen, melanoma associated antigen E-1.

14. The use of claim 12, wherein the genetically manipulated call is a dendritic cell.

15. The use of claim 12, wherein from about ten to about fifty million genetically manipulated cells are to be administered to the mammalian subject.

## Patentansprüche

1. Genetisch manipulierte Zelle, die Interleukin-2 und Interferon-Gamma koexprimiert, wobei die Zelle in Bezug auf einen Patienten, dem die Zelle verabreicht wird, allogen ist.

2. Genetisch manipulierte Zelle nach Anspruch 1, die ein anderes Immunmolekül ausgewählt aus der Gruppe bestehend aus einem Cytokin, einem Adhäsionsmolekül, einem kostimulierenden Faktor, einem tumorassoziiertem Antigen und einem tumorspezifischen Antigen, exprimiert.

3. Genetisch manipulierte Zelle nach Anspruch 1, wobei die Zelle eine nichtprofessionelle antigenpräsentierende Zelle ist.

4. Genetisch manipulierte Zelle nach Anspruch 1, wobei die Zelle ein tumorassoziiertes Antigen und ein tumorspezifisches Antigen exprimiert.

5. Genetisch manipulierte Zelle nach Anspruch 1, wobei die Zelle entweder ein tumorassoziiertes Antigen oder ein tumorspezifisches Antigen exprimiert.

6. Genetisch manipulierte Zelle nach Anspruch 1, wobei die Zelle ausgewählt ist aus einer Gruppe bestehend aus einer Melanomzelle, einer Nierenkrebszelle, einer Brusttumorzelle und einer Gehirntumorzelle.

7. Genetisch manipulierte Zelle nach Anspruch 1, wobei die Zelle einen retroviralen Vektor umfasst, der die folgenden Bestandteile umfasst:
(a) ein langes 3'-terminales Repeat (LTR); und
(b) ein Insert, umfassend:
(i) einen Promotor,
(ii) DNA, die Interleukin-2 und Interferon-Gamma codiert, und
(iii) ein Poly-A-Signal,
wobei der Promotor, die Interleukin-2 und Interferon-Gamma codierende DNA und das Poly-A-Signal im Leserahmen in einer 5'- nach 3'-Orientierung angeordnet sind, und
wobei das Insert innerhalb des 3'-LTR in einer Orientierung gelegen ist, die entgegen der Orientierung des 3'-LTRs ist.

8. Genetisch manipulierte Zelle nach Anspruch 7, wobei der Promotor ausgewählt ist aus der Gruppe bestehend aus einem Promotor des Herpex simplex-Virus-Thymidin-Kinase-Gens, einem Promotor des menschlichen unmittelbaren frühen Haupt ("major immediate early human")-CMV-Gens und einem Promotor des Adenosin-Desaminase-Gens.

9. Genetisch manipulierte Zelle nach Anspruch 7, wobei der Vektor N2 ist.

10. Genetisch manipulierte Zelle nach Anspruch 1, wobei die Zelle ausgewählt ist aus der Gruppe, die im Wesentlichen aus SK-MEL-29, SK-MEL-131, SK-RC-28 und SK-RC-39 besteht.

11. Zusammensetzung, umfassend eine Vielzahl von genetisch manipulierten Zellen nach einem der Ansprüche 1 bis 10.

12. Verwendung der genetisch manipulierten Zelle nach einem der Ansprüche 1 bis 10 für die Herstellung eines Arzneimittels zur Hemmung des Tumorwachstums in einem Säugerindividuum.

13. Verwendung nach Anspruch 12, wobei die Zelle weiterhin das tumorassoziierte Antigen melanomassoziiertes Antigen E-1 exprimiert.

14. Verwendung nach Anspruch 12, wobei die genetisch manipulierte Zelle eine dendritische Zelle ist.

15. Verwendung nach Anspruch 12, wobei dem Säugerindividuum etwa zehn bis etwa fünfzig Millionen genetisch manipulierter Zellen verabreicht werden sollen.

## Revendications

1. Cellule génétiquement manipulée qui co-exprime l'interleukine-2 et l'interféron-gamma et laquelle cellule est allogènique par rapport à un patient chez qui on administre la cellule.

2. Cellule génétiquement manipulée selon la revendication 1, qui exprime une autre immunomolécule choisie dans le groupe constitué d'une cytokine, une molécule d'adhésion, un facteur co-stimulateur, un antigène associé à une tumeur et un antigène spécifique d'une tumeur.

3. Cellule génétiquement manipulée selon la revendication 1, dans laquelle la cellule est une cellule « non professionnelle de la présentation d'antigène ».

4. Cellule génétiquement manipulée selon la revendication 1, dans laquelle la cellule exprime un antigène associé à une tumeur et un antigène spécifique d'une tumeur.

5. Cellule génétiquement manipulée selon la revendication 1, dans laquelle la cellule exprime soit un antigène associé à une tumeur, soit un antigène spécifique d'une tumeur.

6. Cellule génétiquement manipulée selon la revendication 1, dans laquelle la cellule est choisie dans un groupe constitué d'une cellule de mélanome, une cellule de carcinome du rein, une cellule de tumeur mammaire et une cellule de tumeur cérébrale.

7. Cellule génétiquement manipulée selon la revendication 1, dans laquelle la cellule comprend un vecteur rétroviral qui comprend :
(a) une longue répétition terminale (LRT) en 3'; et
(b) un insert comprenant :
(i) un promoteur,
(ii) un ADN codant pour l'interleukine-2 et l'interféron-gamma, et
(iii)un signal poly A,
dans lequel le promoteur, l'ADN codant pour l'interleukine-2 et l'interféron-gamma et le signal poly A sont alignés dans un cadre une orientation 5' à 3', et
dans lequel l'insert est positionné dans la LRT en 3' selon une orientation opposée à l'orientation de la LRT en 3'.

8. Cellule génétiquement manipulée selon la revendication 7, dans laquelle le promoteur est choisi dans le groupe formé par un promoteur du gène de la thymidine kinase de virus Herpès simplex, un promoteur du gène de CMV humain précoce immédiat principal et un promoteur du gène de l'adénoaine désaminase.

9. Cellule génétiquement manipulée selon la revendication 7, dans laquelle le vecteur est N2.

10. Cellule génétiquement manipulée selon la revendication 1, dans laquelle la cellule est choisie dans le groupe formé essentiellement de SK-MEL-29, SK-MEL-131, SK-RC-28 et SK-RC-39.

11. Composition comprenant une pluralité de la cellule génétiquement manipulée selon l'une quelconque des revendications 1 à 10.

12. Utilisation de la cellule génétiquement manipulée selon l'une quelconque des revendications 1 à 10, pour la préparation d'une composition pharmaceutique destinée à inhiber un développement d'un tumeur chez un sujet mammifère.

13. Utilisation selon la revendication 12, dans laquelle la cellule exprime de plus l'antigène associé à une tumeur, l'antigène E-1 associé au mélanome.

14. Utilisation selon la revendication 12, dans laquelle la cellule génétiquement manipulée est une cellule dendritique.

15. Utilisation selon la revendication 12, dans laquelle environ dix à environ cinquante millions de cellules génétiquement manipulées sont à administrer au sujet mammifère.
